# EUROPÄISCHE PATENTSCHRIFT

(11) **EP 2 758 367 B1**
(45) Veröffentlichungstag und Bekanntmachung des Hinweises auf die Patenterteilung: **18.11.2015**
(21) Anmeldenummer: 12766588.3
(22) Anmeldetag: 18.09.2012
(51) Int. Cl.: C07C 265/12, C07C 331/28, A61K 31/26, A61P 35/00

(54) **NEUE ISOCYANAT- UND ISOTHIOCYANAT-VERBINDUNGEN ZUR BEHANDLUNG VON KREBS**
NOVEL ISOCYANATE AND ISOTHIOCYANATE COMPOUNDS FOR CANCER TREATMENT
NOUVEAUX COMPOSÉS ISOCYANATE ET ISOTHIOCYANATE POUR LE TRAITEMENT DU CANCER

(30) Priorität: 23.09.2011 EP 11007772
(43) Veröffentlichungstag der Anmeldung: 30.07.2014
(73) Patentinhaber: DoubleHill GmbH, 01619 Zeithain (DE)
(72) Erfinder: KALBE, Jochen, 42799 Leichlingen (DE); HANSEN, Olaf, 51371 Leverkusen-Hitdorf (DE)
(74) Vertreter: Gille Hrabal
(86) Internationale Anmeldenummer: PCT/EP2012/003887
(87) Internationale Veröffentlichungsnummer: WO 2013/041204

(56) Entgegenhaltungen:
- EP-A1- 2 248 800
- US-A1- 2006 241 178
- ZHANG Y: "Cancer-preventive isothiocyanates: measurement of human exposure and mechanism of action", MUTATION RESEARCH, ELSEVIER, AMSTERDAM, NL, Bd. 555, Nr. 1-2, 2. November 2004 (2004-11-02), Seiten 173-190, XP004598067, ISSN: 0027-5107, DOI: 10.1016/J.MRFMMM.2004.04.017

## Beschreibung

Die vorliegende Erfindung betrifft neue Isocyanat- und Isothiocyanat-Verbindungen, sie umfassende pharmazeutische Zusammensetzungen sowie die Verwendung davon zur Behandlung von Krebserkrankungen beim Menschen und Tier. Die neuen Isocyanat- und Isothiocyanat-Verbindungen zeichnen sich gegenüber den bekannten Isocyanat- und Isothiocyanat-Verbindungen durch eine verbesserte therapeutische Breite, d.h. durch geringere Nebenwirkungen bei hoher Antitumorwirkung, aus.

### EINLEITUNG:

Aus dem Stand der Technik sind bereits verschiedene Isocyanat- und Isothiocyanat-Verbindungen zur Verwendung in der Behandlung von Krebserkrankungen bekannt.

Beispielsweise beschreibt die US 2006/241 1 78 die Behandlung von Lungenkrebs durch Verabreichung von Isothiocyanat-Konjugaten auf Basis von Phenyl-, Benzyl- und Alkyl-Isothiocyanaten sowie von Sulforaphan

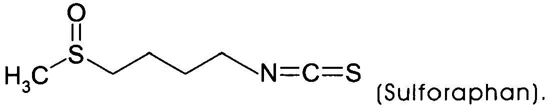

Auch die WO 1994/19948 beschreibt die Verwendung von Sulforaphan-Isothiocyanat-Verbindungen zur Verwendung in der Behandlung von Krebserkrankungen.

Aus der WO 2000/49013 sind Isothiocyanat-Verbindungen mit einem heterocyclischen 5-Ring und deren Verwendung in der Behandlung von Krebs bekannt.

Gegenstand der WO 2008/008954 sind weitere diverse Isothiocyanat-Verbindungen, insbesondere Isothiocyanat-Glucosinolate zur Verwendung in der Behandlung von Krebserkrankungen.

Die WO 2009/089889 beschreibt außerdem die zytotoxische Aktivität von Glucomoringin:

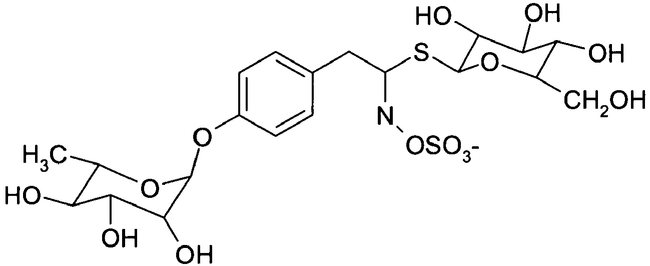

Isothiocyanat-Verbindungen der Formel SCN-R-X-R-NCS sowie deren antikanzerogene Aktivität werden außerdem in der WO 2008/082692 beschrieben.

Die US 2010/0312000 betrifft darüber hinaus neue Isothiocyanat-Verbindungen mit einer Carboxyl-Gruppe (SCN)ₘ-A-B-(CO₂H)ₙ) sowie deren Verwendung als Arzneimittel allgemein.

Der Nachteil dieser bekannten Verbindungen ist insbesondere in der geringen Selektivität gegen Tumorzellen und damit geringeren Wirksamkeit bzw. höherer Nebenwirkungsrate zu sehen.

Keine dieser Druckschriften offenbart jedoch Isocyanat- oder Isothiocyanatverbindungen der allgemeinen Formel (I), wie sie Gegenstand der vorliegenden Erfindung sind.

Aus der WO 2009/004060 sind außerdem neue Triazenverbindungen bekannt, worin anstelle der Isocyanat- bzw. Isothiocyanat-Gruppe eine Triazen-Gruppe der allgemeinen Formel -N=N-NR₁R₂ an ein Grundgerüst gemäß der allgemeinen Formel (I) der vorliegenden Erfindung gebunden ist, sowie deren Verwendung in der Behandlung von Krebs. Ein Hinweis auf die Möglichkeit des Austauschs der Triazen-Gruppe gegen eine Isothiocyanat-Gruppe findet sich darin jedoch nicht.

Der Nachteil dieser bekannten Triazenverbindungen liegt in der schnellen Metabolisierung der Triazen-Gruppe und damit dem schnelleren Verlust der Tumorzellen-abtötenden Komponente. Um hier wirksame Blutspiegel zu erhalten, muss der Wirkstoff über einen längeren Zeitraum verabreicht werden oder sehr hohe Dosierungen gewählt werden. Hierdurch erhöht sich die Rate der Nebenwirkungen. Außerdem ist die Triazen-Gruppe sehr reaktiv und reagiert relativ unselektiv mit verschiedensten gesunden und kranken Zellbestandteilen.

### AUFGABENSTELLUNG:

Die Aufgabe der vorliegenden Erfindung bestand somit darin, neue wirksame zytostatische Verbindungen mit verringerter Toxizität und besserer Wirksamkeit zu finden, um sie für die Therapie beim Menschen oder beim Tier, insbesondere zur Therapie der Krebserkrankungen beim Menschen zugänglich zu machen.

### BESCHREIBUNG DER ERFINDUNG

Die Erfinder fanden neue Isocyanat- und Isothiocyanat-Verbindungen mit hoher zytostatischer Aktivität, deren Toxizität deutlich reduziert ist. Gegenstand der Erfindung sind daher Verbindungen der Formel (1): 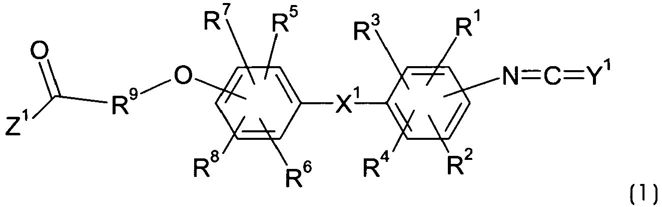 worin
Y¹ = S oder O ist und worin
R¹, R², R³, R⁴, R⁵, R⁶, R⁷ und R⁸ gleich oder verschieden sind, und jeweils ausgewählt werden aus der Gruppe, die besteht aus:
- Wasserstoff,
- Hydroxy,
- Halogen,
- Cyano,
- Nitro,
- Carboxyl,
- Aminocarbonyl,
- Sulfonsäurerest (-SO₃H),
- Aminosulfonyl,
- gegebenenfalls substituiertem Alkyl,
- gegebenenfalls substituiertem Alkoxy,
- gegebenenfalls substituiertem Alkenyl,
- gegebenenfalls substituiertem Aryl,
- gegebenenfalls substituiertem Arylalkyl;
   R⁹ gegebenenfalls substituiertes geradkettiges, verzweigtes oder cyclisches Alkandiyl, gegebenenfalls substituiertes geradkettiges, verzweigtes oder cyclisches Alkendiyl, Aryl oder Heterocyclyl ist;
   Z¹ ausgewählt ist aus
- Hydroxy (-OH) oder
- einem Rest der Formel (-NR¹⁰R¹¹) 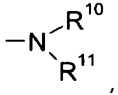 worin
   R¹⁰ Wasserstoff ist und R¹¹ gegebenenfalls substituiertes Alkyl oder Hydroxyl ist, oder
   R¹¹ Wasserstoff ist und R¹⁰ gegebenenfalls substituiertes Alkyl oder Hydroxyl ist, oder
   R¹⁰ und R¹¹ jeweils Alkyl sind, worin mindestens eine der Alkylgruppen mindestens einen Substituenten aufweist, oder
   R¹⁰ und R¹¹ zusammen mit dem Stickstoffatom, an dass sie gebunden sind einen gesättigten oder ungesättigen, gegebenenfalls substituierten 5 bis 8-gliedrigen Cyclus ausbilden, der gegebenenfalls weitere Heteroatome enthalten kann; und worin

X¹ aus der Gruppe ausgewählt wird, die besteht aus:
- einer Einfachbindung
- Carbonyl,
- Schwefel,
- Sauerstoff,
- Sulfoxy,
- Sulfonyl,
- Azo, und
- einem gegebenenfalls substituierten, gesättigten oder ungesättigten aliphatischen Rest mit 1 bis 6 Kohlenstoffatomen,
oder pharmazeutisch verträgliche Salze davon.

Gegebenenfalls substituiertes Alkyl schließt im Rahmen der gesamten Erfindung, d.h. auch im Zusammenhang mit den übrigen Substituentengruppen (wobei wo angegeben, wie im Falle von R¹⁰ und R¹¹, weitere Möglichkeiten eingeschlossen sein können), bevorzugt ein:
Geradkettiges oder verzweigtes Alkyl mit 1 bis 8, bevorzugt 1 bis 6, Kohlenstoffatomen, Cycloalkyl mit 3 bis 8, bevorzugt 5 oder 6 Kohlenstoffatomen, oder Alkyl mit 1 bis 4 Kohlenstoffatomen, welches mit Cycloalkyl substituiert ist, die gegebenenfalls jeweils bevorzugt 1 bis 3 Substituenten tragen können, die bevorzugt aus der Gruppe ausgewählt werden, die besteht aus: Hydroxy, Halogen und Cyano. Dabei schließt Halogen hier und im Rahmen der vorliegenden Erfindung Fluor, Chlor, Brom und lod, bevorzugt Fluor oder Chlor, ein. Des weiteren können ein oder mehrere, bevorzugter 1 bis 3 Kohlenstoffatome durch heteroanaloge Gruppen, die Stickstoff, Sauerstoff oder Schwefel enthalten, ersetzt sein. Dies bedeutet insbesondere, dass beispielsweise eine oder mehrere Methylengruppen in den Alkylresten durch NH, O oder S ersetzt sein können.

Beispiele von Alkylresten mit 1 bis 8 Kohlenstoffatomen schließen ein: eine Methylgruppe, eine Ethylgruppe, eine n-Propylgruppe, eine i-Propylgruppe, eine n-Butylgruppe, eine i-Butylgruppe, eine sec-Butylgruppe, eine t-Butylgruppe, eine n-Pentylgruppe, eine i-Pentylgruppe, eine sec-Pentylgruppe, eine t-Pentylgruppe, eine 2-Methylbutylgruppe, eine n-Hexylgruppe, eine 1-Methylpentylgruppe, eine 2-Methylpentylgruppe, eine 3-Methylpentylgruppe, eine 4-Methylpentylgruppe, eine 1-Ethylbutylgruppe, eine 2-Ethylbutylgruppe, eine 3-Ethylbutylgruppe, eine 1,1-Dimethylbutylgruppe, eine 2,2-Dimethylbutylgruppe, eine 3,3-Dimethylbutylgruppe, eine 1-Ethyl-1-methylpropylgruppe, eine n-Heptylgruppe, eine 1-Methylhexylgruppe, eine 2-Methylhexylgruppe, eine 3-Methylhexylgruppe, eine 4-Methylhexylgruppe, eine 5-Methylhexylgruppe, eine 1-Ethylpentylgruppe, eine 2-Ethylpentylgruppe, eine 3-Ethylpentylgruppe, eine 4-Ethylpentylgruppe, eine 1,1-Dimethylpentylgruppe, eine 2,2-Dimethylpentylgruppe, eine 3,3-Dimethylpentylgruppe, eine 4,4-Dimethylpentylgruppe, eine 1 -Propylbutylgruppe, eine n-Octylgruppe, eine 1-Methylheptylgruppe, eine 2-Methylheptylgruppe, eine 3-Methylheptylgruppe, eine 4-Methylheptylgruppe, eine 5-Methylheptylgruppe, eine 6-Methylheptylgruppe, eine 1-Ethylhexylgruppe, eine 2-Ethylhexylgruppe, eine 3-Ethylhexylgruppe, eine 4-Ethylhexylgruppe, eine 5-Ethylhexylgruppe, eine 1,1-Dimethylhexylgruppe, eine 2,2-Dimethylhexylgruppe, eine 3,3-Dimethylhexylgruppe, eine 4,4-Dimethylhexylgruppe, eine 5,5-Dimethylhexylgruppe, eine 1-Propylpentylgruppe, eine 2-Propylpentylgruppe, usw. Bevorzugt sind solche mit 1 bis 6 Kohlenstoffatomen, insbesondere Methyl, Ethyl und n-Propyl. Am meisten bevorzugt ist Methyl.

Beispiele von Alkylgruppen, die durch Austausch mit einer oder mehreren heteroanalogen Gruppe, wie -O-, -S- oder -NH- hervorgehen sind bevorzugt solche, in denen eine oder mehrere Methylengruppen durch -O- unter Bildung einer Ethergruppe ersetzt sind, wie Methoxymethyl, Ethoxymethyl, 2-Methoxyethylen usw. Erfindungsgemäß sind auch Polyethergruppen von der Definition von Alkyl umfasst.

Cycloalkylreste mit 3 bis 8 Kohlenstoffatomen schließen bevorzugt ein: eine Cyclopropylgruppe, eine Cyclobutylgruppe, eine Cyclopentylgruppe, eine Cyclohexylgruppe, eine Cycloheptylgruppe, eine Cyclooctylgruppe usw. ein. Bevorzugt sind eine Cyclopropylgruppe, eine Cyclobutylgruppe, eine Cyclopentylgruppe und eine Cyclohexylgruppe. Heterocyclische Alkylreste, die durch Austausch von Methylen durch Heteroanaloge Gruppen aus Cycloalkyl gebildet werden, sind beispielsweise 5- oder 6-gliedrige heterocyclische Reste, wie Tetrahydrofuryl, Pyrrolidinyl, Piperidinyl oder Tetrahydropyranyl, welche gegebenenfalls mit aromatischen Ringen kondensiert sein können, etc.

Insbesondere schließen Beispiele eines mit Halogen substituierten linearen oder verzweigten Alkylrestes mit 1 bis 8 Kohlenstoffatomen ein:
eine Fluormethylgruppe, eine Difluormethylgruppe, eine Trifluormethylgruppe, eine Chlormethylgruppe, eine Dichlormethylgruppe, eine Trichlormethylgruppe, eine Brommethylgruppe, eine Dibrommethylgruppe, eine Tribrommethylgruppe, eine 1-Fluorethylgruppe, eine 1-Chlorethylgruppe, eine 1-Bromethylgruppe, eine 2-Fluorethylgruppe, eine 2-Chlorethylgruppe, eine 2-Bromethylgruppe, eine 1,2-Difluorethylgruppe, eine 1,2-Dichlorethylgruppe, eine 1,2-Dibromethylgruppe, eine 2,2,2-Trifluorethylgruppe, eine Heptafluorethylgruppe, eine 1-Fluorpropylgruppe, eine 1-Chlorpropylgruppe, eine 1-Brompropylgruppe, eine 2-Fluorpropylgruppe, eine 2-Chlorpropylgruppe, eine 2-Brompropylgruppe, eine 3-Fluorpropylgruppe, eine 3-Chlorpropylgruppe, eine 3-Brompropylgruppe, eine 1 ,2-Difluorpropylgruppe, eine 1,2-Dichlorpropylgruppe, eine 1,2-Dibrompropylgruppe, eine 2,3-Difluorpropylgruppe, eine 2,3-Dichlorpropylgruppe, eine 2,3-Dibrompropylgruppe, eine 3,3,3-Trifluorpropylgruppe, eine 2,2,3,3,3-Pentafluorpropylgruppe, eine 2-Fluorbutylgruppe, eine 2-Chlorbutylgruppe, eine 2-Brombutylgruppe, eine 4-Fluorbutylgruppe, eine 4-Chlorbutylgruppe, eine 4-Brombutylgruppe, eine 4,4,4-Trifluorbutylgruppe, eine 2,2,3,3,4,4,4-Heptafluorbutylgruppe, eine Perfluorbutylgruppe, eine 2-Fluorpentylgruppe, eine 2-Chlorpentylgruppe, eine 2-Brompentylgruppe, eine 5-Fluorpentylgruppe, eine 5-Chlorpentylgruppe, eine 5-Brompentylgruppe, eine Perfluorpentylgruppe, eine 2-Fluorhexylgruppe, eine 2-Chlorhexylgruppe, eine 2-Bromhexylgruppe, eine 6-Fluorhexylgruppe, eine 6-Chlorhexylgruppe, eine 6-Bromhexylgruppe, eine Perfluorhexylgruppe, eine 2-Fluorheptylgruppe, eine 2-Chlorheptylgruppe, eine 2-Bromheptylgruppe, eine 7-Fluorheptylgruppe, eine 7-Chlorheptylgruppe, eine 7-Bromheptylgruppe, eine Perfluorheptylgruppe, usw.

Beispiele eines mit Hydroxy substituierten Alkylrestes schließen die oben genanten Alkylreste ein, die 1 bis 3 Hydroxylreste aufweisen, wie zum Beispiel Hydroxymethyl, 2-Hydroxyethyl, 3-Hydroxypropyl etc.

Gegebenenfalls substituiertes Alkenyl schließt im gesamten Rahmen der Erfindung bevorzugt ein:
Geradkettiges oder verzweigtkettiges Alkenyl mit 2 bis 8 Kohlenstoffatomen und Cycloalkenyl mit 3 bis 8 Kohlenstoffatomen, die gegebenenfalls durch bevorzugt 1 bis 3 Substituenten substituiert sein können, wie Hydroxy, Halogen oder Alkoxy. Beispiele schließen ist ein: Vinyl, 1-Methylvinyl, Allyl, 1-Butenyl, Isopropenyl, Cyclopropenyl, Cyclobutenyl, Cyclopentenyl, Cyclohexenyl. Bevorzugt sind Vinyl oder Allyl.

Gegebenenfalls substituiertes Aryl schließt im gesamten Rahmen der Erfindung bevorzugt ein:
Aromatische Kohlenwasserstoffreste mit 6 bis 14 Kohlenstoffatomen (wobei die Kohlenstoffatome der Substituenten nicht mitgezählt sind) und 5- bis 10-gliedrige aromatische heterocyclische Reste mit bis zu 3 Heteroatomen aus der Reihe S, O, N, welche mono- oder bicyclisch sein können und die durch bevorzugt 1 bis 3 Substituenten ausgewählt aus Hydroxy, Halogen, Cyano, Alkyl, Acyl und Alkoxy substituiert sein können. Dabei kann bezüglich der Definition von Alkyl und Halogen auf die vorstehenden Definitionen bzw. Beispiele verwiesen werden.
Alkoxy als Substituent von Aryl schließt hier und im folgenden beispielsweise ein: Ein vorstehend genannter Alkylrest, der über ein Sauerstoffatom an Aryl gebunden ist, wie ein linearer oder verzweigter Alkoxyrest mit bis zu 6 Kohlenstoffatomen, wie eine Methoxygruppe, eine Ethoxygruppe, eine n-Propyloxygruppe, eine i-Propyloxygruppe, eine n-Butyloxygruppe, eine i-Butyloxygruppe, eine sec-Butyloxygruppe, eine t-Butyloxygruppe, eine n-Pentyloxygruppe, eine i-Pentyloxygruppe, eine sec-Pentyloxygruppe, eine t-Pentyloxygruppe, eine 2-Methylbutoxygrupe, eine n-Hexyloxygruppe, eine i-Hexyloxygruppe, eine t-Hexyloxygruppe, eine sec-Hexyloxygruppe, eine 2-Methylpentyloxygruppe, eine 3-Methylpentyloxygruppe, eine 1-Ethylbutyloxygruppe, eine 2-Ethylbutyloxygruppe, eine 1,1-Dimethylbutyloxygruppe, eine 2,2-Dimethylbutyloxygruppe, eine 3,3-Dimethylbutyloxygruppe, eine 1-Ethyl-1-methylpropyloxygruppe, usw. ein. Bevorzugt sind eine Methoxygruppe, eine Ethoxygruppe, eine n-Propyloxygruppe, eine i-Propyloxygruppe, eine n-Butyloxygruppe, eine i-Butyloxygruppe, eine sec-Butyloxygruppe, eine t-Butyloxygruppe, usw.. Acyl als Substituent von Aryl schließt hier und im folgenden ein: aliphatisches Acyl, aromatisches Acyl, wie C1 bis C6 Alkanoyl, wie Formyl, Acetyl, Propionyl, Butyryl, Isobutyryl, Valeryl, Isovaleryl, Pivaloyl, Hexanoyl, usw. sowie C6 bis C10 Aroyl, wie Benzoyl, Toluoyl, Xyloyl, usw..

Aromatische Kohlenwasserstoffreste mit 6 bis 14 Kohlenstoffatomen schließen beispielweise ein: Phenyl, Naphthyl, Phenanthrenyl und Anthracenyl, die gegebenenfalls substituiert sein können. Bevorzugt ist Phenyl.

Heteroaromatische Reste schließen beispielsweise ein: Pyridyl, Pyridyl-N-oxid, Pyrimidyl, Pyridazinyl, Pyrazinyl, Thienyl, Furyl, Pyrrolyl, Pyrazolyl, Imidazolyl, Thiazolyl, Oxazolyl oder Isoxazolyl, Indolizinyl, Indolyl, Benzo[b]thienyl, Benzo[b]furyl, indazolyl, Chinolyl, Isochinolyl, Naphthyridinyl, Chinazolinyl. 5-oder 6-gliedrige aromatische Heterocyclen wie z.B. Pyridyl, Pyridyl-N-oxid, Pyrimidyl, Pyridazinyl, Furyl und Thienyl sind bevorzugt.

Gegebenenfalls substituiertes Arylalkyl schließt im gesamten Rahmen der Erfindung bevorzugt ein:
Geradkettiges oder verzweigtes Alkyl mit 1 bis 8, bevorzugt 1 bis 4 Kohlenstoffatomen, wie oben beschrieben, welches mit Aryl, wie oben beschrieben, substituiert ist. Bevorzugtes Arylalkyl ist Benzyl.

Zusätzlich stellt Aminocarbonyl im Rahmen der gesamten Erfindung bevorzugt Carbamoyl (H₂NCO-) oder Mono- oder Dialkylaminocarbonyl (H(Alkyl)NCO- oder (Alkyl)₂NCO-) dar, worin hinsichtlich der Definition von Alkyl auf die oben stehenden Erläuterungen verwiesen werden kann und auch gegebenenfalls substituiertes Alkyl einschließt.

Des weiteren stellt Aminosulfonyl im Rahmen der gesamten Erfindung insbesondere Sulfamoyl (H₂N-SO₂-) oder Mono- oder Dialkylaminosulfonyl (Alkyl)₂N-SO₂ dar, worin hinsichtlich der Definition von Alkyl auf die oben stehenden Erläuterungen verwiesen werden kann und auch gegebenenfalls substituiertes Alkyl einschließt.

Gegebenenfalls substituiertes Alkoxy schließt oben erwähntes als Substituent von Aryl exemplifiziertes Alkoxy ein, welches gegebenenfalls durch bevorzugt 1 bis 3 Substituenten substituiert sein kann, welche bevorzugt ausgewählt werden aus der Gruppe von Halogen, Hydroxy und Cyano.

Bevorzugt werden R¹, R², R³, R⁴, R⁵, R⁶, R⁷ und R⁸ ausgewählt aus:
- Wasserstoff,
- Hydroxy,
- Halogen,
- Cyano,
- Nitro,
- Carboxyl,
- Aminocarbonyl,
- Sulfonsäure (-SO₃H),
- Aminosulfonyl,
- gegebenenfalls substituiertem Alkyl, und
- gegebenenfalls substituiertem Alkoxy.

Bevorzugter werden R¹, R², R³, R⁴, R⁵, R⁶, R⁷ und R⁸ ausgewählt aus:
- Wasserstoff,
- Hydroxy,
- Halogen, und
- gegebenenfalls substituiertem Alkoxy.

Bevorzugt stellen wenigstens 6, bevorzugter wenigstens 7 der Reste R¹, R², R³, R⁴, R⁵, R⁶, R⁷ und R⁸ Wasserstoff dar. Am meisten bevorzugt stellen alle Reste R¹, R², R³, R⁴, R⁵, R⁶, R⁷ und R⁸ Wasserstoff dar.

R⁹ ist in der allgemeinen Formel (1) gegebenenfalls substituiertes geradkettiges, verzweigtes (verzweigtkettiges) oder cyclisches Alkandiyl, gegebenenfalls substituiertes geradkettiges, verzweigtes (verzweigtkettiges) oder cyclisches Alkendiyl, gegebenenfalls substituiertes Aryl oder gegebenenfalls substituiertes Heterocyclyl.

Gegebenenfalls substituiertes Alkandiyl ist bevorzugt ein zweiwertiger geradkettiger oder verzweigter Alkandiylrest mit 1 bis 7 bevorzugt 1 bis 6, noch bevorzugter 1 bis 4 Kohlenstoffatomen, welcher gegebenenfalls 1 bis 3 Substituenten tragen kann, welche aus der Gruppe ausgewählt werden, die aus Hydroxy, Halogen und Cyano besteht. Beispielhaft und vorzugsweise seien genannt: Methylen, 1,2-Ethandiyl, Ethan-1,1-diyl, 1,3-Propylen, Propan-1,1-diyl, Propan-1,2-diyl, Propan-2,2-diyl, 1,4-Butylen, Butan-1,2-diyl, Butan-1,3-diyl, Butan-2,3-diyl, Pentan-1,5-diyl, Pentan-2,4-diyl, 3-Methyl-pentan-2,4-diyl und Hexan-1,6-diyl. Einen bevorzugten, substituierten Alkandiyl-Rest stellt ein hydroxysubstituierter Alkandiylrest dar.

Weiterhin bevorzugt ist der Alkandiylrest ein cyclischer Alkandiylrest mit 3 bis 8, bevorzugt 5 oder 6 Kohlenstoffatomen wie vorstehend im Zusammenhang mit gegebenenfalls substituiertem Alkyl definiert. Hinsichtlich der bevorzugten cyclischen Alkandiylreste kann auf die vorstehende Nennung bevorzugter Cycloalkylreste verwiesen werden.

Gegebenenfalls substituiertes Alkendiyl ist bevorzugt ein zweiwertiger geradkettiger oder verzweigter Alkendiylrest mit 2 bis 7, bevorzugter 2 bis 6, noch bevorzugter 2 bis 4 Kohlenstoffatomen, welcher gegebenenfalls 1 bis 3 Substituenten tragen kann, welche aus der Gruppe ausgewählt werden, die aus Hydroxy, Halogen und Cyano besteht. Beispielhaft und vorzugsweise seien genannt: Ethen-1,1-diyl, Ethen-1,2-diyl, Propen-1,1-diyl, Propen-1,2-diyi, Propen-1,3-diyl, But-1-en-1,4-diyl, But-1-en-1,3-diyl, But-2-en-1,4-diyl, Buta-1,3-dien-1,4-diyl, Pent-2-en-1,5-diyl, Hex-3-en-1,6-diyl und Hexa-2,4-dien-1,6-diyl.

Weiterhin bevorzugt ist der Alkendiylrest ein cyclischer Alkendiylrest mit 4 bis 8, bevorzugt 5 oder 6 Kohlenstoffatomen.

Bezüglich der Definition von gegebenenfalls substituiertem Aryl als Substituent für R⁹ wird auf die vorstehende Definition von gegebenenfalls substituertem Aryl verwiesen.

Gegebenenfalls substituiertes Heterocyclyl schließt im gesamten Rahmen der Erfindung bevorzugt ein:
Aliphatische, gesättigte oder ungesättigte heterocyclische 5 bis 8 gliedrige cyclische Reste, die 1 bis 3, bevorzugt 1 bis 2 Heteroatome, ausgewählt aus N, O oder S aufweisen, und die gegebenenfalls bevorzugt durch 1 bis 3 Substituenten substituiert sein können, wobei bezüglich möglicher Substituenten auf die Definition der möglichen Substituenten von Alkyl verwiesen werden kann. Bevorzugt sind 5- oder 6-gliedrige gesättigte oder ungesättigte, gegebenenfalls substituierte heterocyclische Reste, wie Tetrahydrofuran-2-yl, Tetrahydrofuran-3-yl, Tetrahydro-thiophen-2-yl, Tetrahydro-thiophen-3-yl, Pyrrolidin-1 -yl, Pyrrolidin-2-yl, Pyrrolidin-3-yl, Morpholin-1-yl, Morpholin-2-yl, Morpholin-3-yl, Piperidin-1 -yl, Piperidin-2-yl, Piperidin-3-yl, Piperidin-4-yl, Piperazin-1-yl, Piperazin-2-yl, Tetrahydropyran-2-yl, Tetrahydropyran-3-yl, Tetrahydropyran-4-yl, etc., welche gegebenenfalls mit aromatischen Ringen kondensiert sein können, etc.

Außerdem schließt gegebenenfalls substituiertes Heterocyclyl im gesamten Rahmen der Erfindung heteroaromatische Kohlenwasserstoffreste mit 4 bis 9 Ring-Kohlenstoffatomen, die zusätzlich bevorzugt 1 bis 3 gleiche oder verschiedene Heteroatome aus der Reihe S, O, N im Ring aufweisen, und die somit bevorzugt 5- bis 12-gliedrige heteroaromatische Reste bilden, welche bevorzugt monocyclisch aber auch bicyclisch sein können. Bevorzugte aromatische heterocyclische Reste schließen ein: Pyridinyl, wie Pyridin-2-yl, Pyridin-3-yl und Pyridin-4-yl, Pyridyl-N-oxid, Pyrimidyl, Pyridazinyl, Pyrazinyl, Thienyl, Furyl, Pyrrolyl, Pyrazolyl, Imidazolyl, Thiazolyl, Oxazolyl oder Isoxazolyl, Indolizinyl, Indolyl, Benzo[b]thienyl, Benzo[b]furyl, indazolyl, Chinolyl, Isochinolyl, Naphthyridinyl, Chinazolinyl. 5- oder 6-gliedrige aromatische Heterocyclen wie z.B. Pyridinyl, inbesondere Pyridin-2-yl, Pyridyl-N-oxid, Pyrimidyl, Pyridazinyl, Furyl und Thienyl sind bevorzugt.

Die erfindungsgemäßen Heterocyclyl-Reste können durch bevorzugt 1 bis 3 gleiche oder verschiedene Substituenten ausgewählt beispielsweise aus Hydroxy, Halogen, wie vorstehend definiert, Cyano, Amino, wie nachstehend definiert, Mercapto, Alkyl, wie vorstehend definiert, Acyl, wie nachstehend definiert, und Alkoxy, wie vorstehend definiert, Aryloxy, wie vorstehend definiert, Heterocyclyloxy, wie vorstehend definiert, Aryl, wie vorstehend definiert, Heterocyclyl, wie hier definiert, substituiert sein.

Heterocyclyl schließt bevorzugt ein: Tetrahydrofuranyl, Pyrrolidinyl, Morpholinyl, Piperidinyl oder Tetrahydropyranyl, Pyridinyl, Pyridyl-N-oxid, Pyrimidyl, Pyridazinyl, Pyrazinyl, Thienyl, Furanyl, Pyrrolyl, Pyrazolyl, Imidazolyl, Thiazolyl, Oxazolyl oder Isoxazolyl, Indolizinyl, Indolyl, Benzo[b]thienyl, Benzo[b]furyl, Indazolyl, Chinolyl, Isochinolyl, Naphthyridinyl, Chinazolinyl, Chinoxazolinyl. 5-oder 6-gliedrige Heterocyclen wie z. B. Morpholinyl sowie aromatische Heterocyclen wie z.B. Pyridyl, Pyridyl-N-oxid, Pyrimidyl, Pyridazinyl, Furanyl, Thienyl sowie Chinolyl und Isochinolyl sind bevorzugt. Bevorzugt sind Morpholinyl, Pyridyl, Pyrimidyl und Furanyl. Besonders bevorzugtes Heterocyclyl schließt ein: Morpholinyl, Pyridyl, wie Pyridin-2-yl, Pyridin-3-yl, Pyridin-4-yl, Pyrimidinyl, wie Pyrimidin-2-yl und Pyrimidin-5-yl, Pyrazin-2-yl, Thienyl, wie Thien-2-yl und Thien-3-yl sowie Furanyl, wie Furan-2-yl und Furan-3-yl.

R⁹ ist im Rahmen der vorliegenden Erfindung besonders bevorzugt Alkandiyl, bevorzugter Alkandiyl mit 1, 2 und 3 Kohlenstoffatomen, entsprechend Methylen (-CH₂-), 1,2-Ethandiyl (-CH₂CH₂-), 1,2-Isopropandiyl (-CH₂-C(CH₃)H-bzw. -C(CH₃)H-CH₂-) oder 1,3-Propandiyl (-CH₂CH₂CH₂-). Bevorzugt ist R⁹ 1,2-Ethandiyl (-CH₂CH₂-), 1,3-Propandiyl oder 1,2-Isoprandiyl.

In der allgemeinen Formel (1) wird X¹ aus der Gruppe ausgewählt, die besteht aus:
- einer Einfachbindung
- Carbonyl (-CO-),
- Schwefel (-S-),
- Sauerstoff (-O-),
- Sulfoxy (-SO-),
- Sulfonyl (-SO₂-),
- Azo (-N=N-), und
- einem gegebenenfalls substituierten, gesättigten oder ungesättigten aliphatischen Rest mit 1 bis 6 Kohlenstoffatomen.

Ein gegebenenfalls substituierter, gesättigter oder ungesättigter aliphatischer Rest mit 1 bis 6 Kohlenstoffatomen für X¹ schließt im Rahmen der vorliegenden Erfindung ein: gegebenenfalls substituiertes Alkandiyl, wie oben definiert, gegebenenfalls substituiertes Alkendiyl, wie oben definiert, und Alkindiyl ein. Bevorzugt ist X¹ Alkandiyl, Alkendiyl oder Alkindiyl mit bis zu 4, bevorzugter mit bis zu 2 Kohlenstoffatomen, wie Methylen (-CH₂-), welcher gegebenenfalls durch Hydroxyl substituiert sein kann (wie zum Beispiel -CH(OH)-).

Am meisten bevorzugt sind Verbindungen der allgemeinen Formel (1) in denen X¹ Carbonyl (-CO-) ist.

In der allgemeinen Formel (1) hat Y¹ die Bedeutung von S unter Bildung einer Isothiocyanatgruppe (-NCS) oder O unter Bildung einer Isocyanatgruppe (-NCO). Bevorzugt ist die Isothiocyanatgruppe mit Y'=S.

In der allgemeinen Formel (1) hat Z¹ die Bedeutung von Hydroxy (-OH) oder einem Rest der Formel -NR¹⁰R¹¹.

Darin werden die Substituenten R¹⁰ und R¹¹ im Rahmen der vorliegenden Erfindung unter den folgenden Alternativen ausgewählt:
1) R¹⁰ ist Wasserstoff und R¹¹ ist gegebenenfalls substituiertes Alkyl oder Hydroxyl, oder
   R¹¹ ist Wasserstoff und R¹⁰ ist gegebenenfalls substituiertes Alkyl oder Hydroxyl.
   Die unter 1) genannten Alternativen sind an sich äquivalent. Sie entsprechen dem Fall, worin ein Substituent von R¹⁰ oder R¹¹ Wasserstoff und der jeweils andere Substituent gegebenenfalls substituiertes Alkyl oder Hydroxyl ist.
2) R¹⁰ und R¹¹ sind jeweils Alkyl, worin mindestens eine der Alkylgruppen mindestens einen Substituenten aufweist, R¹⁰ und R¹¹ also substituiertes Alkyl sind, oder
3) R¹⁰ und R¹¹ bilden zusammen mit dem Stickstoffatom, an dass sie gebunden sind, einen gesättigten oder ungesättigten, gegebenenfalls substituierten 5 bis 8-gliedrigen Cyclus aus, der gegebenenfalls weitere Heteroatome enthalten kann.

### Alternative 1):

Vorstehend genannte Alternative 1) ist im Rahmen der vorliegenden Erfindung eine bevorzugte Alternative. Noch bevorzugter ist im Rahmen dieser Alternative:
R¹⁰ Wasserstoff, und R¹¹ ist substituiertes Alkyl, oder
R¹¹ Wasserstoff, und R¹⁰ ist substituiertes Alkyl.

Alkyl schließt dabei geradkettiges oder verzweigtes Alkyl mit 1 bis 8, bevorzugt 1 bis 6, Kohlenstoffatomen, Cycloalkyl mit 3 bis 8, bevorzugt 5 oder 6 Kohlenstoffatomen, oder Alkyl mit 1 bis 4 Kohlenstoffatomen, welches mit Cycloalkyl substituiert ist, ein. Hinsichtlich möglicher Beispiele von Alkyl kann auf die oben genannten Beispiele verwiesen werden. Besonders bevorzugt ist Alkyl hier eine C1 bis C6, bevorzugt C1 bis C5 Alkylgruppe, welche verzweigt oder geradkettig sein kann, wie insbesondere Methyl, Ethyl, Propyl, 2-Methylpropan, Butyl, wie n-Butyl, 2-Methylbutyl, 3-Methylbutyl, Pentyl, wie n-Pentyl, oder n-Hexyl. Besagte Alkylgruppen sind dabei mit mindestens einem Substituenten substituiert. Bevorzugte Substituenten an Alkyl sind polare funktionelle Gruppen, welche ein oder mehrere Heteroatome enthalten, welche bevorzugt ausgewählt werden aus: N, O, S, Halogen, wie Cl, F, Br und I. Substituenten an Alkyl schließen in der Definition von R¹⁰ und R¹¹ insbesondere ein:

Eine Gruppe der Formel:

-X-R¹²,

worin
X² ausgewählt wird aus der Gruppe, die besteht aus:
- Carbonyl,
- Sulfoxy, und
- Sulfonyl, und
R¹² ausgewählt wird aus der Gruppe, die besteht aus:
- Hydroxy,
- gegebenenfalls substituiertem Amino, und
- gegebenenfalls substituiertem Alkoxy.

Bevorzugt ist X² dabei Carbonyl und R¹² ist Hydroxy.

Wenn R¹² Hydroxy ist, ergibt sich dabei für die Substituentengruppe -X²R¹² = Carboxy. Wenn R¹² gegebenenfalls substituiertes Amino ist, ergibt sich dabei beispielsweise für die Substituentengruppe -X²R¹² = -CONH₂, also Carbamoyl im Falle von R¹² = Amino, oder -X²R¹² = Mono- oder Dialkylaminocarbonyl im Falle R¹² = Alkylamino oder Dialkylamino. Wenn R¹² gegebenenfalls substituiertes Alkoxy ist, ergibt sich dabei beispielsweise für die Substituentengruppe -X²R¹² = Alkoxycarbonyl im Falle von R¹² = Alkoxy, also eine Estergruppe. Bevorzugt weisen Substituenten für Alkyl in der Definition von R¹⁰ und R¹¹ mindestens eine Gruppe, bevorzugt eine oder zwei Gruppen der Formel -X²R¹² auf.

Weitere bevorzugte Substituenten an Alkyl in der Definition von R¹⁰ und R¹¹ schließen neben der bevorzugt vorhandenen Gruppe -X²R¹² folgende Substituenten ein:
- Guanidino,
- Thiol (-SH),
- Alkylthio, wie insbesondere Methylthio,
- Amino (-NH₂)
- Mono- oder Dialkylamino,
- Acylamino, worin Acyl insbesondere wie oben definiert ist,
- gesättige, ungesättigte oder aromatische, mono- oder bicyclische, gegebenenfalls substituierte heterocyclische Reste, wie beispielsweise vorstehend erwähnte gegebenenfalls substituierte heteroaromatische Reste, bevorzugt Imidazolyl, wie Imidazol-5-yl, 1 H-Indolyl, wie 1H-Indol-3-yl,
- gegebenenfalls substituiertes Aryl, wie oben beschrieben, insbesondere Phenyl, Hydroxyphenyl, wie 4-Hydroxyphenyl, Alkoxyphenyl, wie Methoxyphenyl,
- Hydroxyl,
- Alkoxy, wie vorstehend beschrieben.

Alkyl in der Definition von R¹⁰ und R¹¹ weist bevorzugt ein oder zwei Substituenten auf, wovon bevorzugt mindestens ein Substituent die Gruppe -X²R¹² darstellt.

In einer bevorzugten Ausführungsform der vorstehend erwähnten Alternative 1) ist:
R¹⁰ Wasserstoff und R¹¹ ein Rest A einer Verbindung der Formel H₂N-A oder
R¹¹ ist Wasserstoff und R¹⁰ ein Rest A einer Verbindung der Formel H₂N-A, worin
A jeweils ein Rest ist der sich formal durch Abspaltung einer Aminogruppe (-NH₂) aus einer natürlichen oder synthetischen Aminosäure, einem natürlichen oder synthetischen Aminosäurederivat oder einer Polyaminosäure oder einem Polyaminosäurederivat ableitet.

Zur Veranschaulichung:
Ist die Aminosäure H₂N-A beispielsweise Glycin: 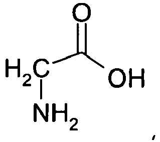 so ist A = R¹⁰ oder R¹¹ ein Rest der Formel:

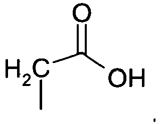

Bevorzugter sind erfindungsgemäße Verbindungen, worin A der Rest ist, der sich formal durch Abspaltung der Aminogruppe aus einer Aminosäure oder einem Aminosäurederivat ableitet (Zur Klarstellung: Die formale Abspaltung der Aminogruppe aus einer Aminosäure meint nicht die Abspaltung einer Aminogruppe aus einer eventuell vorhandenen Amidgruppe (H₂N-CO-), sondern einer Aminogruppe, welche an ein Kohlenstoffatom gebunden ist, dass außer H oder C keine weiteren Substituenten trägt. Also der entsprechende durch Abspaltung einer Aminogruppe aus Asparagin hervorgehende Rest R¹⁰ oder R¹¹ wäre: 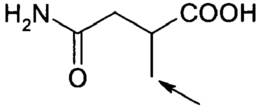 (der Pfeil symbolisiert die Bindungsstelle) und nicht: 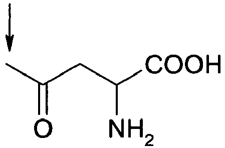 (der Pfeil symbolisiert die Bindungsstelle)).

Noch bevorzugter geht der Rest A aus der Abspaltung einer H₂N-Gruppe aus der Gruppe der folgenden Aminosäuren hervor:
- Alanin, entsprechend dem Fall, worin R¹⁰ oder R¹¹ Ethyl, substituiert mit Carboxy, ist,
- Arginin (weniger bevorzugt), entsprechend dem Fall, worin R¹⁰ oder R¹¹ Butyl, substituiert mit Carboxy und Guanidino, ist,
- Asparagin, entsprechend dem Fall, worin R¹⁰ oder R¹¹ Ethyl, substituiert mit Aminocarbonyl (Carbamoyl) und Carboxy, ist,
- Asparaginsäure, entsprechend dem Fall, worin R¹⁰ oder R¹¹ Ethyl, substituiert mit zwei Carboxygruppen, ist,
- Cystein (weniger bevorzugt), entsprechend dem Fall, worin R¹⁰ oder R¹¹ Ethyl, substituiert mit Thio (-SH) und Carboxy, ist,
- Glutamin, entsprechend dem Fall, worin R¹⁰ oder R¹¹ Propyl, substituiert mit Aminocarbonyl (Carbamoyl) und Carboxy, ist,
- Glutaminsäure, entsprechend dem Fall, worin R¹⁰ oder R¹¹ Propyl, substituiert mit zwei Carboxygruppen, ist,
- Glycin, entsprechend dem Fall, worin R¹⁰ oder R¹¹ Methyl, substituiert mit Carboxy, ist,
- Histidin, entsprechend dem Fall, worin R¹⁰ oder R¹¹ Ethyl, substituiert mit Carboxy und Imidazolyl, ist,
- Isoleucin, entsprechend dem Fall, worin R¹⁰ oder R" 2-Methylbutyl, substituiert mit Carboxy, ist,
- Leucin, entsprechend dem Fall, worin R¹⁰ oder R¹¹ 3-Methylbutyl, substituiert mit Carboxy, ist,
- Lysin, entsprechend dem Fall, worin R¹⁰ oder R¹¹ n-Pentyl, substituiert mit Carboxy und Amino, ist, wobei die Bindung über die zur Carboxylgruppe nachbarständige Aminogruppe: 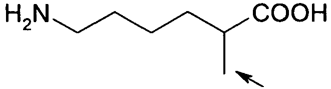 (Pfeil zeigt auf den Bindungsstrich bzw. die Bindungsstelle) oder über die endständige Aminogruppe erfolgen kann: 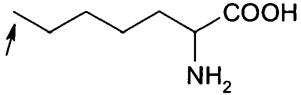 (Pfeil zeigt auf den Bindungsstrich bzw. die Bindungsstelle), die entsprechenden Verbindungen der Formel (1) also wie folgt aussehen: 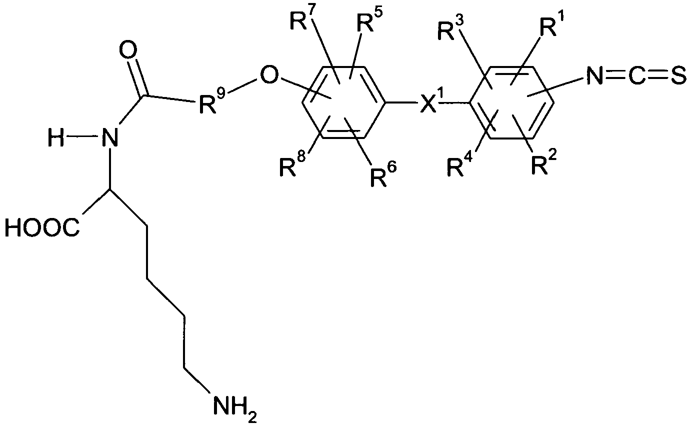 bzw. 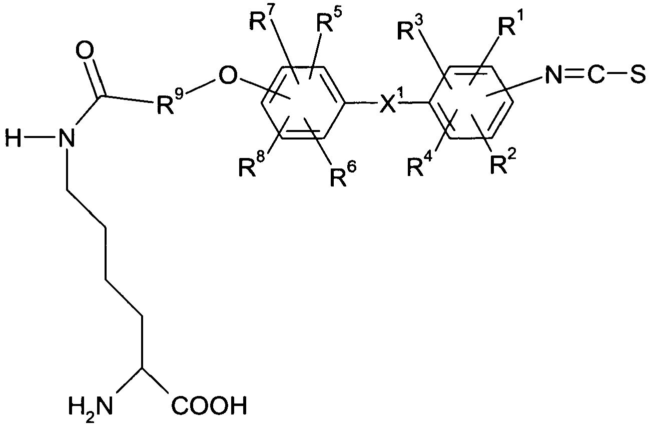 (dies gilt analog für andere basische Aminosäuren mit mehr als einer Aminogruppe),
- Methionin, entsprechend dem Fall, worin R¹⁰ oder R¹¹ n-Propyl, substituiert mit Carboxy und Methylthio, ist,
- Phenylalanin, entsprechend dem Fall, worin R¹⁰ oder R¹¹ Ethyl, substituiert mit Carboxy und Phenyl, ist,
- Serin, entsprechend dem Fall, worin R¹⁰ oder R¹¹ Ethyl, substituiert mit Carboxy und Hydroxy, ist,
- Threonin, entsprechend dem Fall, worin R¹⁰ oder R¹¹ n-Propyl, substituiert mit Carboxy und Hydroxy, ist,
- Tryptophan, entsprechend dem Fall, worin R¹⁰ oder R¹¹ Ethyl, substituiert mit Carboxy und Indolyl, ist,
- Tyrosin, entsprechend dem Fall, worin R¹⁰ oder R¹¹ Ethyl, substituiert mit Carboxy und Hydroxyphenyl, ist, und
- Valin, entsprechend dem Fall, worin R¹⁰ oder R¹¹ 2-Methylpropyl ist, substituiert mit Carboxy, ist,
oder Derivate, wie insbesondere Ester oder Amide davon, entsprechend dem Fall, worin R¹² Alkoxy oder gegebenenfalls substituiertes Amino ist, oder Derivate oder Polyaminosäuren davon, welche durch peptidische Verknüpfung mit einer oder mehreren weiteren Aminosäuren an die vorstehend oder nachstehend genannten Aminosäuren hervorgehen.

Weitere Aminosäure-Verbindungen bzw. Derivate davon, aus denen formal durch Abspaltung einer Aminogruppe ein Rest R¹⁰ oder R¹¹ resultiert schließen ein: Kreatin (weniger bevorzugt), Kreatinin, Taurin, oder Derivate oder Polyaminosäuren davon, welche durch peptidische Verknüpfung mit einer oder mehreren weiteren Aminosäuren an die vorstehend oder nachstehend genannten Aminosäuren hervorgehen. Weiterhin sind auch sogenannte Nicht-Proteinogene Aminosäuren eingeschlossen, wie zum Beispiel: 4-Aminobuttersäure (GABA), L-Homoserin (2-Amino-4-hydroxybuttersäure), Ornithin (2,5-Diaminovaleriansäure), L-(+)-Citrullin (N5-(Aminocarbonyl)-L-ornithin), 5-Hydroxytryptophan (5-HTP), β-Alanin (3-Aminopropionsäure), β-Methylamino-Alanin, D-Valin, D-Alanin, D-Glutaminsäure und 2,6-Diaminopimelinsäure.

Die Derivate der vorstehend genannten Aminosäure-Verbindungen H₂N-A sind insbesondere solche, die durch Austausch eines Wasserstoffatoms gegen eine Hydroxylfunktion hervorgegangen sind.

Sehr bevorzugt sind erfindungsgemäße Verbindungen, worin der Rest A sich formal durch Abspaltung der H₂N-Gruppe aus der Gruppe der Aminosäuren Glycin und dessen Derivate und Histidin und dessen Derivaten ableitet.

Am meisten bevorzugt sind erfindungsgemäße Verbindungen, worin der Rest A sich durch Abspaltung der H₂N-Gruppe aus der Gruppe der folgenden Aminosäuren oder Aminosäurederivate ableitet:
Glycin: 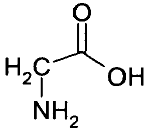 entsprechend R¹⁰ oder R¹¹ = 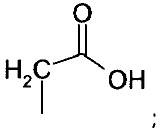
Glycinamid: 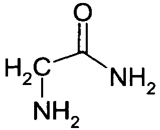 (2-Amino-acetamid), entsprechend R¹⁰ oder 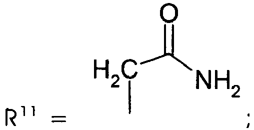
Glycinethylester: 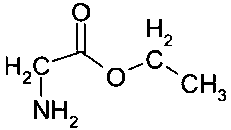 (Amino-essigsäureethylester), entsprechend R¹⁰ oder R¹¹ = 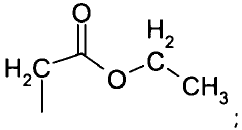
Histidin: 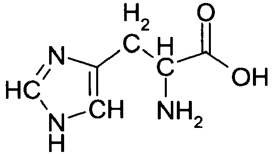 entsprechend R¹⁰ oder R¹¹ = 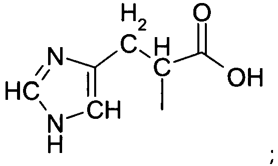 oder das Histidinamid: 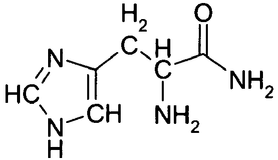 (2-Amino-3-(1H-imidazol-4-yl)-propionamid), entsprechend R¹⁰ oder R¹¹ =

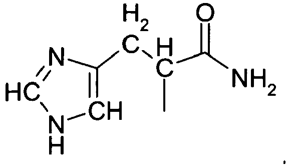

Mit Ausnahme des Glycins weisen alle Aminosäuren asymmetrische Kohlenstoffatome auf. Die erfindungsgemäßen Verbindungen, in denen R¹⁰ oder R¹¹ ein Rest A ist, welcher formal durch Abspaltung einer Aminogruppe aus einer natürlichen Aminosäure hervorgeht, weist daher die natürliche Konfiguration (L-Konfiguration) der Aminosäure auf. Dies gilt auch für die Verbindungen, die sich formal durch Abspaltung einer Aminogruppe aus einem Aminosäurederivat, einer Polyaminosäuren und Polyaminosäurederivaten. Erfindungsgemäß ist jedoch auch der Fall eingeschlossen, in denen die Aminosäuren die nicht-natürliche D-Konfiguration, wie D-Alanin, D-Glutaminsäure etc. aufweisen.

Erfindungsgemäß sind die Verbindungen, worin die Aminosäuren H₂N-A die L-Konfiguration aufweisen, bzw. worin R¹⁰ oder R¹¹ den Rest A einer solchen Aminosäure darstellen, und solche Verbindungen bevorzugt, worin sich die zugrundeliegenden Aminosäurederivate, die Polyaminosäuren und die Polyaminosäurederivate von Aminosäuren H₂N-A ableiten, die die L-Konfiguration aufweisen.

Im Hinblick auf ihre bessere Wasserlöslichkeit sind des weiteren Verbindungen bevorzugt, bei denen der Rest R¹⁰ oder R¹¹ formal durch Abspaltung einer NH₂-Gruppe aus einer sauren Aminosäure mit mindestens zwei Carboxylgruppen wie Asparaginsäure, Glutaminsäure hervorgeht. Auch die Verwendung von Hydroxyl-gruppen-enthaltenden Aminosäuren wie beispielsweise Threonin kann unter diesem Aspekt bevorzugt sein.

### Alternative 2:

In vorstehend erwähnter Alternative 2), worin R¹⁰ und R¹¹ jeweils Alkyl sind, worin mindestens eine der Alkylgruppen mindestens einen, bevorzugt ein oder zwei Substituenten aufweist, kann hinsichtlich der Definitionen bzw. Beispiele für Alkyl zu den oben in Alternative 1) angegebenen verwiesen werden. Substituenten an Alkyl schließen somit vorstehende für "gegebenenfalls substituiertes Alkyl" gegebene Beispiele ein, wie Hydroxy, Halogen und Cyano. Zusätzlich schließen mögliche Substituenten von Alkyl in der Alternative 2) auch die für R¹⁰ und R¹¹ in vorstehend beschriebener Alternative 1) gegebenen Beispiele ein, wie
- Guanidino,
- Thiol (-SH),
- Alkylthio, wie insbesondere Methylthio,
- Amino (-NH₂)
- Mono- oder Dialkylamino,
- Acylamino, worin Acyl insbesondere wie oben definiert ist,
- gesättigte, ungesättigte oder aromatische, mono- oder bicyclische, gegebenenfalls substituierte heterocyclische Reste, wie beispielsweise vorstehend erwähnte gegebenenfalls substituierte heteroaromatische Reste, bevorzugt Imidazolyl, wie Imidazol-5-yl, 1 H-Indolyl, wie 1H-Indol-3-yl,
- gegebenenfalls substituiertes Aryl, wie oben beschrieben, insbesondere Phenyl, Hydroxyphenyl, wie 4-Hydroxyphenyl, Alkoxyphenyl, wie Methoxyphenyl,
- Hydroxy,
- Alkoxy, wie vorstehend beschrieben, und
- eine Gruppe der Formel:

   -X²-R¹² ,

   worin X² und R¹² wie oben definiert sind,
sowie insbesondere die Reste, welche formal aus der Abspaltung der NH₂-Gruppe aus den Aminosäuren NH₂-A resultieren.

### Alternative 3):

In vorstehend erwähnter Alternative 3), worin R¹⁰ und R¹¹ zusammen mit dem Stickstoffatom, an das sie gebunden sind, einen gesättigten oder ungesättigten, gegebenenfalls substituierten 5 bis 8-gliedrigen Cyclus ausbilden, der gegebenenfalls weitere Heteroatome enthalten kann, schließen mögliche Ringsysteme, die aus R¹⁰ und R¹¹ und dem Stickstoffatom, an das sie gebunden sind, bestehen, bevorzugt 5- oder 6-gliedrige, gegebenenfalls substituierte Ringe ein, wie Piperidin-1-yl, Morpholin-4-yl, Thiomorpholin-4-yl, Pyrrolidin-1-yl, Oxazolidin-3-yl, Thiazolidin-3-yl, 2-Carboxyl-pyrrolidin-1-yl (Prolyl), 3- oder 4-Hydoxy-carboxyl-pyrrolidin-1-yl (3- oder 4-Hydroxy-prolyl) etc. Besonders bevorzugt sind hier Prolyl bzw. Hydroxy-prolyl.

Im Rahmen der vorliegenden Erfindung sind insbesondere Verbindungen bevorzugt, worin Z¹ die Bedeutung von Hydroxy (-OH) aufweist, oder pharmazeutisch verträgliche Salze davon, wie insbesondere Natrium, Kalium oder Calciumsalze, bevorzugt.

Erfindungsgemäß besonders bevorzugte Isocyanat- und Isothiocyanat-Verbindungen sind solche, worin R¹, R², R³, R⁴, R⁵, R⁶, R⁷ und R⁸ jeweils Wasserstoff sind.

Erfindungsgemäß besonders bevorzugte Isocyanat- und Isothiocyanat-Verbindungen sind solche, worin X¹ Carbonyl ist.

Erfindungsgemäß besonders bevorzugte Isocyanat- und Isothiocyanat-Verbindungen sind solche, worin R⁹ ein lineares, verzweigtes oder cyclisches Alkandiyl, bevorzugt lineares Alkandiyl mit 1 bis 6 Kohlenstoffatomen, besonders bevorzugt Methylen (-CH₂-) oder Ethan-1,2-diyl oder 1,3-Propandiyl oder 1,2-Isopropandiyl ist.

Erfindungsgemäß besonders bevorzugte Isocyanat- und Isothiocyanat-Verbindungen sind solche, worin Z¹ Hydroxy ist.

Erfindungsgemäß besonders bevorzugte Isocyanat- und Isothiocyanat-Verbindungen sind solche, worin die Reste

X¹ und der Rest 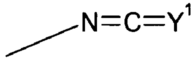 am Phenylenrest para zueinander stehen.

Erfindungsgemäß besonders bevorzugte Isocyanat- und Isothiocyanat-Verbindungen sind solche, worin die Reste

X¹ und der Rest 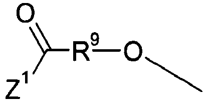 am Phenylenrest para zueinander stehen.

Besonders bevorzugt sind erfindungsgemäß Verbindungen der Formel (2): 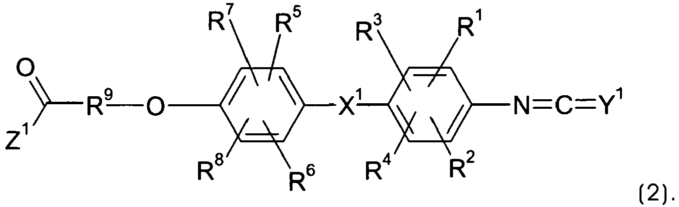 Besonders bevorzugt sind erfindungsgemäß Verbindungen der Formeln (1) oder (2), worin
Y¹ = S oder O ist,
R¹, R², R³, R⁴, R⁵, R⁶, R⁷ und R⁸ jeweils Wasserstoff sind,
R⁹ geradkettiges, verzweigtes oder cyclisches Alkandiyl ist,
Z¹ Hydroxy oder ein Rest der Formel -NR¹⁰R¹¹ ist, worin
R¹⁰ Wasserstoff ist und R¹¹ ein Rest A einer Verbindung der Formel H₂N-A oder R¹¹ Wasserstoff ist und R¹⁰ ein Rest A einer Verbindung der Formel H₂N-A ist, worin
A jeweils ein Rest ist der sich durch Abspaltung der Aminogruppe (-NH₂) aus einer natürlichen oder synthetischen Aminosäure, einem natürlichen oder synthetischen Aminosäurederivat oder einer Polyaminosäure oder einem Polyaminosäurederivat ableitet, und
X¹ Carbonyl (-CO-) ist.

Ganz besonders bevorzugt sind erfindungsgemäß Verbindungen der Formeln (1) oder (2), worin
Y¹ = S oder O ist,
R¹, R², R³, R⁴, R⁵, R⁶, R⁷ und R⁸ jeweils Wasserstoff sind,
R⁹ geradkettiges, verzweigtes oder cyclisches Alkandiyl ist,
Z¹ Hydroxy ist, und
X¹ Carbonyl (-CO-) ist.

Am meisten bevorzugt sind die Verbindungen, die aus der Gruppe ausgewählt werden, wie in den nachfolgenden Beispielen 1 bis 70 dargestellt, oder pharmazeutisch verträgliche Salze davon, insbesondere deren Natriumsalze.

(In diesen Strukturformeln bedeutet dabei beispielsweise ein Strukturelement der Formel 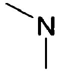 eine Dimethylaminogruppe, d.h. die Methylgruppen sind durch einfache Striche dargestellt, eine Schreibweise, die dem Fachmann gut bekannt ist. Analog stellt 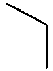 eine verkürzte Schreibweise eines Methylenrestes (-CH₂-) dar).

Erfindungsgemäß sind weiterhin Verbindungen der Formel (1) bevorzugt, worin
R¹⁰ Wasserstoff ist und R¹¹ substituiertes Alkyl ist, oder
R¹¹ Wasserstoff ist und R¹⁰ substituiertes Alkyl ist, worin
substituiertes Alkyl eine Alkylgruppe ist, welche mindestens einen Sulfonsäurerest, Sulfonsäureesterrest oder einen Sulfonamidorest aufweist. Besonders bevorzugt sind in diesem Zusammenhang Verbindungen in denen substituiertes Alkyl in der Definition von R¹⁰ oder R¹¹ ein Rest der Formel: 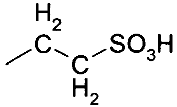 ist, welcher sich aus Taurin (2-Aminoethansulfonsäure) ableitet.

Erfindungsgemäße Isothiocyanat-Verbindungen, die basische Gruppen enthalten, können in Form ihrer pharmazeutisch annehmbaren Salze mit pharmazeutisch annehmbaren Säuren angewendet werden, wie z.B. Salze mit Mineralsäuren, Carbonsäuren und Sulfonsäuren, wie zum Beispiel mit Salzsäure, Bromwasserstoffsäure, Iodwasserstoffsäure, Schwefelsäure, Phosphorsäure, Weinsäure, Methansulfonsäure, Hydroxyethansulfonsäure, Acetursäure (Acetylglycin), Maleinsäure, Propionsäure, Fumarsäure, Toluolsulfonsäure, Benzolsulfonsäure, Trifluoressigsäure, Naphthalindisulfonsäure-1,5, Salicylsäure, Benzoesäure, Milchsäure, Äpfelsäure, 3-Hydroxy-2-naphthoesäure-2, Zitronensäure oder Essigsäure.

Erfindungsgemäße Isothiocyanat-Verbindungen, die saure Gruppen enthalten, können in Form ihrer pharmazeutisch annehmbaren Salze mit pharmazeutisch annehmbaren Basen angewendet werden, wie z.B. Salze mit Alkali- oder Erdalkalihydroxiden, wie NaOH, KOH, Ca(OH)₂, Mg(OH)₂ etc., Aminverbindungen, wie Ethylamin, Diethylamin, Triethylamin, Ethyldiisopropylamin, Ethanolamin, Diethanolamin, Triethanolamin, Methylglucamin, Dicyclohexylamin, Dimethylaminoethanol, Prokain, Dibenzylamin, N-Methylmorpholin, Arginin, Lysin, Ethylendiamin, N-Methylpiperidin 2-Amino-2-methyl-propanol-(1), 2-Amino-2-methyl-propandiol-(1,3), 2-Amino-2-hydroxyl-methyl-propandiol-(1,3) (TRIS) etc..

Die Wasserlöslichkeit bzw. die Löslichkeit in physiologischer Kochsalzlösung und damit gegebenenfalls auch die Wirksamkeit der erfindungsgemäßen Verbindungen kann durch Salzbildung generell, speziell auch durch die Wahl des Gegenions signifikant beeinflusst werden.

Die Wasserlöslichkeit bzw. die Löslichkeit in physiologischer Kochsalzlösung und damit gegebenenfalls auch die Wirksamkeit der erfindungsgemäßen Verbindungen hängt aber unter Umständen auch von der Grundstruktur der Verbindungen selbst signifikant ab. So kann durch Variation der Struktur der Substituentengruppe R⁹ die Wasserlöslichkeit und damit die physiologische Verträglichkeit gezielt variiert werden.

Eine hohe Wasserlöslichkeit der erfindungsgemäßen Verbindungen ist nicht unbedingt entscheidend, da in der Blutbahn wahrscheinlich ohnehin der überwiegende Anteil der Substanz proteingebunden vorliegt. Von Bedeutung ist im allgemeinen eher, dass die Substanzen als Substrat für ein körpereigenes Transportsystem erkannt werden. Im Zusammenhang mit der vorliegenden Erfindung sind dabei vermutlich insbesondere die sogenannten OATs (organic anion transporters) und OATPs (organic anion transporter proteins) von Bedeutung. Diese haben aber keine 100% Spezifität für Anionen. Beispiel hierfür ist das Digitoxin. Ebenso können auch Peptidtransporter als relevante Aufnahme- und Ausscheidungsmechanismen die den Aminosäure bzw. Aminosäureamid-Rest erkennen, diskutiert werden.

Die Verwendung von 2-Amino-2-hydroxyl-methyl-propandiol-(1,3) (TRIS)- und Natrium-Salzen ist vor dem Hintergrund der Erhöhung der Wasserlöslichkeit der erfindungsgemäßen Verbindungen bevorzugt.

Die erfindungsgemäßen Verbindungen können in Abhängigkeit von ihrer Struktur bei Vorliegen asymmetrischer Kohlenstoffatome in stereoisomeren Formen (Enantiomere, Diastereomere) existieren. Die Erfindung umfasst deshalb die Verwendung der Enantiomeren oder Diastereomeren und ihrer jeweiligen Mischungen. Die enantiomerenreinen Formen können gegebenenfalls durch übliche Verfahren der optischen Auflösung, wie durch fraktionierte Kristallisation von Diastereomeren daraus durch Umsetzung mit optisch aktiven Verbindungen erhalten werden. Sofern die erfindungsgemäßen Verbindungen in tautomeren Formen vorkommen können, umfasst die vorliegende Erfindung die Verwendung sämtlicher tautomerer Formen.

Die vorliegende Erfindung betrifft weiterhin Verbindungen der Formel (1) zur Verwendung als Arzneimittel sowie die Verwendung der Verbindungen der Formel (1) zur Herstellung eines Arzneimittels, insbesondere zur Behandlung von Krebserkrankungen.

Die erfindungsgemäßen Verbindungen können beispielsweise zur Behandlung folgender Tumorarten verwendet werden: Adenokarzinom, Aderhautmelanom, Akuter Leukämie, Akustikusneurinom, Ampullenkarzinom, Analkarzinom, Astrozytome, Basaliom, Bauchspeicheldrüsenkrebs, Bindegewebstumor, Blasenkrebs, Bronchialkarzinom, Nicht-kleinzelliges Bronchialkarzinom, Brustkrebs, Burkitt-Lymphom, Corpuskarzinom, CUP-Syndrom, Dickdarmkrebs, Dünndarmkrebs, Dünndarmtumore, Eierstockkrebs, Endometriumkarzinom, Ependymom, Epithel-Krebsarten, Ewing-Tumoren, Gastrointestinale Tumoren, Gallenblasenkrebs, Gallenkarzinome, Gebärmutterkrebs, Gebärmutterhalskrebs, Glioblastome, Gynäkologische Tumoren, Hals-, Nasen- und Ohrentumoren, Hämatologische Neoplasien, Haarzell-Leukämie, Harnröhrenkrebs, Hautkrebs, Hirntumoren (Gliome), Hirnmetastasen, Hodenkrebs, Hypophysentumor, Karzinoide, Kaposi-Sarkom, Kehlkopfkrebs, Keimzellentumor, Knochenkrebs, kolorektales Karzinom, Kopf-Hals-Tumore (Tumore des Hals- Nasen- und Ohrenbereichs), Kolonkarzinom, Kraniopharyngeome, Krebs im Mundbereich und auf der Lippe, Leberkrebs, Lebermetastasen, Leukämie, Lidtumor, Lungenkrebs, Lymphdrüsenkrebs (Hodgkin/Non-Hodgkin), Lymphome, Magenkrebs, Malignes Melanom, malignes Neoplasma, Malignome des Magen-Darm-Traktes, Mammakarzinom, Mastdarmkrebs, Medulloblastome, Melanom, Meningeome, Merkelzell Karzinom, Morbus Hodgkin, Mycosis fungoides, Nasenkrebs, Neurinom, Neuroblastom, Nierenkrebs, Nierenzellkarzinome, Non-Hodgkin-Lymphome, Oligodendrogliom, Ösophaguskarzinom, osteolytische Karzinome und osteoplastische Karzinome, Osteosarkom, Ovarial-Karzinom, Pankreaskarzinom, Peniskrebs, Plasmozytom, Plattenepithelkarzinome des Kopfes und Halses, Prostatakrebs, Rachenkrebs, Rektumkarzinom, Retinoblastom, Scheidenkrebs, Schilddrüsenkarzinom, Schneeberger Krankheit, Speiseröhrenkrebs, Spinaliom, T-Zell-Lymphom (Mycosis fungoides), Thymom, Tubenkarzinom, Tumoren des Auges, Urethrakrebs, Urologische Tumoren, Urothelkarzinom, Vulvakrebs, Warzenbeteiligung, Weichteiltumoren, Weichteilsarkom, Wilms Tumor, Zervixkarzinom und Zungenkrebs. Ergänzend kann auch auf die Aufzählung der Krebsarten beispielsweise in WO2007061978 (Seite 16, Zeile 22 bis Seite 18, Zeile 2) oder in der US20071 35424A1 (Seite 9, linke Spalte, Abschnitt 122) verwiesen werden, welche zum Offenbarungsgehalt der vorliegenden Anmeldung zu rechnen sind. Die Verbindungen der vorliegenden Erfindung können auch in weiteren Indikationen verwendet werden, wie den in der US20071 35424A1 in den Abschnitten 123 bis 142 Genannten.

Besonders bevorzugt werden die erfindungsgemäßen Verbindungen zur Behandlung von Brustkrebs, Darmkrebs oder Melanomen eingesetzt.

Besonders bevorzugt werden die erfindungsgemäßen Verbindungen zur Behandlung von Brustkrebs eingesetzt.

Die Erfindung betrifft weiterhin die Verwendung der Verbindungen der Formel (1) in Kombination mit mindestens einem weiteren Chemotherapeutika zur Behandlung von Krebs.

Die Verbindungen der vorliegenden Erfindung können demnach auch in Kombination mit weiteren in der Behandlung von Krebs bzw. Tumoren bekannten Chemotherapeutika und/oder mit Arzneimitteln, die begleitend mit den Chemotherapeutika während einer Chemotherapie verabreicht werden, verwendet werden. Beispiele solcher in Kombination anwendbarer Chemotherapeutika und weiterer in der Chemotherapie eingesetzter Arzneimittel finden sich beispielsweise in der WO2007061978 unter dem Stichwort "Combination Therapy" (Seite 23, Zeile 1 bis Seite 30, Zeile 18) oder in der US2007135424A1 (Abschnitte 1 53 bis 171), auf welche auch insoweit vollinhaltlich Bezug genommen wird.

Die vorliegende Erfindung betrifft weiterhin pharmazeutische Zusammensetzungen, enthaltend mindestens eine der Verbindungen der Formel (1) zusammen mit mindestens einem pharmakologisch verträglichen Träger, Hilfsstoff oder Lösungsmittel. Dabei handelt es sich um übliche pharmazeutische Träger, Hilfsstoff oder Lösungsmittel. Genannte pharmazeutische Zusammensetzungen sind beispielsweise geeignet zur Inhalation oder zur intravenösen, intraperitonealen, intramuskulären, intravaginalen, intrabuccalen, perkutanen, subkutanen, mucokutanen, oralen, rektalen, transdermalen, topikalen, intradermalen, intragastralen oder intrakutanen Applikation und liegen beispielsweise in der Form von Pillen, Tabletten, magensaftresistente Tabletten, Filmtabletten, Schichttabletten, Retardformulierungen zur oralen, subkutanen oder kutanen Verabreichung (insbesondere als Pflaster), Depotformulierung, Dragees, Zäpfchen, Gelen, Salben, Sirup, Inhalationspulvern, Granulaten, Suppositorien, Emulsionen, Dispersionen, Mikrokapseln, Mikroformulierungen, Nanoformulierungen, liposomalen Formulierungen, Kapseln, magensaftresistente Kapseln, Pulver, Inhalationspulvern, mikrokristallinen Formulierungen, Inhalationssprays, Puder, Tropfen, Nasentropfen, Nasensprays, Aerosolen, Ampullen, Lösungen, Säfte, Suspensionen, Emulsionen, Infusionslösungen oder Injektionslösungen etc. vor.

Die erfindungsgemäßen Verbindungen können in pharmazeutischen Zusammensetzung verabreicht werden, die verschiedene organische oder anorganische Träger- und/oder Hilfsmaterialien enthalten können, wie sie üblicherweise für pharmazeutische Zwecke insbesondere für feste Arzneimittelformulierungen verwendet werden, wie beispielsweise Exzipienten (wie Saccharose, Stärke, Mannit, Sorbit, Lactose, Glucose, Cellulose, Talk, Calciumphosphat, Calciumcarbonat), Bindemittel (wie Cellulose, Methylcellulose, Hydroxypropylcellulose, Polypropylpyrrolidon, Gelatine, Gummiarabicum, Polyethylenglykol, Saccharose, Stärke), Desintegrationsmittel (wie Stärke, hydrolysierte Stärke, Carboxymethylcellulose, Calciumsalz von Carboxymethylcellulose, Hydroxypropylstärke, Natriumglycolstärke, Natriumbicarbonat, Calciumphosphat, Calciumcitrat), Gleit- bzw. Schmiermittel (wie Magnesiumstearat, Talk, Natriumlaurylsulfat), ein Geschmacksbildner (wie Citronensäure, Menthol, Glycin, Orangenpulver), Konservierungsmtitel (wie Natriumbenzoat, Natriumbisulfit, Methylparaben, Propylparaben), Stabilisatoren (wie Citronensäure, Natriumcitrat, Essigsäure, und Multicarbonsäuren aus der Titriplex Reihe wie z.B. Diethylentriaminpentaessigsäure (DTPA), Suspendiermittel (wie Methylcellulose, Polyvinylpyrrolidon, Aluminiumstearat), Dispergiermittel, Verdünnungsmittel (wie Wasser, organische Lösungsmittel), Bienenwachs, Kakaobutter, Polyethylenglykol, weißes Petrolatum etc..

Flüssige Arzneimittelformulierungen, wie Lösungen, Suspensionen und Gele enthalten üblicherweise einen flüssigen Träger, wie Wasser und/oder pharmazeutisch verträgliche organische Lösungsmittel. Weiterhin können derartige flüssigen Formulierungen auch pH-einstellende Mittel, Emulgatoren oder dispergierende Agenzien, puffernde Agenzien, Konservierungsmittel, Netzmittel, Geliermittel (beispielsweise Methylcellulose), Färbemittel und/oder Aromastoffe enthalten. Die Zusammensetzungen können isotonisch sein, das heißt diese können den gleichen osmotischen Druck wie Blut haben. Die Isotonie der Zusammensetzung kann durch die Verwendung von Natriumchlorid oder anderer pharmazeutisch annehmbare Agenzien wie beispielsweise Dextrose, Maltose, Borsäure, Natriumtartrat, Propylenglykol oder andere anorganische oder organisch lösliche Substanzen eingestellt werden. Die Viskosität der flüssigen Zusammensetzungen kann unter Verwendung eines pharmazeutisch annehmbaren Verdickungsmittels, wie Methylcellulose eingestellt werden. Andere geeignete Verdickungsmittel umfassen beispielsweise Xanthan, Carboxymethylcellulose, Hydroxypropylcellulose, Carbomer und dergleichen. Die bevorzugte Konzentration des Verdickungsmittels wird von dem ausgewählten Agens abhängen. Pharmazeutisch annehmbare Konservierungsmittel können verwendet werden, um die Haltbarkeit der flüssigen Zusammensetzung zu erhöhen. Benzylalkohol kann geeignet sein, obwohl eine Vielzahl von Konservierungsmitteln einschließlich beispielsweise Paraben, Thimerosal, Chlorbutanol oder Benzalkoniumchlorid ebenfalls verwendet werden können.

Als Stabilisierungsmittel für die pharmazeutischen festen oder flüssigen Formulierungen der erfindungsgemäßen Verbindungen, wie insbesondere der Verbindung von Beispiel 2a), erweist sich insbesondere die Diethylentriaminpentaessigsäure (DTPA).

Der Wirkstoff kann beispielsweise mit einer Einheitsdosis von 0,01 mg/kg bis 500 mg/kg Körpergewicht beispielweise bis zu 1 bis 4 mal am Tag verabreicht werden. Die Dosierung kann jedoch je nach Alter, Gewicht, Zustand des Patienten, Schwere der Erkrankung oder Art der Verabreichung erhöht oder herabgesetzt werden.

Die Erfindung wird durch die folgenden Beispiele näher veranschaulicht. Die Beispiele stellen lediglich Exemplifizierungen dar, und der Fachmann ist in der Lage die spezifischen Beispiele auf weitere beanspruchte Verbindungen auszudehnen.

### BEISPIELE:

Gegenstand der Erfindung sind insbesondere die folgenden Beispielverbindungen:

| **Nr** | **Isothlocyanat-Verbindung** | **Nr** | **Isocyanat-Verbindung** |
|---|---|---|---|
| 1a | 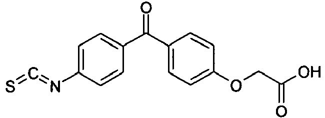 | 1b | 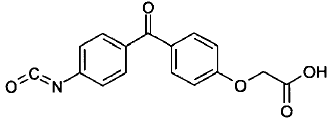 |
| 2a | 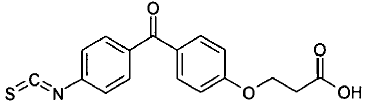 | 2b | 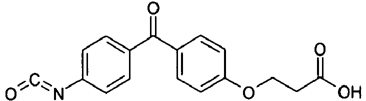 |
| 3a | 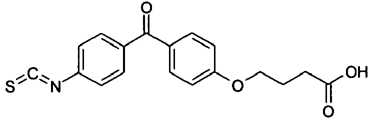 | 3b | 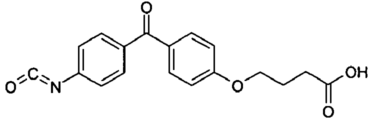 |
| 4a | 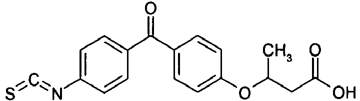 | 4b | 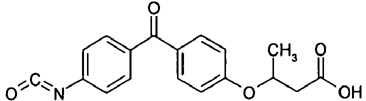 |
| 5a | 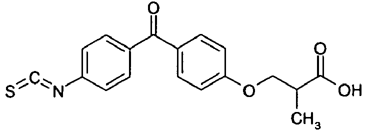 | 5b | 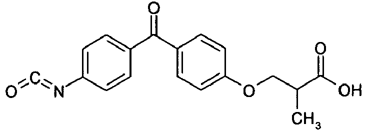 |
| 6a | 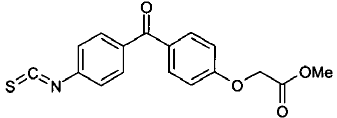 | 6b | 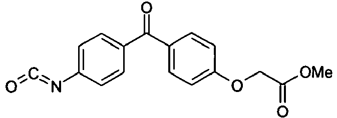 |
| 7a | 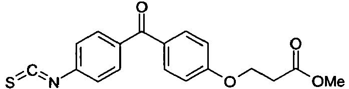 | 7b | 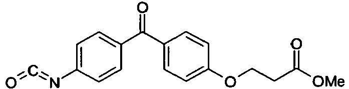 |
| 8a | 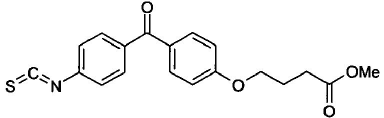 | 8b | 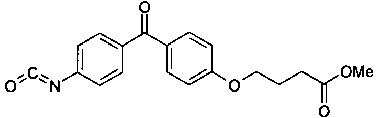 |
| 9a | 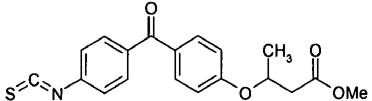 | 9b | 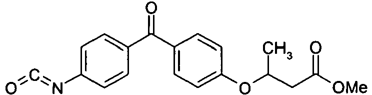 |
| 10 a | 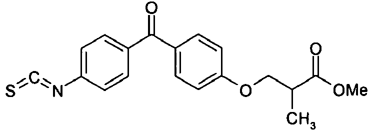 | 10 b | 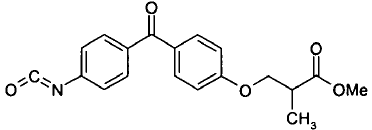 |
| 11 a | 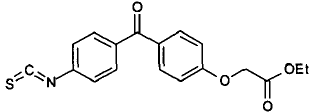 | 11 b | 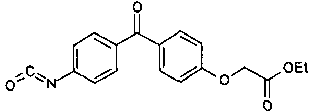 |
| 12 a | 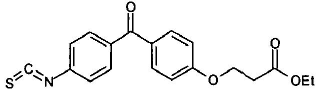 | 12 b | 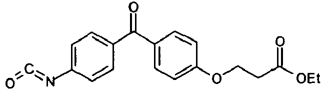 |
| 13 a | 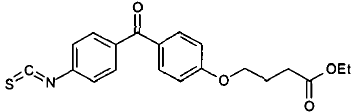 | 13 b | 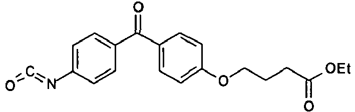 |
| 14 a | 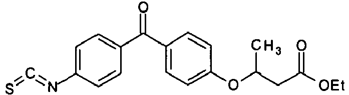 | 14 b | 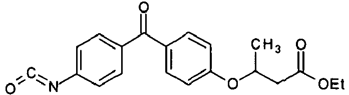 |
| 15 a | 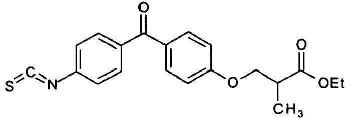 | 15 b | 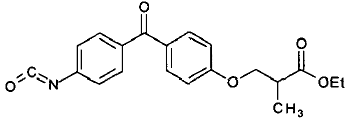 |
| 16 a | 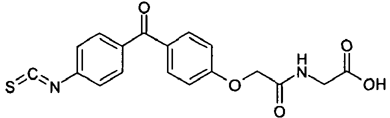 | 16 b | 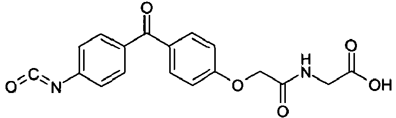 |
| 17 a | 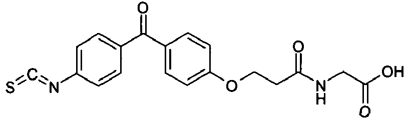 | 17 b | 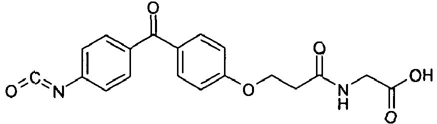 |
| 18 a | 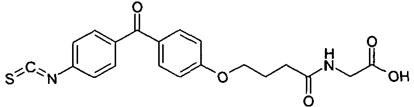 | 18 b | 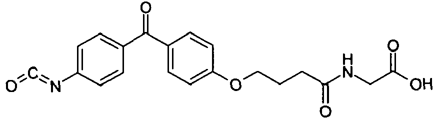 |
| 19 a | 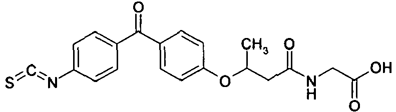 | 19 b | 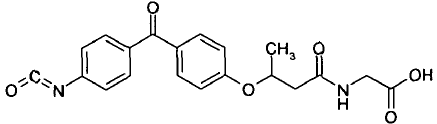 |
| 20 a | 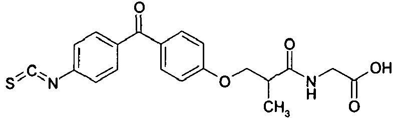 | 20 b | 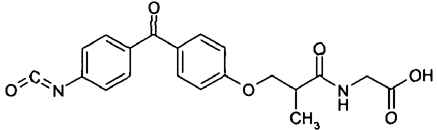 |
| 21 a | 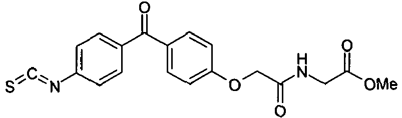 | 21 b | 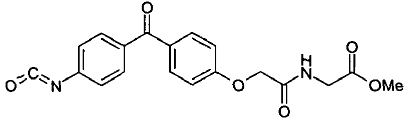 |
| 22 a | 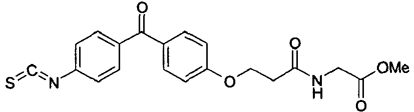 | 22 b | 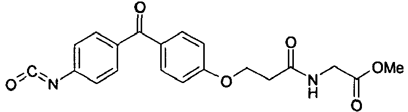 |
| 23 a | 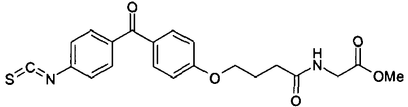 | 23 b | 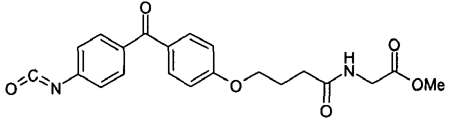 |
| 24 a | 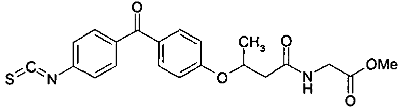 | 24 b | 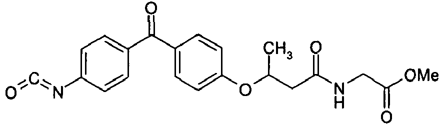 |
| 25 a | 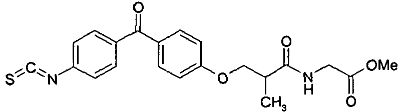 | 25 b | 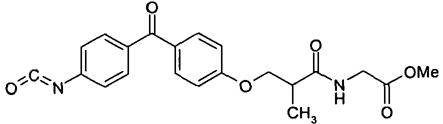 |
| 26 a | 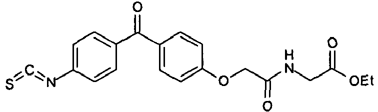 | 26 b | 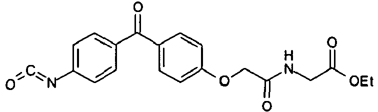 |
| 27 a | 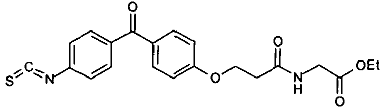 | 27 b | 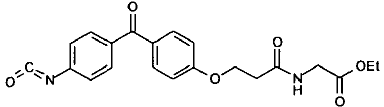 |
| 28 a | 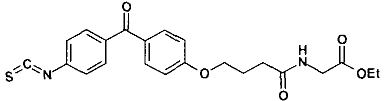 | 28 b | 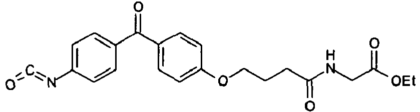 |
| 29 a | 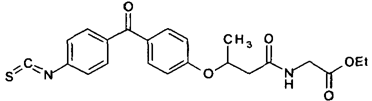 | 29 b | 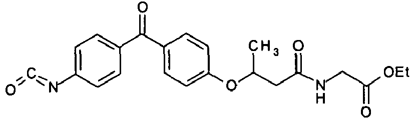 |
| 30 a | 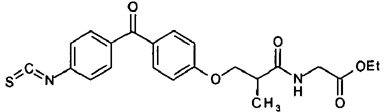 | 30 b | 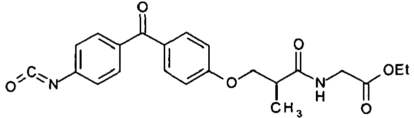 |
| 31 a | 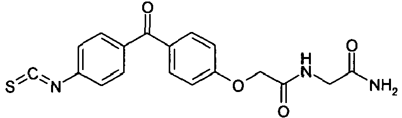 | 31 b | 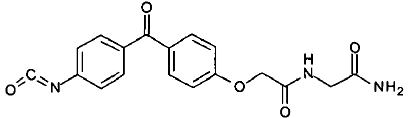 |
| 32 a | 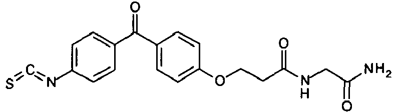 | 32 b | 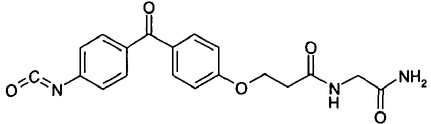 |
| 33 a | 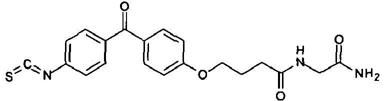 | 33 b | 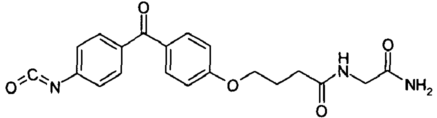 |
| 34 a | 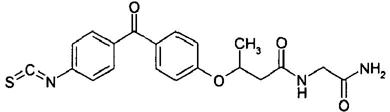 | 34 b | 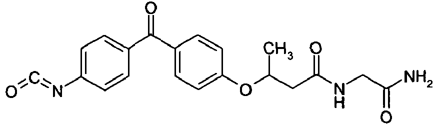 |
| 35 a | 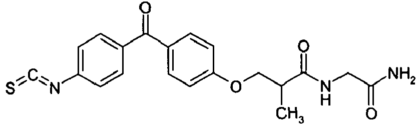 | 35 b | 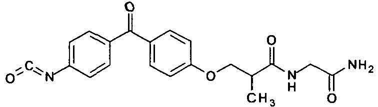 |
| 36 a | 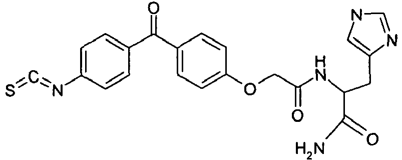 | 36 b | 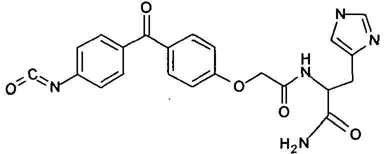 |
| 37 a | 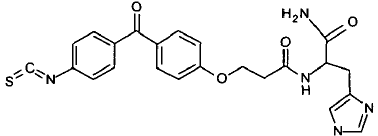 | 37 b | 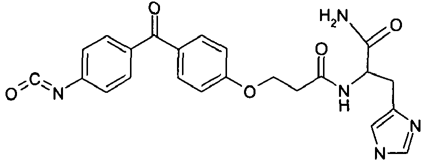 |
| 38 a | 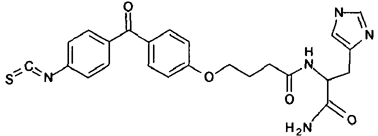 | 38 b | 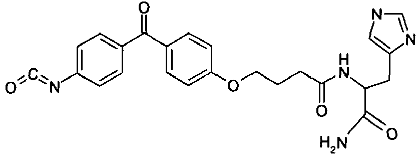 |
| 39 a | 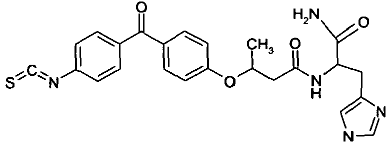 | 39 b | 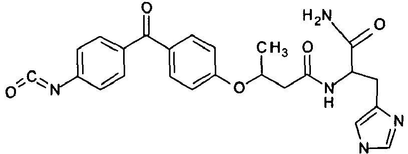 |
| 40 a | 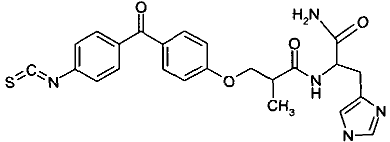 | 40 b | 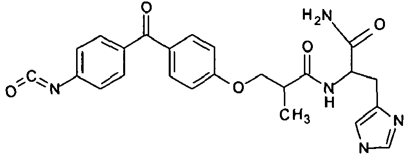 |
| 41 a | 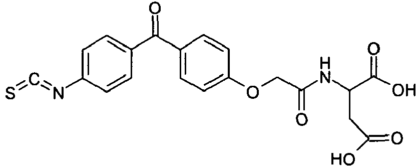 | 41 b | 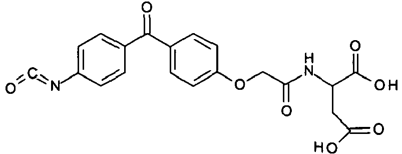 |
| 42 a | 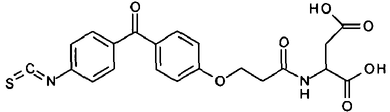 | 42 b | 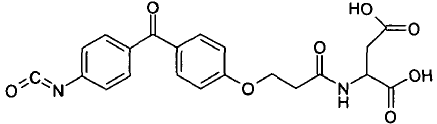 |
| 43 a | 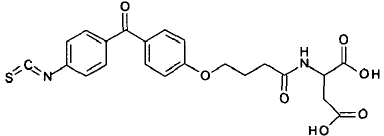 | 43 b | 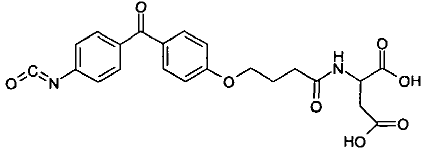 |
| 44 a | 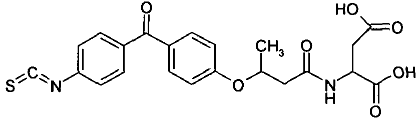 | 44 b | 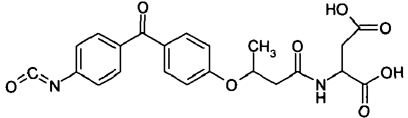 |
| 45 a | 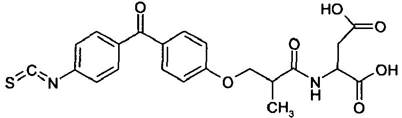 | 45 b | 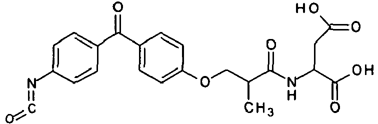 |
| 46 a | 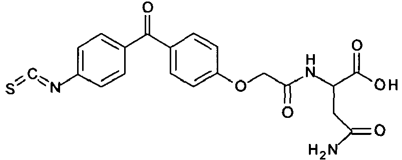 | 46 b | 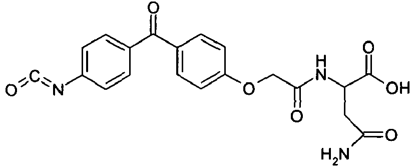 |
| 47 a | 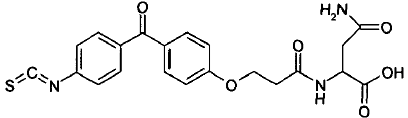 | 47 b | 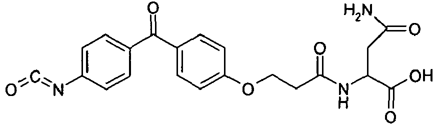 |
| 48 a | 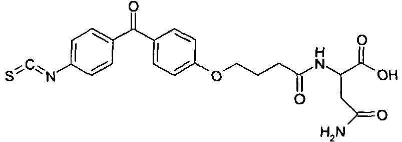 | 48 b | 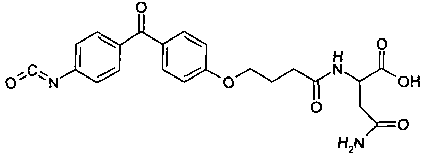 |
| 49 a | 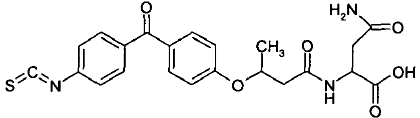 | 49 b | 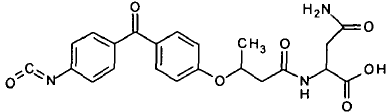 |
| 50 a | 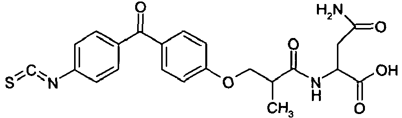 | 50 b | 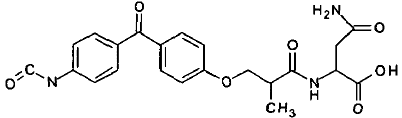 |
| 51 a | 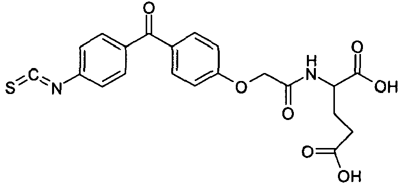 | 51 b | 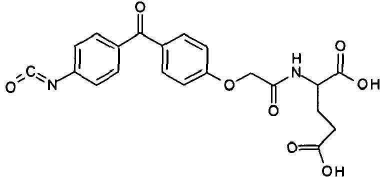 |
| 52 a | 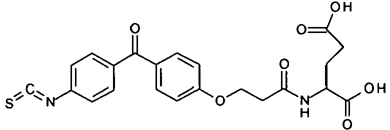 | 52 b | 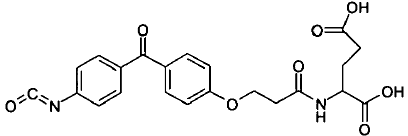 |
| 53 a | 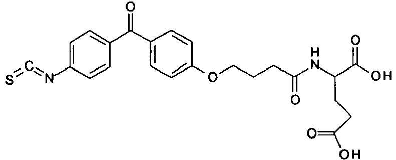 | 53 b | 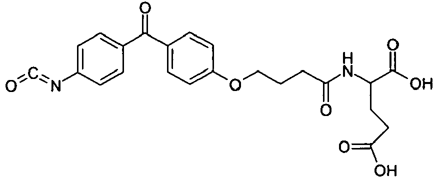 |
| 54 a | 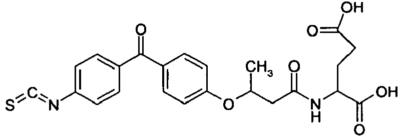 | 54 b | 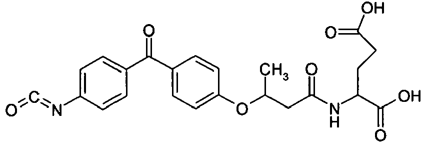 |
| 55 a | 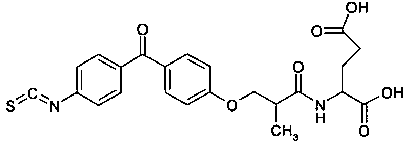 | 55 b | 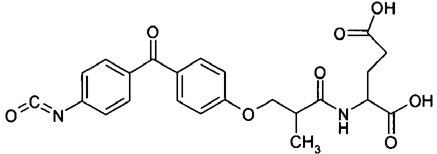 |
| 56 a | 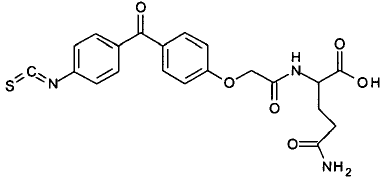 | 56 b | 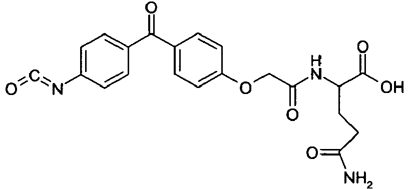 |
| 57 a | 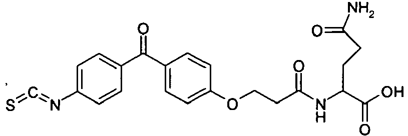 | 57 b | 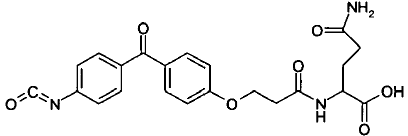 |
| 58 a | 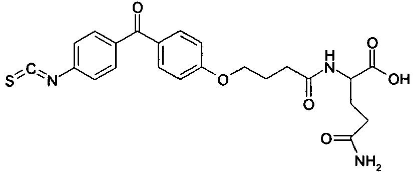 | 58 b | 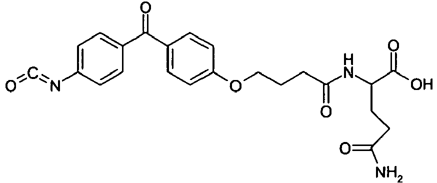 |
| 59 a | 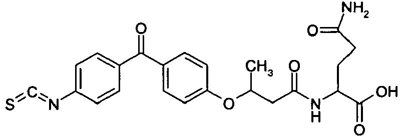 | 59 b | 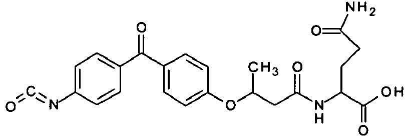 |
| 60 a | 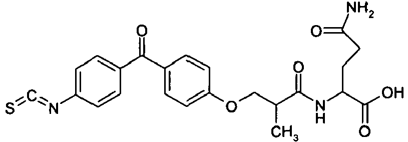 | 60 b | 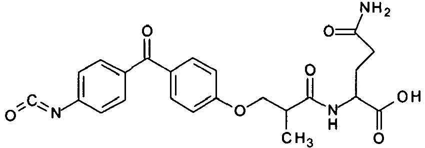 |
| 61 a | 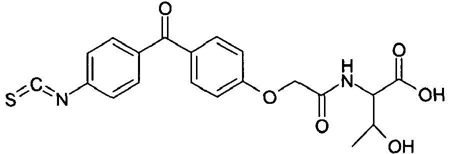 | 61 b | 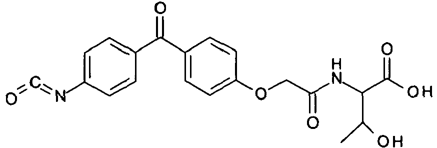 |
| 62 a | 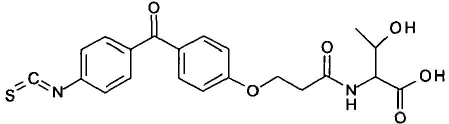 | 62 b | 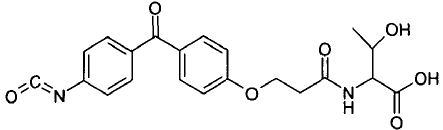 |
| 63 a | 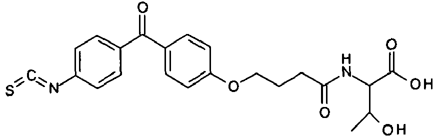 | 63 b | 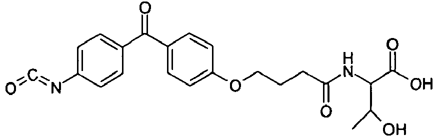 |
| 64 a | 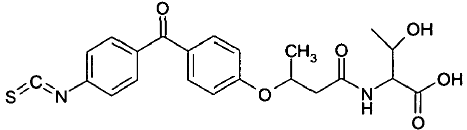 | 64 b | 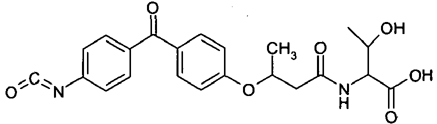 |
| 65 a | 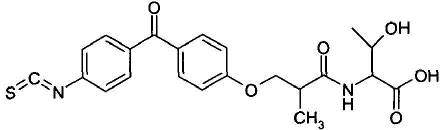 | 65 b | 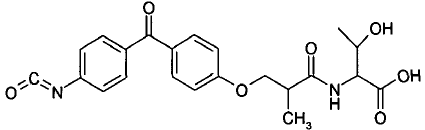 |
| 66 a | 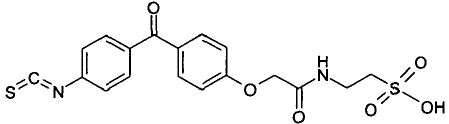 | 66 b | 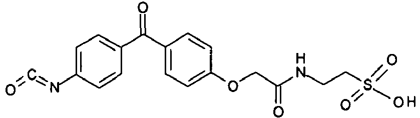 |
| 67 a | 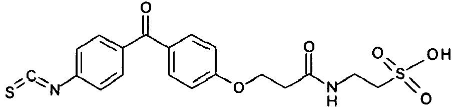 | 67 b | 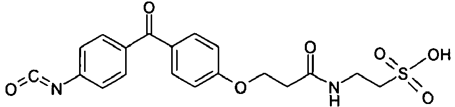 |
| 68 a | 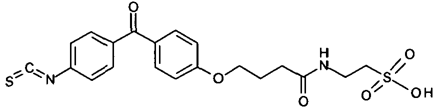 | 68 b | 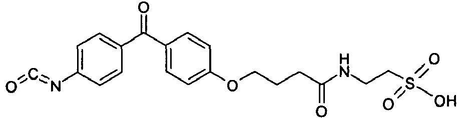 |
| 69 a | 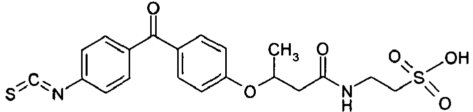 | 69 b | 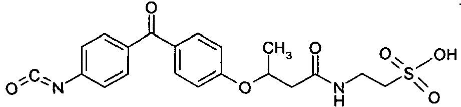 |
| 70 a | 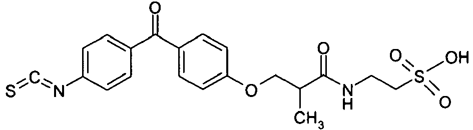 | 70 b | 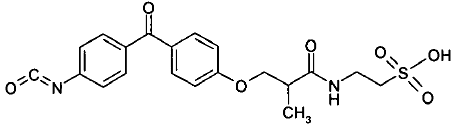 |

sowie pharmazeutisch verträgliche Salze davon, insbesondere deren Natriumsalze.

Analog können durch Umsetzung mit anderen Basen, wie TRIS (Tris(hydroxymethyl)-aminomethan bzw. 2-Amino-2-(hydroxymethyl)-propan-1,3-diol), weitere Salze der Beispielverbindungen erhalten werden.

Dabei weist insbesondere das TRIS-Salz eine deutlich höhere Wasserlöslichkeit bei Raumtemperatur als das Natriumsalz auf.

### SYNTHESE-BEISPIEL

### SYNTHESE DER VERBINDUNG AUS BEISPIEL 2A

Die Synthese von Beispiel 2A verläuft nach folgendem Schema:

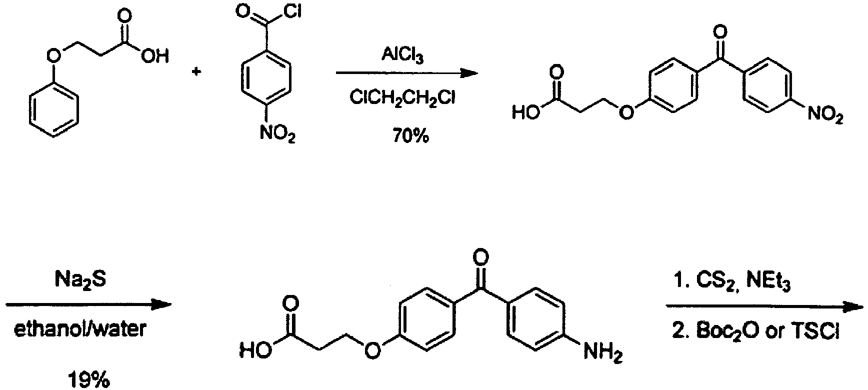

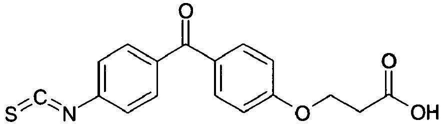

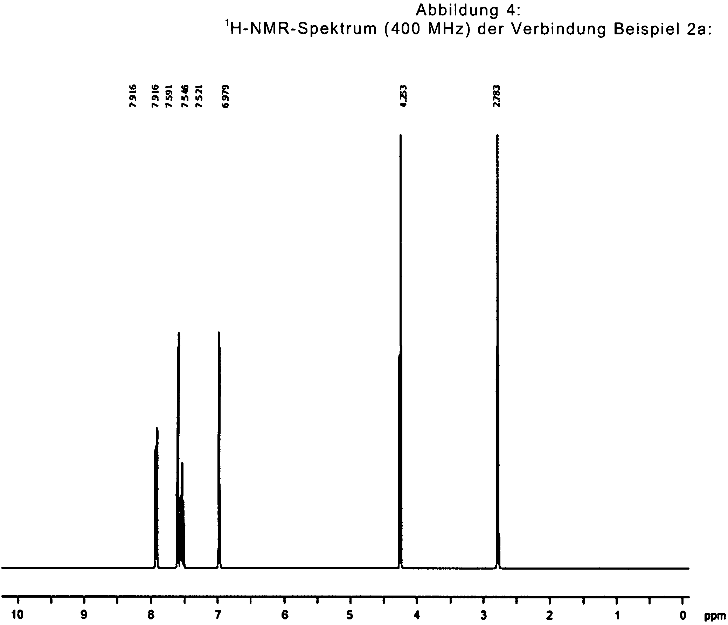

Das ¹H-NMR-Spektrum ist in Abbildung 4 gezeigt.

Die Synthesen der übrigen Beispiele erfolgen analog.

### PHARMAKOLOGISCHE WIRKVERSUCHE

Alle Versuche wurden unter Verwendung von weiblichen Nacktmäusen unter Standardtierhaltungsbedingungen mit kontrollierter Beleuchtung und Temperatur durchgeführt. Die Tiere erhielten Wasser und Futter nach Belieben.

Brusttumore (MAXF 401) wurden subkutan in die hinteren Gliedmaßen der Mäuse im Alter von 10 Wochen eingepflanzt. Die Volumenvergrößerung der einzelnen Tumore wurde mit Mikrogreifzirkeln gemessen, und die Tumorgröße wurde nach der Formel a = b²/2 berechnet, (wobei a der größte Durchmesser des Tumors ist und b die senkrechte Achse ist). Als das Tumorvolumen auf 80 - 1 20 mm³ angestiegen war, wurden die Tiere willkürlich in Versuchsgruppen von je 6 Tieren angeordnet.

Die Testverbindung (als Natriumsalz) wurde in einer Kochsalzlösung aufgelöst, und 5 % Klucel (Hydroxypropylcellulose) wurden hinzugefügt, um die Testverbindung in dem Träger aufzulösen. Die Verbindung wurde durch intraperitoneale Injektion verabreicht. Ein Volumen von 10 ml/kg Körpergewicht wurde injiziert. Tiere, die nur den Träger erhielten, dienten als Kontrolle. Die Testverbindung wurde nach dem Schema einer wöchentlich zweimaligen Verabreichung über einen Zeitraum von 5 Wochen behandelt. Das Tumorvolumen und das Körpergewicht wurden zweimal wöchentlich kontrolliert, und die relativen Tumorvolumina wurden als Verhältnis Tumorgröße vs. Körpergewicht berechnet, Der Versuch wurde beendet, wenn das Tumorvolumen der Kontrollgruppe eine Größe erreicht hatte, welche das Töten der Tiere erforderte, um den Tierschutzvorschriften zu genügen.

Auf der Basis der relativen Tumorvolumina der mit der Testverbindung behandelten Tiere verglichen mit den Tumorvolumina der nur mit dem Träger behandelten Kontrollen, wurden die T/C-Werte berechnet die als Index der Anti-Tumoraktivität dienten. (Der T/C-Index stellt das Verhältnis der Tumorgröße von behandelten und nicht behandelten Tieren dar. Je kleiner das Verhältnis umso besser die Aktivität, 100% wäre keine Aktivität: Tumor gleich groß).

**Tabelle T/C-Wert und Sterblichkeiten für die Testsubstanz aus Beispiel 2a.**

| Testverbindung | T/C-Wert | Sterblichkeit |
|---|---|---|
| Verbindung aus Beispiel 2a | 1,7 | 0/6 |

### Anti-Tumoraktivität:

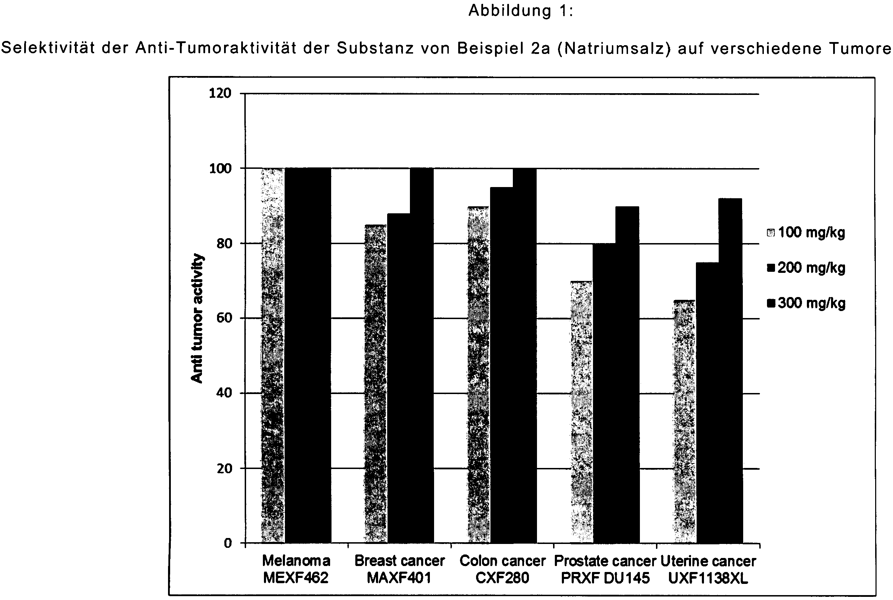

Abbildung 1 zeigt die Selektivität der Anti-Tumoraktivität der Substanz von Beispiel 2a (Natriumsalz) auf verschiedene Tumore

Die Antitumor-Aktivität der Substanz von Beispiel 2a (Natriumsalz) wurde in einem Xenograft Tumorpanel getestet. Hierzu wurden Xenografte humaner Tumore, die sich aus den genannten Krebsarten ableiten, in Nacktmäuse implantiert. Die Implantierung und die Bestimmung von Tumorvolumen erfolgten wie zuvor beschrieben. Gruppen von 5-8 Tieren wurden eingesetzt Die Prüfsubstanz wurde als Natriumsalz als wässrige Lösung in den genannten Dosierungen zweimal wöchentlich durch ip-Injektion verabreicht.

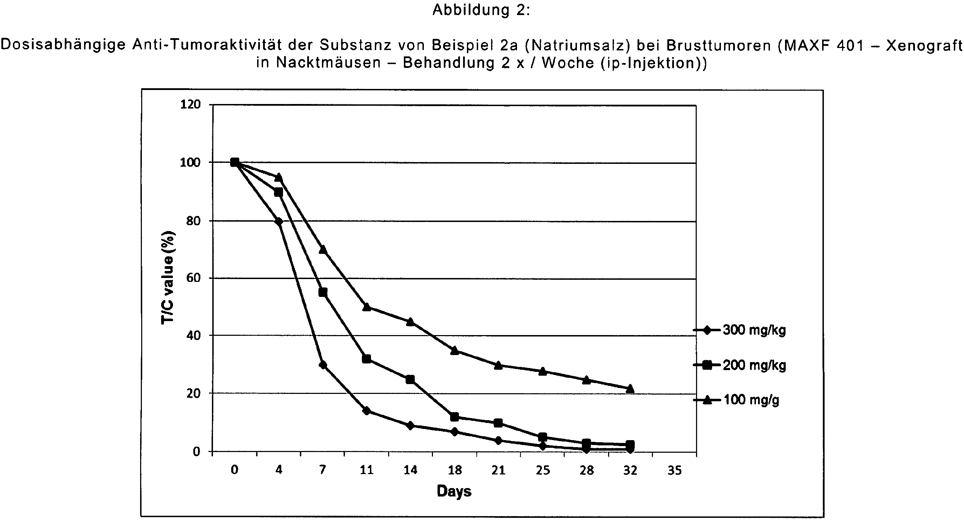

Abbildung 2 zeigt die dosisabhängige Anti-Tumoraktivität der Substanz von Beispiel 2a (Natriumsalz) bei Brusttumoren (MAXF 401 - Xenograft in Nacktmäusen).

Dabei wurde die Antitumor Aktivität der Substanz aus Beispiel 2a (Natriumsalz) im MAXF-401 Xenograft Tumormodell in der Nacktmaus untersucht. Die subkutane Implantierung des Tumors und die Bestimmung von Tumorvolumen erfolgten wie zuvor beschrieben. Die Prüfsubstanz wurde als Natriumsalz als wässrige Lösung in den genannten Dosierungen zweimal wöchentlich durch ip-Injektion über einen Zeitraum von 4 Wochen verabreicht. In der Abbildung ist der zeitliche Verlauf der Antitumorwirkung der Prüfsubstanz als T/C-Wert dosisabhängig wiedergegeben. Eine Dosis von 100 mg/kg wurde als Schwellendosis, ≥ 200 mg/kg als ED90 der Antitumorwirkung bestimmt. In den Dosierungen ≥ 200mg/kg wurde unter Therapie eine Remission der Tumore beobachtet. Die Substanz war im eingesetzten Dosisbereich sehr gut verträglich.

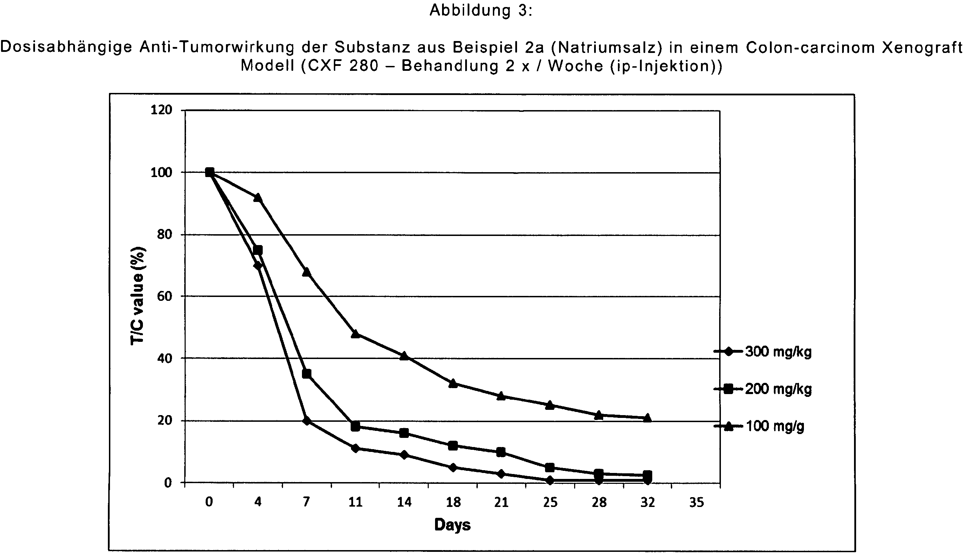

Abbildung 3 zeigt die dosisabhängige Anti-Tumorwirkung der Substanz aus Beispiel 2a (Natriumsalz) in einem Colon-carcinom Xenograft Modell (CXF 280 - Xenograft in Nacktmäusen)

Dabei wurde die Antitumor-Aktivität der Substanz aus Beispiel 2a im CXF280 - Xenograft Tumormodell in der Nacktmaus bestimmt. Die subkutane Implantierung des Tumors und die Bestimmung von Tumorvolumen erfolgten wie zuvor beschrieben, Die Prüfsubstanz wurde als Natriumsalz wurde als wässrige Lösung in den genannten Dosierungen zweimal wöchentlich durch ip-Injektion über einen Zeitraum von 4 Wochen verabreicht. Gruppen zu je 8 Tieren wurden eingesetzt. Nach Verabreichung in Dosierungen von 100, 200 oder 300 mg/kg Beispiel 2a wurde in allen eingesetzten Dosierungen eine ausgeprägte Antitumorwirkung beobachtet. Eine vollständige Hemmung von Tumorwachstum und der Induktion von Remissionen bis hin zur Auslöschung des Tumors bei den Dosierungen 200mg/kg und 300mg/kg.
ED90 Dosen wurde bei Dosierungen ≥ 200mg/kg festgestellt. Die Substanz war im eingesetzten Dosisbereich gut verträglich und mit keinem signifikanten Gewichtsverlust der Tiere verbunden.

Zusammenfassend zeigen sich die erfindungsgemäßen Verbindungen bzw. die pharmazeutischen Zusammensetzungen davon als potente Anti-Tumorarzneien mit einer verbesserten therapeutischen Bandbreite und geringeren Nebenwirkungen.

**Tabelle**

| Zusammensetzung | Akute Einzellzelle Nekrose | Fokale tubuläre Nekrose |
|---|---|---|
| Kontrolle | 0/6 | 0/6 |
| Testsubstanz, Beispiel 2a | 0/8 | 0/8 |

In den Nieren der Tiere die mit der erfindungsgemäßen Verbindung behandelt wurden, wurden keine nekrotischen Veränderungen festgestellt. Dies bestätigt das ausgezeichnete Verträglichkeitsprofil der erfindungsgemäßen Verbindungen.

## Patentansprüche

1. Verbindungen der Formel (1): 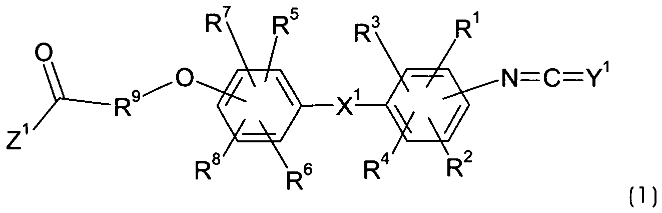 worin
Y¹ = S oder O ist und worin
R¹, R², R³, R⁴, R⁵, R⁶, R⁷ und R⁸ gleich oder verschieden sind, und jeweils ausgewählt werden aus der Gruppe, die besteht aus:
- Wasserstoff,
- Hydroxy,
- Halogen,
- Cyano,
- Nitro,
- Carboxyl,
- Aminocarbonyl,
- Sulfonsäurerest (-SO₃H),
- Aminosulfonyl,
- gegebenenfalls substituiertem Alkyl,
- gegebenenfalls substituiertem Alkoxy,
- gegebenenfalls substituiertem Alkenyl,
- gegebenenfalls substituiertem Aryl,
- gegebenenfalls substituiertem Arylalkyl;
R⁹ gegebenenfalls substituiertes geradkettiges, verzweigtes oder cyclisches Alkandiyl, gegebenenfalls substituiertes geradkettiges, verzweigtes oder cyclisches Alkendiyl, Aryl oder Heterocyclyl ist;
Z¹ ausgewählt ist aus
- Hydroxy (-OH) oder
- einem Rest der Formel -N-R¹⁰R¹¹, worin
R¹⁰ Wasserstoff ist und R¹¹ gegebenenfalls substituiertes Alkyl oder Hydroxyl ist, oder
R¹¹ Wasserstoff ist und R¹⁰ gegebenenfalls substituiertes Alkyl oder Hydroxyl ist, oder
R¹⁰ und R¹¹ jeweils Alkyl sind, worin mindestens eine der Alkylgruppen mindestens einen Substituenten aufweist, oder
R¹⁰ und R¹¹ zusammen mit dem Stickstoffatom, an dass sie gebunden sind einen gesättigten oder ungesättigen, gegebenenfalls substituierten 5 bis 8-gliedrigen Cyclus ausbilden, der gegebenenfalls weitere Heteroatome enthalten kann; und worin
X¹ aus der Gruppe ausgewählt wird, die besteht aus:
- einer Einfachbindung
- Carbonyl,
- Schwefel,
- Sauerstoff,
- Sulfoxy,
- Sulfonyl,
- Azo, und
- einem gegebenenfalls substituierten, gesättigten oder ungesättigten aliphatischen Rest mit 1 bis 6 Kohlenstoffatomen,
oder pharmazeutisch verträgliche Salze davon.

2. Verbindungen nach Anspruch 1, worin Z¹ Hydroxy ist.

3. Verbindungen nach Anspruch 1, worin Z¹ ein Rest der Formel -N-R¹⁰R¹¹ ist, worin
R¹⁰ Wasserstoff ist und R¹¹ substituiertes Alkyl ist, oder
R¹¹ Wasserstoff ist und R¹⁰ substituiertes Alkyl ist.

4. Verbindungen nach Anspruch 3, worin
substituiertes Alkyl, eine Alkylgruppe ist, welche mindestens eine Gruppe der Formel
-X²-R¹²
aufweist, worin
X² ausgewählt wird aus der Gruppe, die besteht aus:
- Carbonyl,
- Sulfoxy, und
- Sulfonyl, und
R¹² ausgewählt wird aus der Gruppe, die besteht aus:
- Hydroxy,
- gegebenenfalls substituiertem Amino, und
- gegebenenfalls substituiertem Alkoxy.

5. Verbindungen nach einem der Ansprüche 1, 3 oder 4, worin
R¹⁰ Wasserstoff ist und R¹¹ ein Rest A einer Verbindung der Formel H₂N-A oder
R¹¹ Wasserstoff ist und R¹⁰ ein Rest A einer Verbindung der Formel H₂N-A ist, worin
A ein Rest ist, der sich durch Abspaltung der Aminogruppe (-NH₂) aus einer natürlichen oder synthetischen Aminosäure, einem natürlichen oder synthetischen Aminosäurederivat oder einer Polyaminosäure oder einem Polyaminosäurederivat ableitet.

6. Verbindungen nach einem oder mehreren der Ansprüche 1 bis 5, der Formel (2):

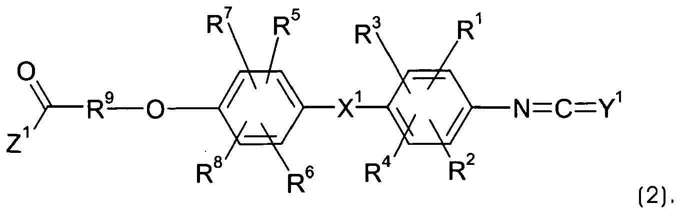

7. Verbindungen nach einem der Ansprüche 1 bis 6, worin
Y¹ = S oder O ist,
R¹, R², R³, R⁴, R⁵, R⁶, R⁷ und R⁸ jeweils Wasserstoff sind,
R⁹ geradkettiges, verzweigtes oder cyclisches Alkandiyl ist,
Z¹ Hydroxy oder ein Rest der Formel -N-R¹⁰R¹¹ ist, worin
R¹⁰ Wasserstoff ist und R¹¹ ein Rest A einer Verbindung der Formel H₂N-A oder
R¹¹ Wasserstoff ist und R¹⁰ ein Rest A einer Verbindung der Formel H₂N-A ist, worin
A jeweils ein Rest ist der sich durch Abspaltung der Aminogruppe (-NH₂) aus einer natürlichen oder synthetischen Aminosäure, einem natürlichen oder synthetischen Aminosäurederivat oder einer Polyaminosäure oder einem Polyaminosäurederivat ableitet, und
X¹ Carbonyl (-CO-) ist.

8. Verbindungen nach einem der Ansprüche 1 bis 6, worin
Y¹ = S oder O ist,
R¹, R², R³, R⁴, R⁵, R⁶, R⁷ und R⁸ jeweils Wasserstoff sind,
R⁹ geradkettiges, verzweigtes oder cyclisches Alkandiyl ist,
Z¹ Hydroxy ist, und
X¹ Carbonyl (-CO-) ist.

9. Verbindungen nach einem oder mehreren der Ansprüche 1 bis 8, ausgewählt aus der Gruppe, die besteht aus:
| | | | |
|---|---|---|---|
| 1a | 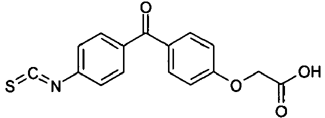 | 1b | 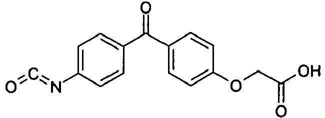 |
| 2a | 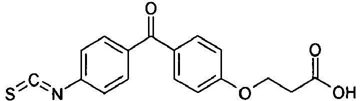 | 2b | 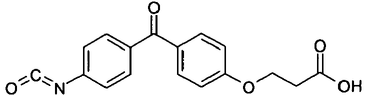 |
| 3a | 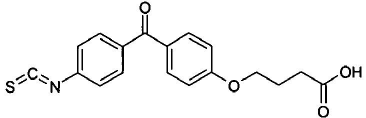 | 3b | 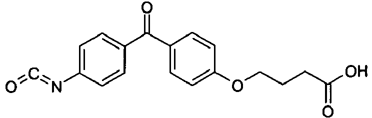 |
| 4a | 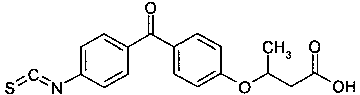 | 4b | 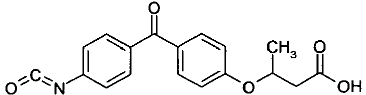 |
| 5a | 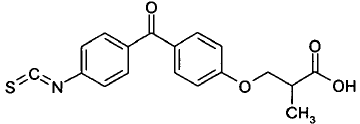 | 5b | 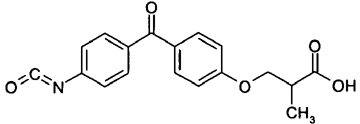 |
| 6a | 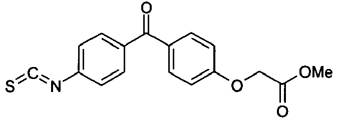 | 6b | 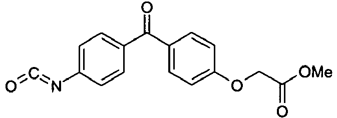 |
| 7a | 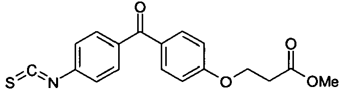 | 7b | 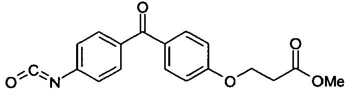 |
| 8a | 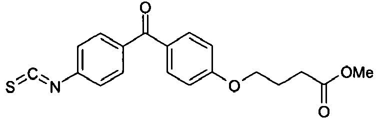 | 8b | 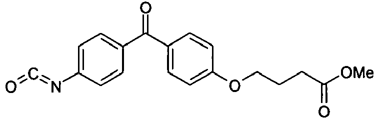 |
| 9a | 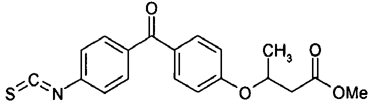 | 9b | 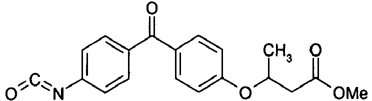 |
| 10 a | 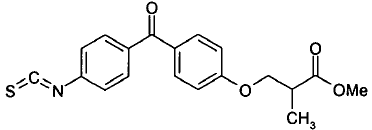 | 10 b | 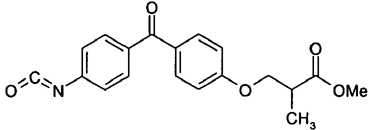 |
| 11 a | 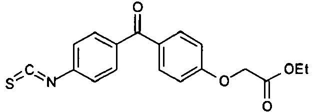 | 11 b | 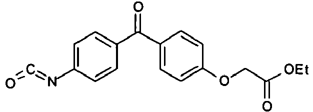 |
| 12 a | 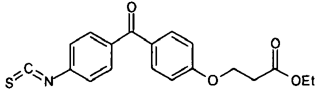 | 12 b | 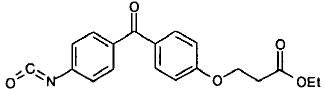 |
| 13 a | 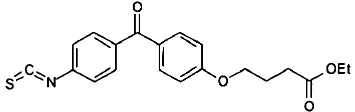 | 13 b | 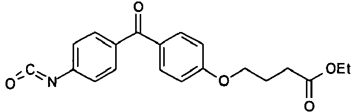 |
| 14 a | 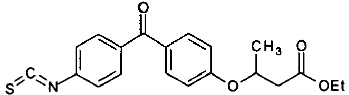 | 14 b | 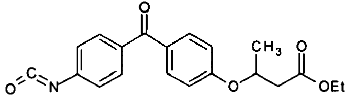 |
| 15 a | 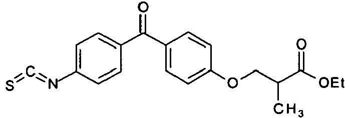 | 15 b | 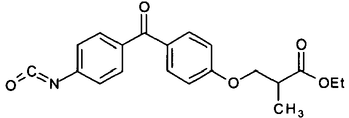 |
| 16 a | 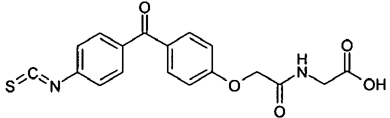 | 16 b | 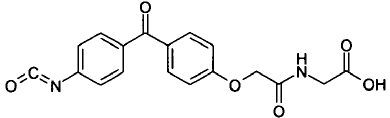 |
| 17 a | 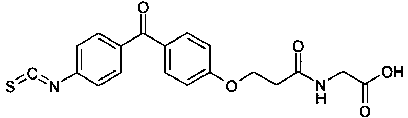 | 17 b | 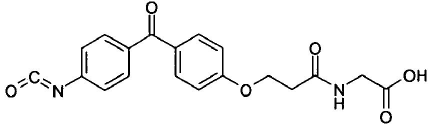 |
| 18 a | 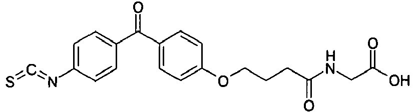 | 18 b | 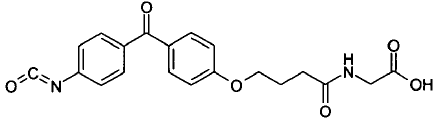 |
| 19 a | 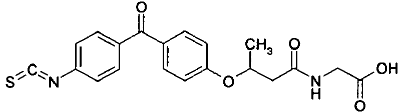 | 19 b | 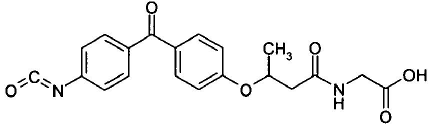 |
| 20 a | 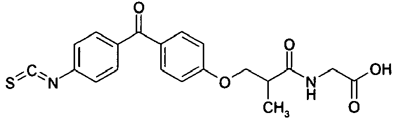 | 20 b | 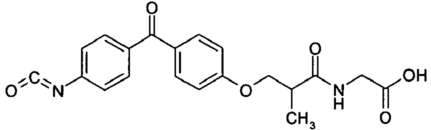 |
| 21 a | 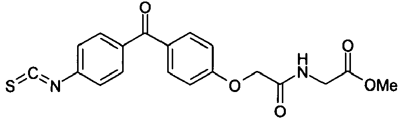 | 21 b | 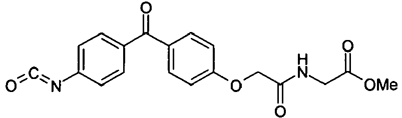 |
| 22 a | 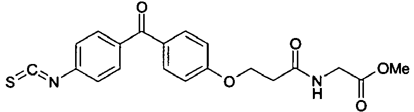 | 22 b | 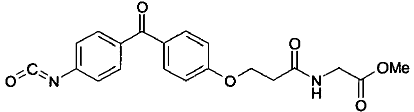 |
| 23 a | 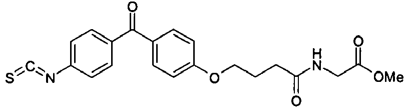 | 23 b | 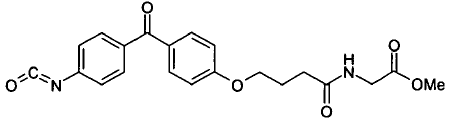 |
| 24 a | 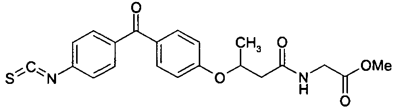 | 24 b | 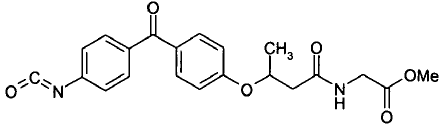 |
| 25 a | 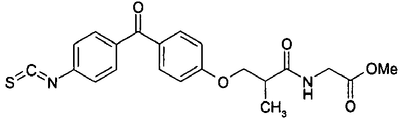 | 25 b | 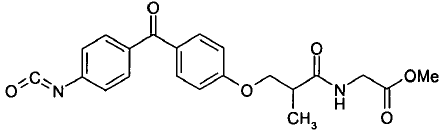 |
| 26 a | 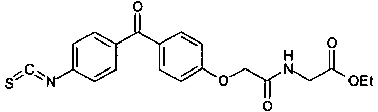 | 26 b | 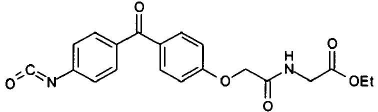 |
| 27 a | 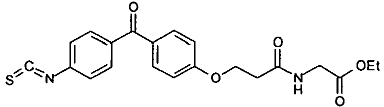 | 27 b | 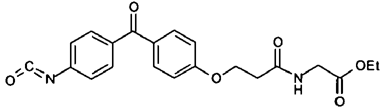 |
| 28 a | 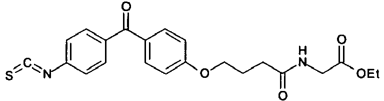 | 28 b | 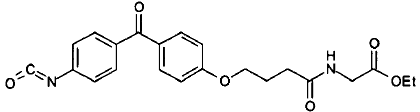 |
| 29 a | 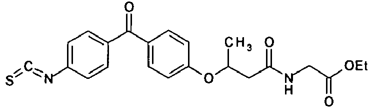 | 29 b | 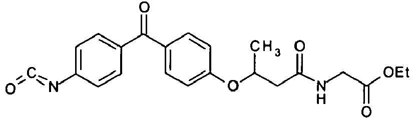 |
| 30 a | 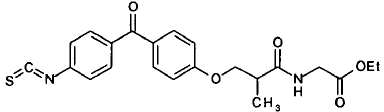 | 30 b | 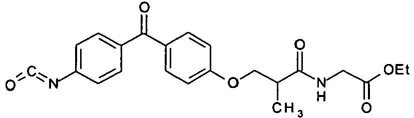 |
| 31 a | 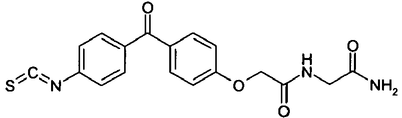 | 31 b | 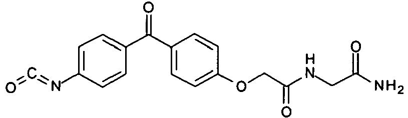 |
| 32 a | 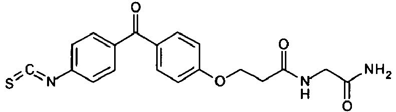 | 32 b | 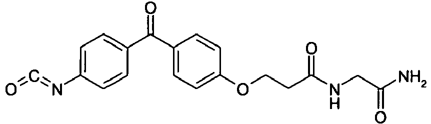 |
| 33 a | 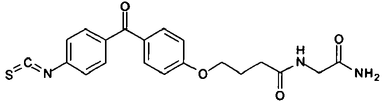 | 33 b | 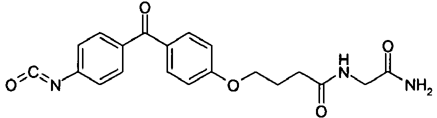 |
| 34 a | 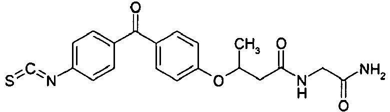 | 34 b | 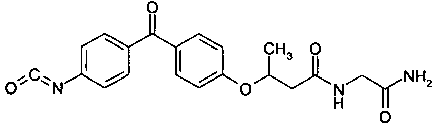 |
| 35 a | 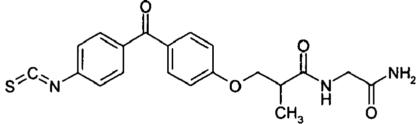 | 35 b | 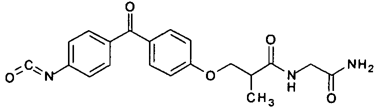 |
| 36 a | 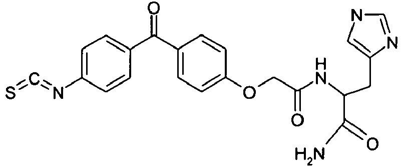 | 36 b | 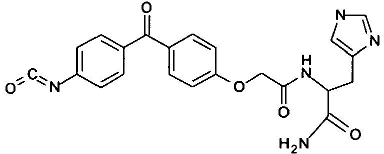 |
| 37 a | 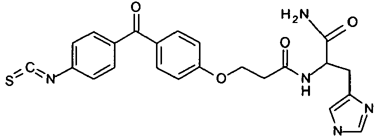 | 37 b | 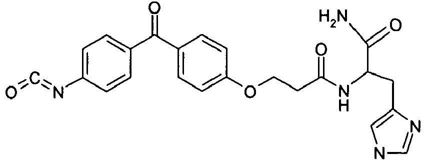 |
| 38 a | 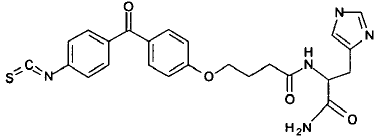 | 38 b | 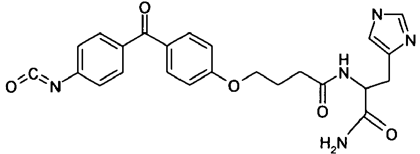 |
| 39 a | 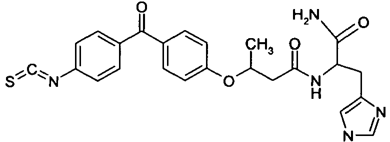 | 39 b | 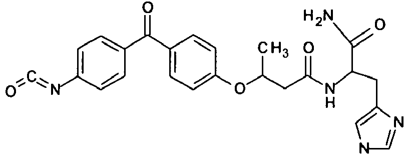 |
| 40 a | 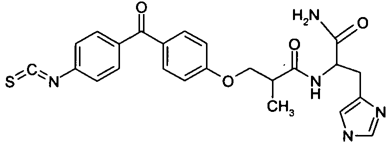 | 40 b | 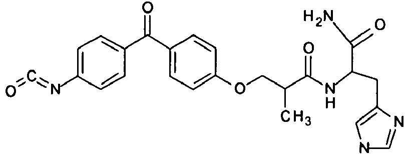 |
| 41 a | 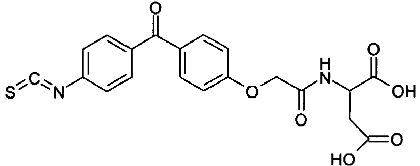 | 41 b | 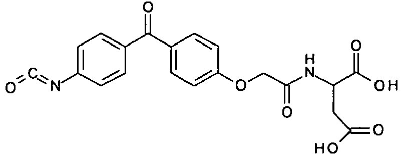 |
| 42 a | 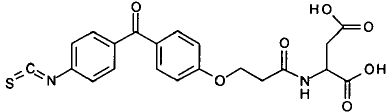 | 42 b | 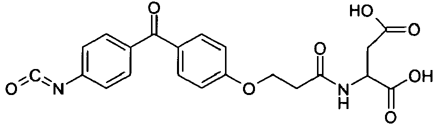 |
| 43 a | 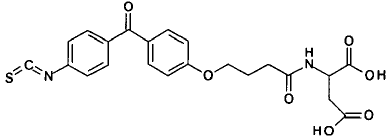 | 43 b | 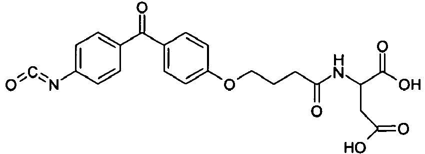 |
| 44 a | 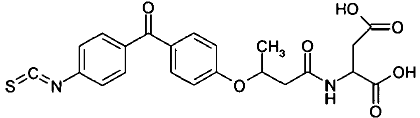 | 44 b | 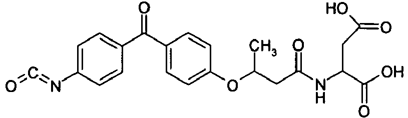 |
| 45 a | 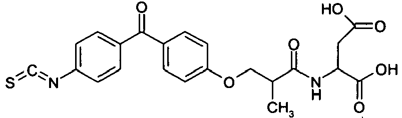 | 45 b | 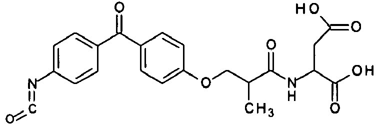 |
| 46 a | 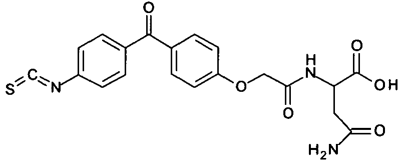 | 46 b | 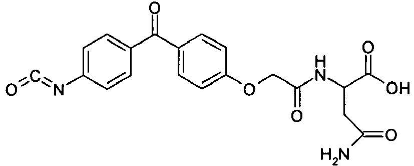 |
| 47 a | 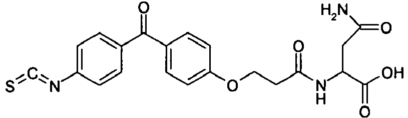 | 47 b | 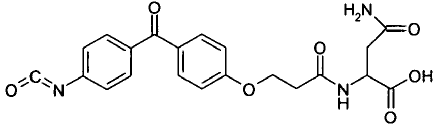 |
| 48 a | 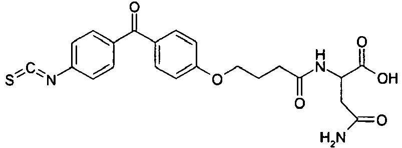 | 48 b | 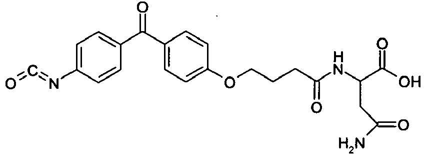 |
| 49 a | 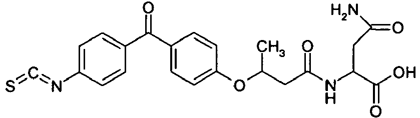 | 49 b | 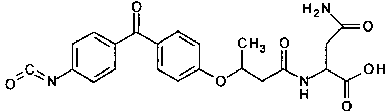 |
| 50 a | 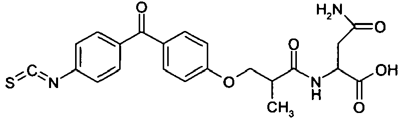 | 50 b | 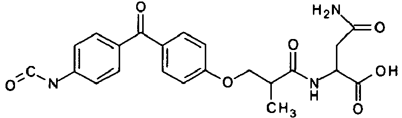 |
| 51 a | 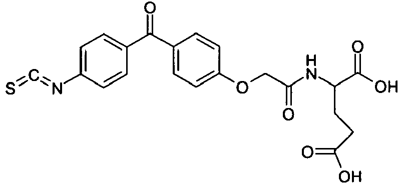 | 51 b | 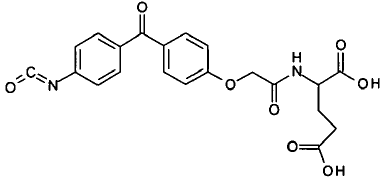 |
| 52 a | 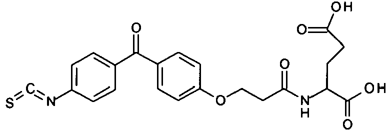 | 52 b | 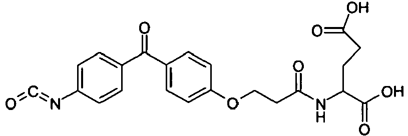 |
| 53 a | 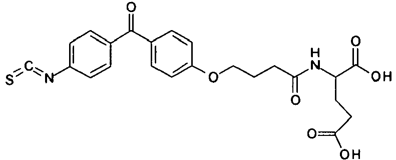 | 53 b | 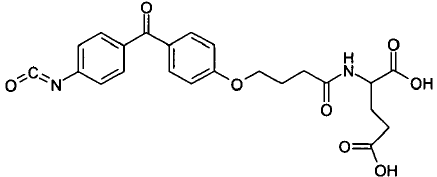 |
| 54 a | 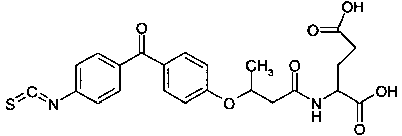 | 54 b | 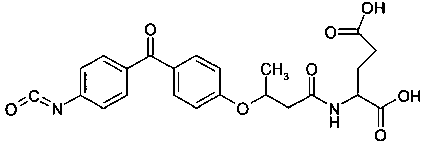 |
| 55 a | 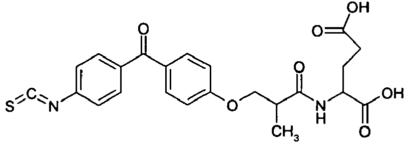 | 55 b | 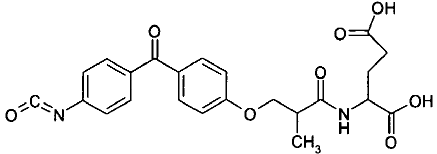 |
| 56 a | 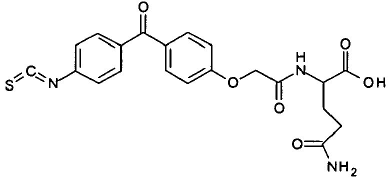 | 56 b | 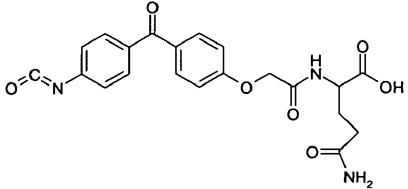 |
| 57 a | 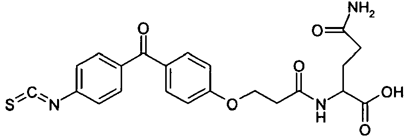 | 57 b | 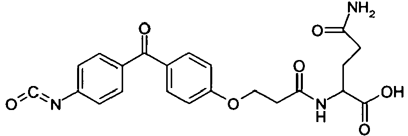 |
| 58 a | 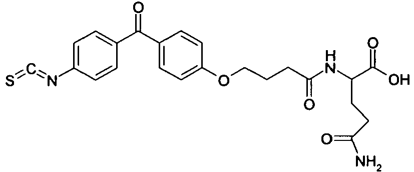 | 58 b | 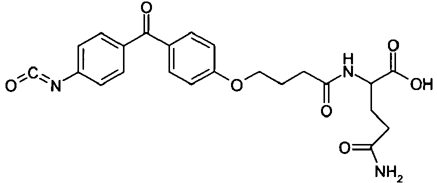 |
| 59 a | 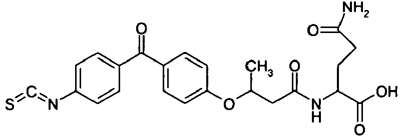 | 59 b | 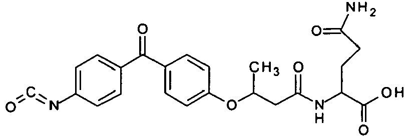 |
| 60 a | 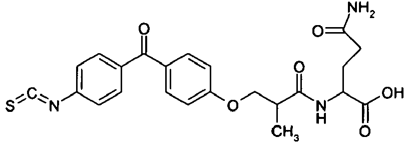 | 60 b | 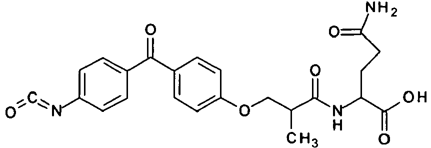 |
| 61 a | 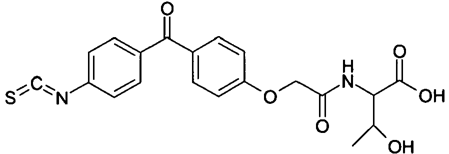 | 61 b | 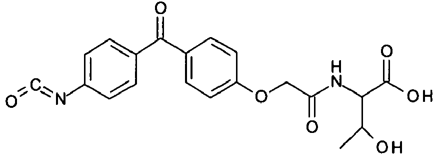 |
| 62 a | 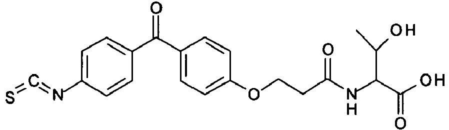 | 62 b | 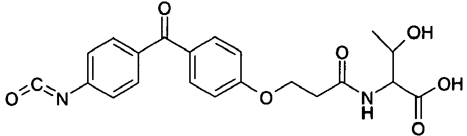 |
| 63 a | 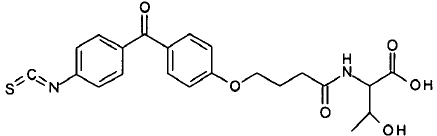 | 63 b | 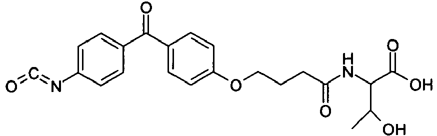 |
| 64 a | 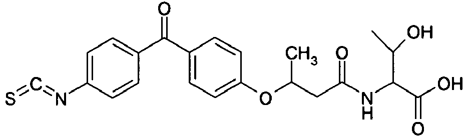 | 64 b | 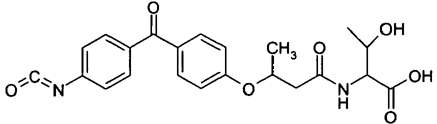 |
| 65 a | 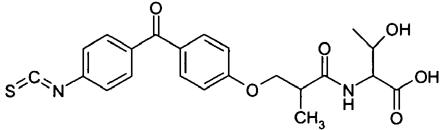 | 65 b | 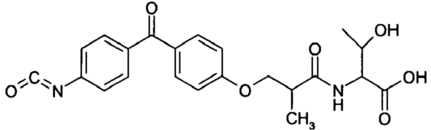 |
| 66 a | 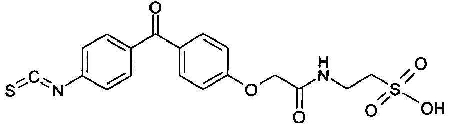 | 66 b | 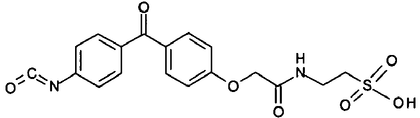 |
| 67 a | 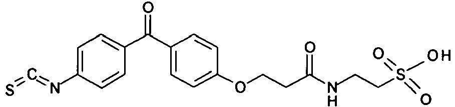 | 67 b | 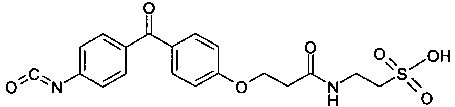 |
| 68 a | 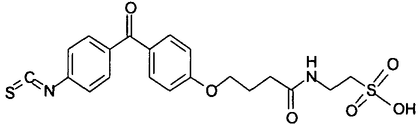 | 68 b | 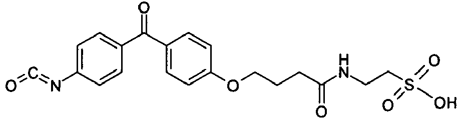 |
| 69 a | 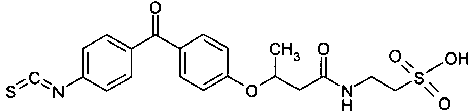 | 69 b | 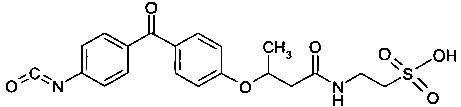 |
| 70 a | 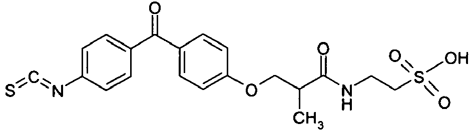 | 70 b | 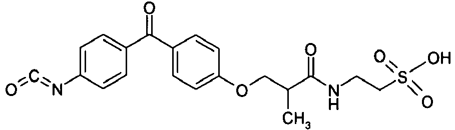 |
oder pharmazeutisch verträgliche Salze davon.

10. Verbindungen nach einem oder mehreren der Ansprüche 1 bis 9 zur Verwendung als Arzneimittel.

11. Verbindungen nach einem oder mehreren der Ansprüche 1 bis 9 zur Verwendung als Arzneimittel zur Behandlung von Krebserkrankungen.

## Claims

1. Compounds of the formula (1): 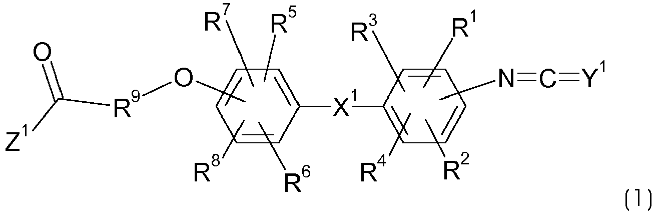 wherein
Y¹ = S or O and wherein
R¹, R², R³, R⁴, R⁵, R⁶, R⁷ and R⁸ are identical or different and are in each case selected from the group consisting of:
- hydrogen,
- hydroxy,
- halogen,
- cyano,
- nitro,
- carboxyl,
- aminocarbonyl,
- sulfonic acid radical (-SO3H),
- aminosulfonyl,
- optionally substituted alkyl,
- optionally substituted alkoxy,
- optionally substituted alkenyl,
- optionally substituted aryl,
- optionally substituted alkylaryl;
R⁹ is optionally substituted linear, branched or cyclic alkanediyl, optionally substituted linear, branched or cyclic alkenediyl, aryl or heterocyclyl;
Z¹ is selected from
- hydroxy (-OH) or
- a radical of the formula -N-R¹⁰R¹¹, wherein
R¹⁰ is hydrogen and R¹¹ is optionally substituted alkyl or hydroxyl, or
R¹¹ is hydrogen and R¹⁰ optionally substituted alkyl or hydroxyl, or
R¹⁰ and R¹¹ are each alkyl, wherein at least one of the alkyl groups has at least one substituent, or
R¹⁰ and R¹¹, together with the nitrogen atom to which they are bonded, form a saturated or unsaturated, optionally substituted 5- to 8-membered ring which can optionally contain further heteroatoms; and wherein
X¹ is selected from the group consisting of:
- a single bond,
- carbonyl,
- sulfur,
- oxygen,
- sulfoxy,
- sulfonyl,
- azo and
- an optionally substituted, saturated or unsaturated aliphatic radical having from 1 to 6 carbon atoms,
or pharmaceutically acceptable salts thereof.

2. Compounds according to claim 1, wherein Z¹ is hydroxy.

3. Compounds according to claim 1, wherein Z¹ is a radical of the formula -N-R¹⁰R¹¹, wherein
R¹⁰ is hydrogen and R¹¹ is substituted alkyl, or
R¹¹ is hydrogen and R¹⁰ is substituted alkyl.

4. Compounds according to claim 3, wherein
substituted alkyl, is an alkyl group, which comprises at least one group of the formula
-X²-R¹²,
wherein
X² is selected from the group consisting of:
- carbonyl,
- sulfoxy, and
- sulfonyl, and
R¹² is selected from the group consisting of:
- hydroxy,
- optionally substituted amino, and
- optionally substituted alkoxy.

5. Compounds according to any one of claims 1, 3 or 4, wherein
R¹⁰ is hydrogen and R¹¹ is a radical A of a compound of the formula H₂N-A, or
R¹¹ is hydrogen and R¹⁰ is a radical A of a compound of the formula H₂N-A, wherein
A is a radical that is derived by cleavage of the amino group (-NH₂) from a natural or synthetic amino acid, a natural or synthetic amino acid derivative or a polyamino acid or polyamino acid derivative.

6. Compounds according to one or more of claims 1 to 5, of formula (2):

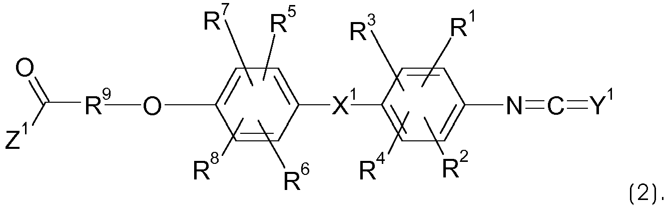

7. Compounds according to any one of claims 1 to 6, wherein
Y¹ = S or O,
R¹, R², R³, R⁴, R⁵, R⁶, R⁷ and R⁸ are in each case hydrogen,
R⁹ is linear, branched or cyclic alkanediyl,
Z¹ is hydroxy or a radical of the formula -N-R¹⁰R¹¹, wherein
R¹⁰ is hydrogen and R¹¹ is a radical A of a compound of the formula H₂N-A or
R¹¹ is hydrogen and R¹⁰ is a radical A of a compound of the formula H₂N-A, wherein
A is in each case a radical that is derived by cleavage of the amino group (-NH₂) from a natural or synthetic amino acid, a natural or synthetic amino acid derivative or a polyamino acid or polyamino acid derivative, and
X¹ is carbonyl (-CO-).

8. Compounds according to any one of claims 1 to 6, wherein
Y¹ = S or O,
R¹, R², R³, R⁴, R⁵, R⁶, R⁷ and R⁸ are in each case hydrogen,
R⁹ is linear, branched or cyclic alkanediyl,
Z¹ is hydroxy, and
X¹ is carbonyl (-CO-).

9. Compounds according to any one of claims 1 to 8, selected from the group consisting of:
| | | | |
|---|---|---|---|
| 1a | 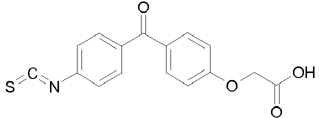 | 1b | 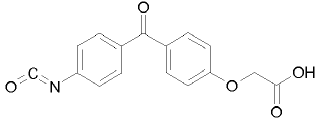 |
| 2a | 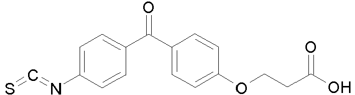 | 2b | 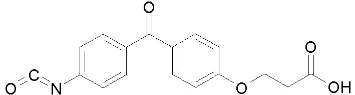 |
| 3a | 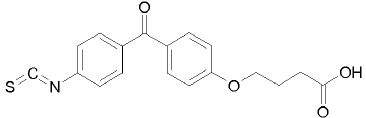 | 3b | 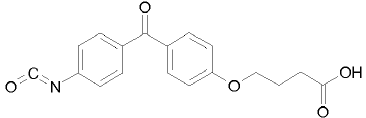 |
| 4a | 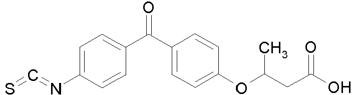 | 4b | 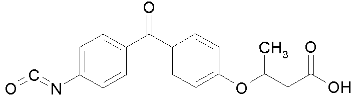 |
| 5a | 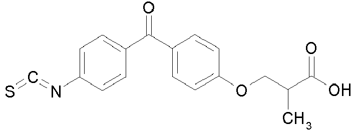 | 5b | 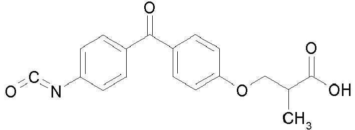 |
| 6a | 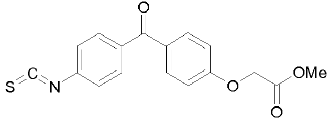 | 6b | 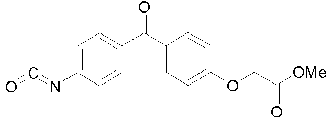 |
| 7a | 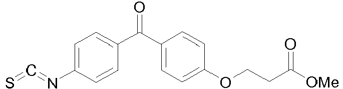 | 7b | 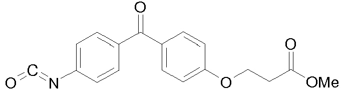 |
| 8a | 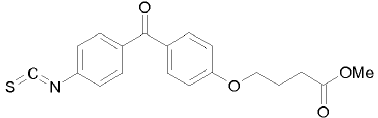 | 8b | 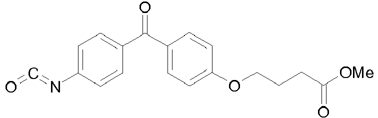 |
| 9a | 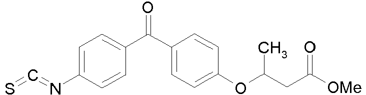 | 9b | 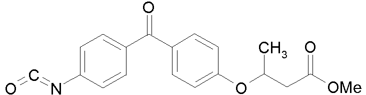 |
| 10 a | 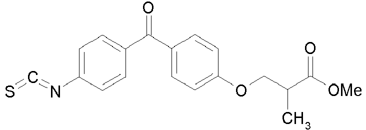 | 10 b | 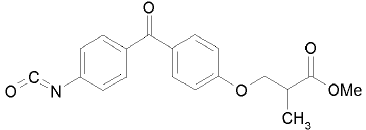 |
| 11 a | 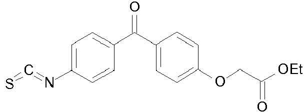 | 11 b | 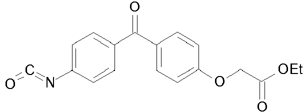 |
| 12 a | 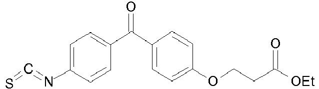 | 12 b | 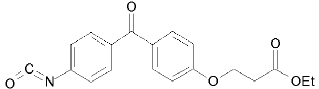 |
| 13 a | 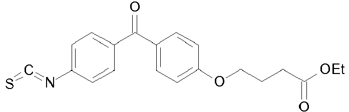 | 13 b | 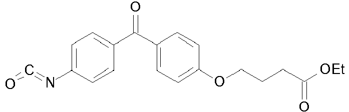 |
| 14 a | 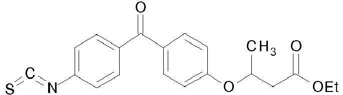 | 14 b | 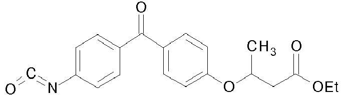 |
| 15 a | 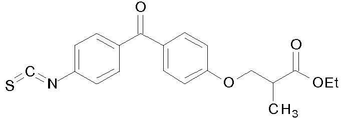 | 15 b | 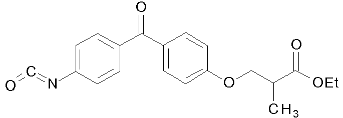 |
| 16 a | 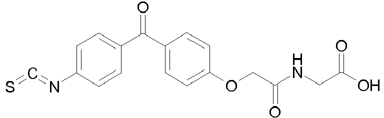 | 16 b | 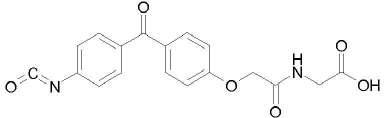 |
| 17 a | 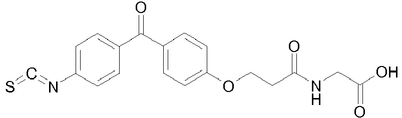 | 17 b | 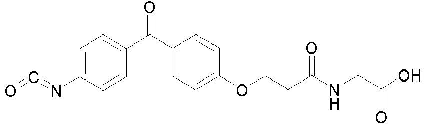 |
| 18 a | 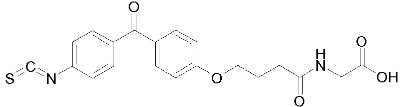 | 18 b | 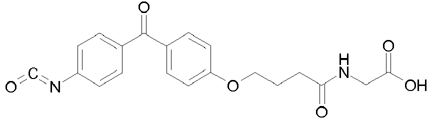 |
| 19 a | 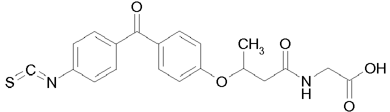 | 19 b | 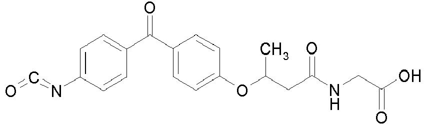 |
| 20 a | 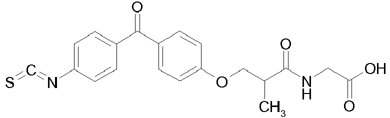 | 20 b | 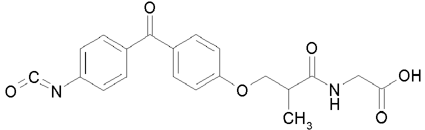 |
| 21 a | 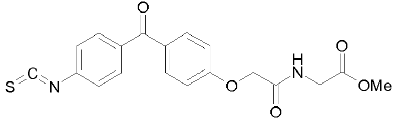 | 21 b | 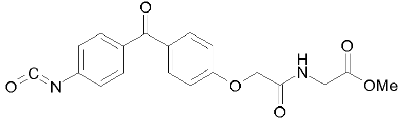 |
| 22 a | 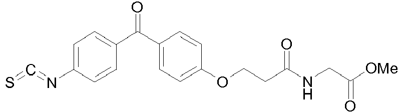 | 22 b | 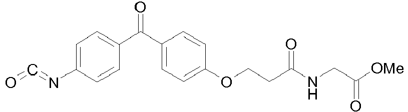 |
| 23 a | 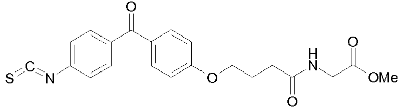 | 23 b | 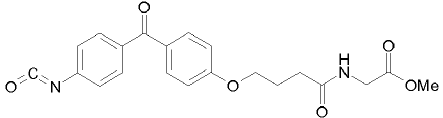 |
| 24 a | 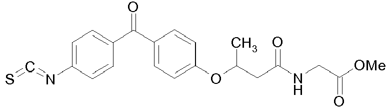 | 24 b | 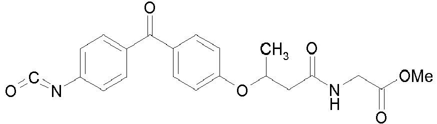 |
| 25 a | 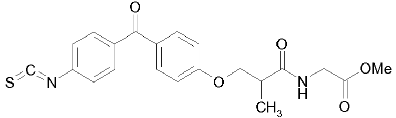 | 25 b | 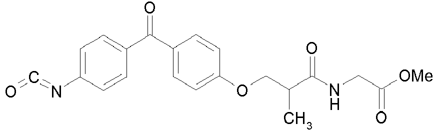 |
| 26 a | 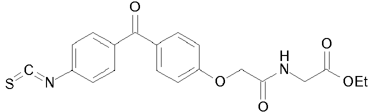 | 26 b | 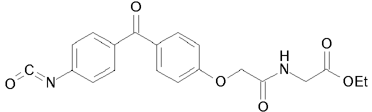 |
| 27 a | 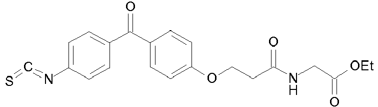 | 27 b | 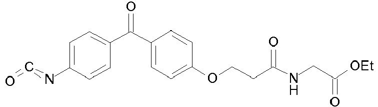 |
| 28 a | 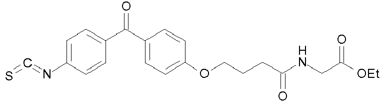 | 28 b | 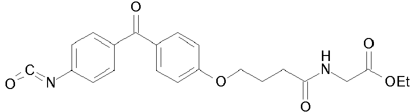 |
| 29 a | 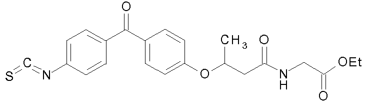 | 29 b | 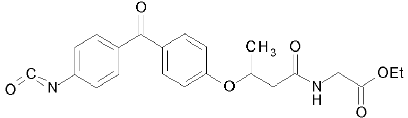 |
| 30 a | 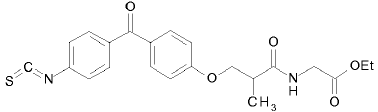 | 30 b | 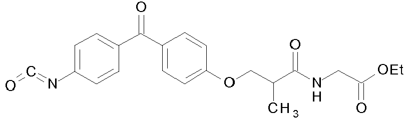 |
| 31 a | 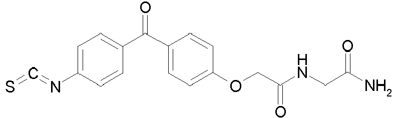 | 31 b | 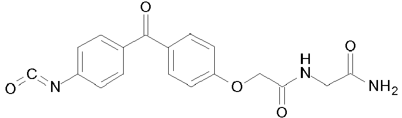 |
| 32 a | 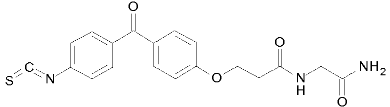 | 32 b | 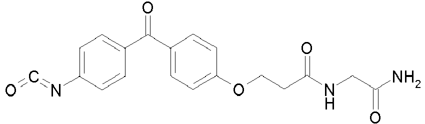 |
| 33 a | 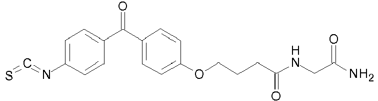 | 33 b | 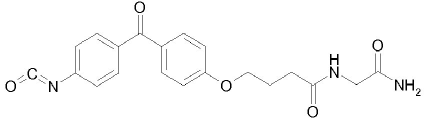 |
| 34 a | 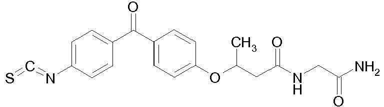 | 34 b | 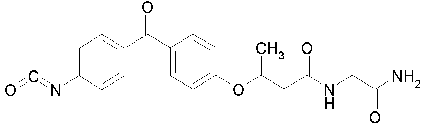 |
| 35 a | 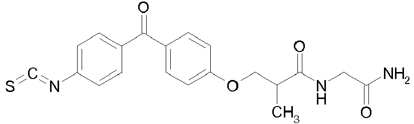 | 35 b | 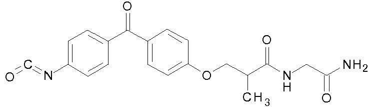 |
| 36 a | 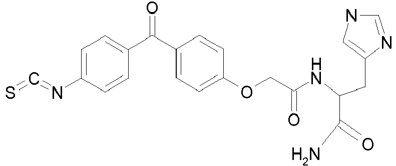 | 36 b | 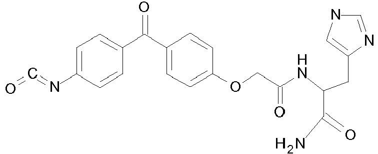 |
| 37 a | 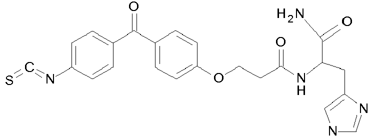 | 37 b | 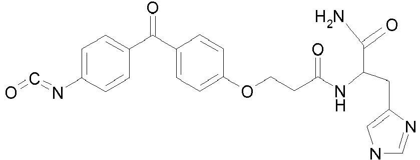 |
| 38 a | 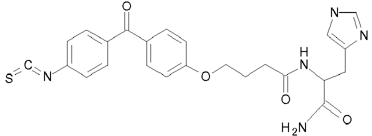 | 38 b | 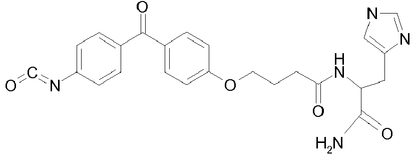 |
| 39 a | 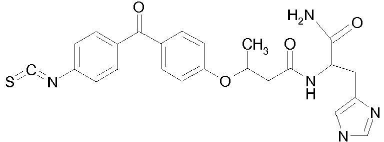 | 39 b | 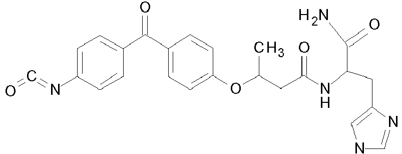 |
| 40 a | 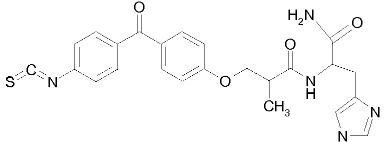 | 40 b | 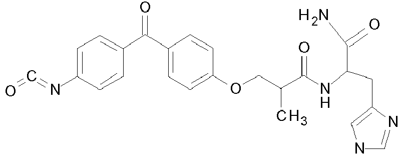 |
| 41 a | 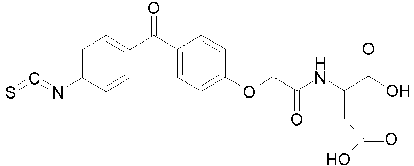 | 41 b | 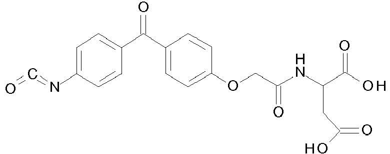 |
| 42 a | 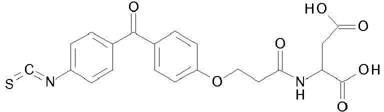 | 42 b | 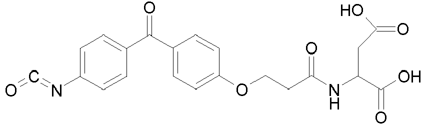 |
| 43 a | 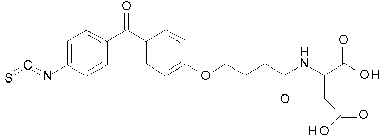 | 43 b | 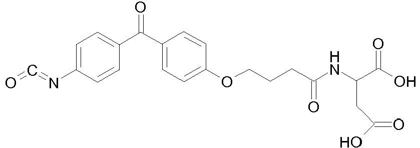 |
| 44 a | 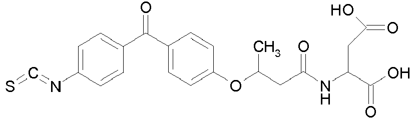 | 44 b | 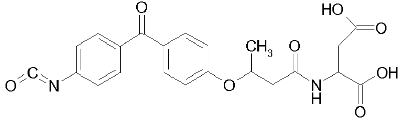 |
| 45 a | 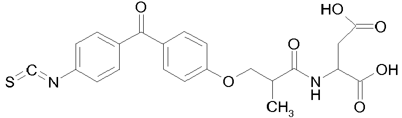 | 45 b | 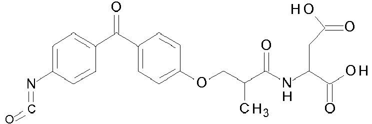 |
| 46 a | 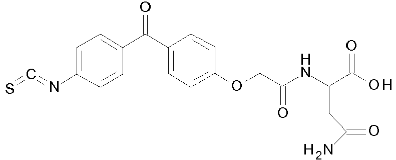 | 46 b | 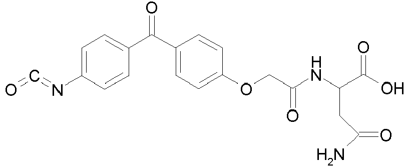 |
| 47 a | 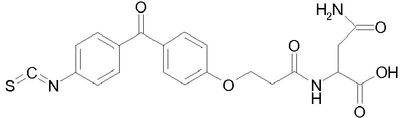 | 47 b | 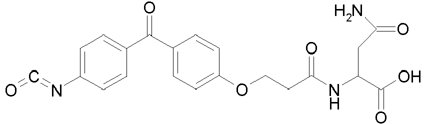 |
| 48 a | 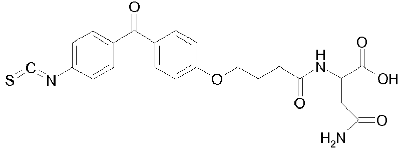 | 48 b | 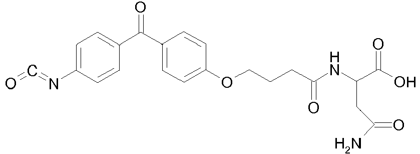 |
| 49 a | 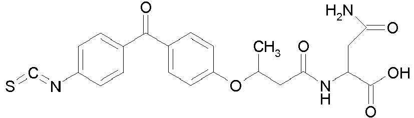 | 49 b | 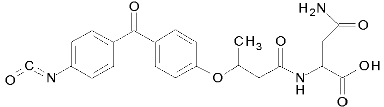 |
| 50 a | 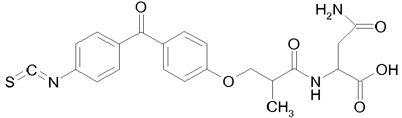 | 50 b | 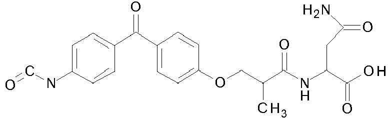 |
| 51 a | 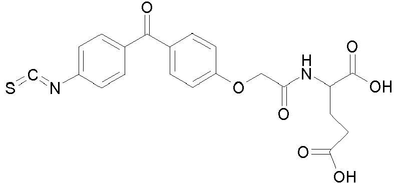 | 51 b | 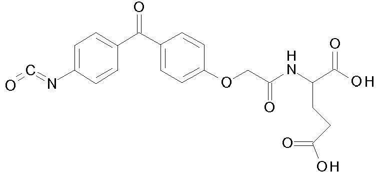 |
| 52 a | 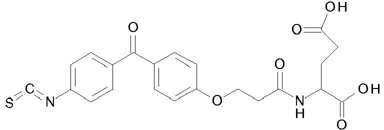 | 52 b | 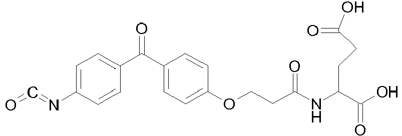 |
| 53 a | 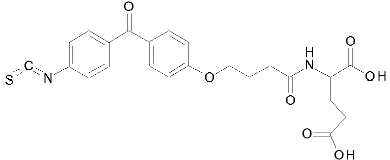 | 53 b | 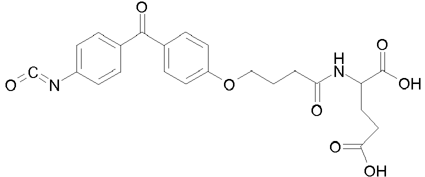 |
| 54 a | 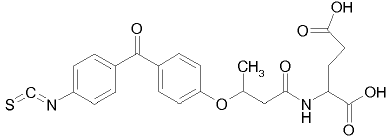 | 54 b | 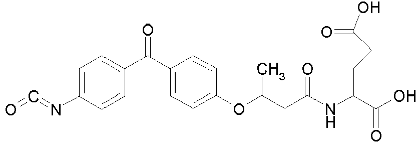 |
| 55 a | 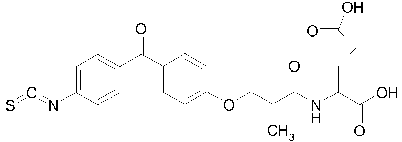 | 55 b | 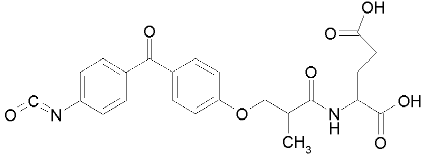 |
| 56 a | 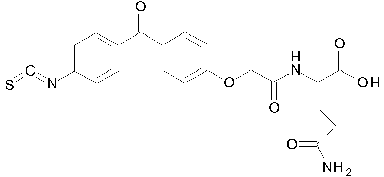 | 56 b | 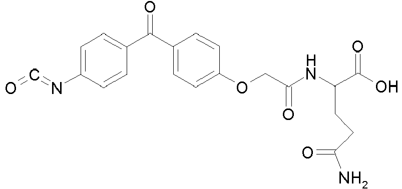 |
| 57 a | 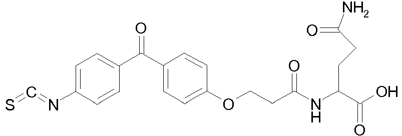 | 57 b | 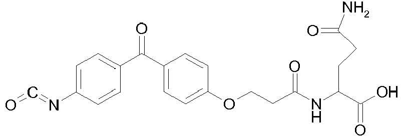 |
| 58 a | 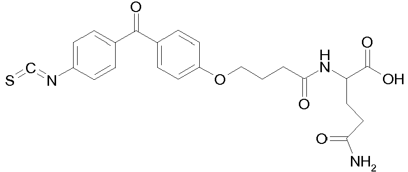 | 58 b | 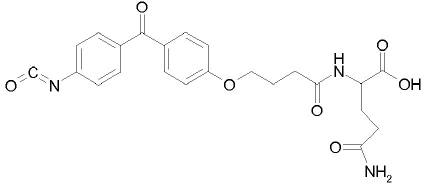 |
| 59 a | 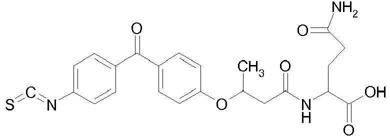 | 59 b | 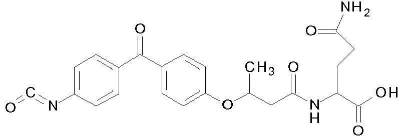 |
| 60 a | 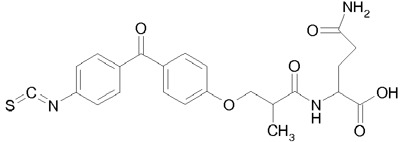 | 60 b | 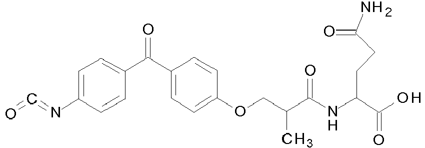 |
| 61 a | 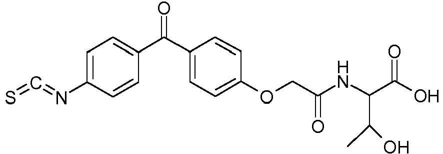 | 61 b | 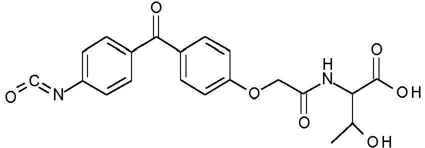 |
| 62 a | 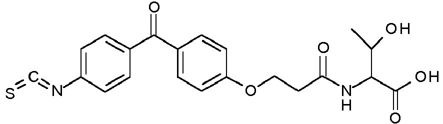 | 62 b | 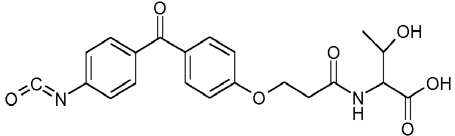 |
| 63 a | 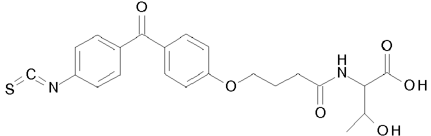 | 63 b | 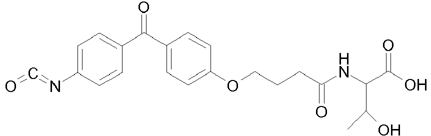 |
| 64 a | 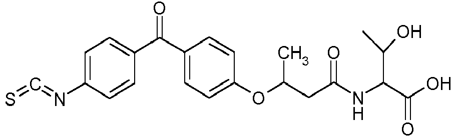 | 64 b | 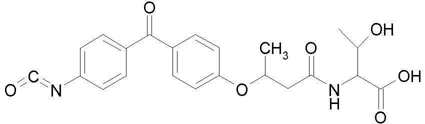 |
| 65 a | 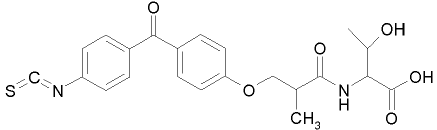 | 65 b | 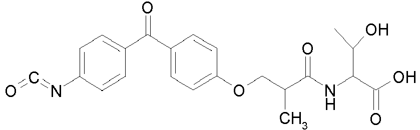 |
| 66 a | 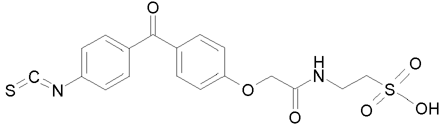 | 66 b | 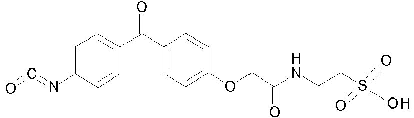 |
| 67 a | 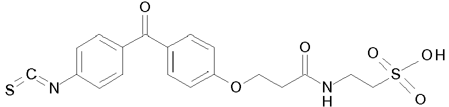 | 67 b | 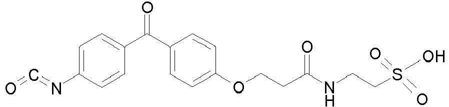 |
| 68 a | 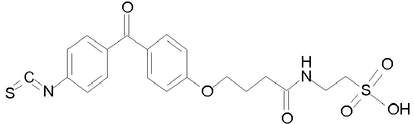 | 68 b | 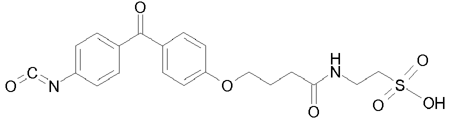 |
| 69 a | 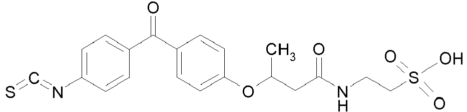 | 69 b | 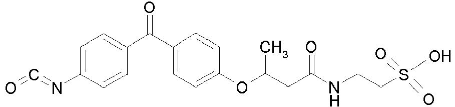 |
| 70 a | 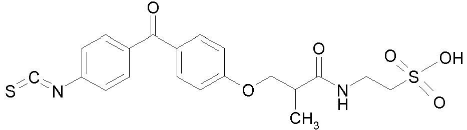 | 70 b | 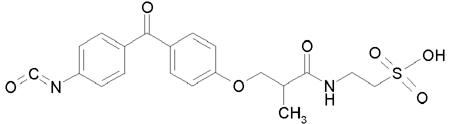 |
or pharmaceutically acceptable salts thereof.

10. Compounds according to one or more of claims 1 to 9 for use as a medicament.

11. Compounds according to one or more of claims 1 to 9 for use as a medicament for the treatment of cancer diseases.

## Revendications

1. Composés de la formule (1): 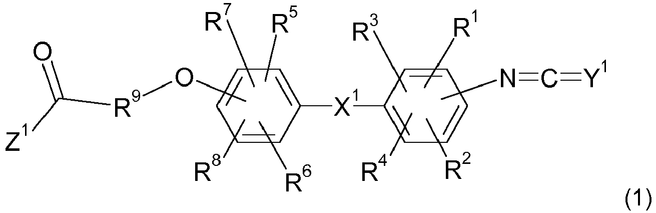 dans laquelle
Y¹ est = S ou O et dans laquelle
R¹, R², R³, R⁴, R⁵, R⁶, R⁷ et R⁸ sont identiques ou différents et chacun de ceux est sélectionné parmi le groupe consistant en :
- hydrogène
- hydroxyle,
- halogène,
- cyano,
- nitro,
- carboxyle,
- aminocarbonyle,
- un résidu de l'acide sulfonique (-SO₃H),
- aminosulfonyle,
- alklye qui est optionnellement substitué,
- alcoxyle qui est optionnellement substitué,
- alcényle qui est optionnellement substitué,
- aryle qui est optionnellement substitué,
- arylalkyle qui est optionnellement substitué ;
R⁹ est un alcanediyle linéaire, ramifié ou cyclique optionnellement substitué, un alcènediyle linéaire, ramifié ou cyclique optionnellement substitué, de l'aryle ou hétérocyclyle ;
Z¹ est sélectionné parmi
- hydroxyle (-OH) ou
- un résidu de la formule -N-R¹⁰R¹¹, dans laquelle
R¹⁰ est de l'hydrogène et R¹¹ est un alkyle qui est optionnellement substitué ou un hydroxyle ou
R¹¹ est de l'hydrogène et R¹⁰ est un alkyle qui est optionnellement substitué ou un hydroxyle ou
chacun de R¹⁰ et R¹¹ est un alkyle, où au moins l'un des groupes alkyle a au moins un substituent ou
R¹⁰ et R¹¹ forment un cycle saturé ou insaturé à 5 à 8 membres qui est optionnellement substitué ensemble avec l'atome d'azote auquel ils sont liés ; et dans laquelle
X¹ est sélectionné parmi le groupe consistant en :
- une liaison simple,
- carbonyle,
- souffre,
- oxygène,
- sulfoxyle,
- sulfonyle,
- azo et
- un résidu aliphatique saturé ou insaturé qui est optionnellement substitué ayant 1 à 6 atomes de carbone,
ou des sels pharmaceutiquement acceptables de ceux-ci.

2. Composés selon la revendication 1, dans lesquels Z¹ est hydroxyle.

3. Composés selon la revendication 1, dans lesquels Z¹ est un résidu de la formule -N-R¹⁰R¹¹, dans laquelle
R¹⁰ est de l'hydrogène et R¹¹ est un alkyle substitué ou
R¹¹ est de l'hydrogène et R¹⁰ est un alkyle substitué.

4. Composés selon la revendication 3, dans lesquels l'alkyle substitué est un groupe alkyle qui a au moins un groupe de la formule
-X²-R¹², dans laquelle
X² est sélectionné parmi le groupe consistant en :
- carbonyle,
- sulfoxyle et
- sulfonyle et
R¹² est sélectionné parmi le groupe consistant en :
- hydroxyle,
- amino qui est optionnellement substitué et
- alcoxyle qui est optionnellement substitué.

5. Composés selon l'une des revendications 1, 3 ou 4, dans lesquels
R¹⁰ est de l'hydrogène et R¹¹ est un résidu A du composé de la formule H₂N-A ou
R¹¹ est de l'hydrogène et R¹⁰ est un résidu A du composé de la formule H₂N-A, dans laquelle
A est un résidu qui est dérivé par la scission du groupe amino (-NH₂) d'un acide aminé naturel ou synthétique, d'un dérivé d'un acide aminé naturel ou synthétique ou d'un acide polyaminé ou d'un dérivé d'un acide polyaminé.

6. Composés selon l'une ou plusieurs des revendications 1 à 5 de la formule (2) :

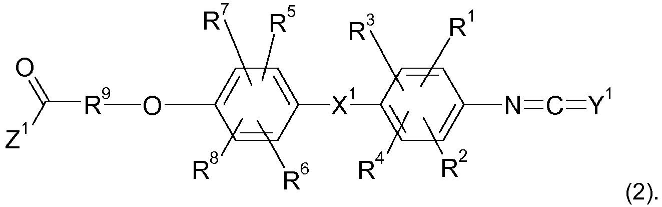

7. Composés selon l'une des revendications 1 à 6, dans lesquels
Y¹ est = S ou O,
chacun de R¹, R², R³, R⁴, R⁵, R⁶, R⁷ et R⁸ est de l'hydrogène,
R⁹ est un alcandiyle linéaire, ramifié ou cyclique,
Z¹ est un hydroxyle ou un résidu de la formule -N-R¹⁰R¹¹, dans laquelle R¹⁰ est de l'hydrogène et R¹¹ est un résidu A du composé de la formule H₂N-A ou
R¹¹ est de l'hydrogène et R¹⁰ est un résidu A du composé de la formule H₂N-A, dans laquelle
A est un résidu qui est dérivé par la scission du groupe amino (-NH₂) d'un acide aminé naturel ou synthétique, d'un dérivé d'un acide aminé naturel ou synthétique ou d'un acide polyaminé ou d'un dérivé d'un acide polyaminé et
X¹ est du carbonyle (-CO-).

8. Composés selon l'une des revendications 1 à 6, dans lesquels
Y¹ est = S ou O,
chacun de R¹, R², R³, R⁴, R⁵, R⁶, R⁷ et R⁸ est de l'hydrogène,
R⁹ est un alcandiyle linéaire, ramifié ou cyclique,
Z¹ est de l'hydroxyle et
X¹ est du carbonyle (-CO-).

9. Composés selon l'une ou plusieurs des revendications 1 à 8 qui sont sélectionnés parmi le groupe consistant en :
| | | | |
|---|---|---|---|
| 1a | 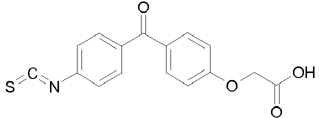 | 1b | 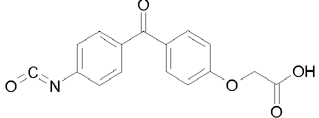 |
| 2a | 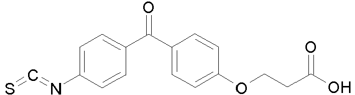 | 2b | 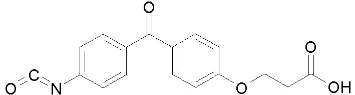 |
| 3a | 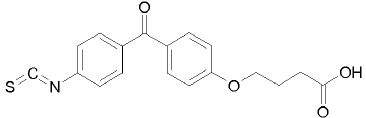 | 3b | 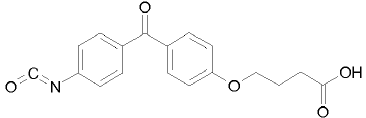 |
| 4a | 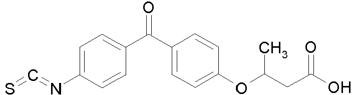 | 4b | 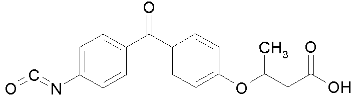 |
| 5a | 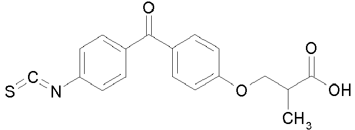 | 5b | 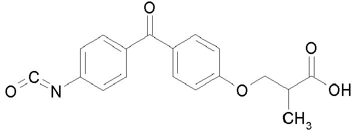 |
| 6a | 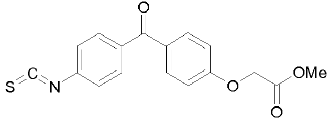 | 6b | 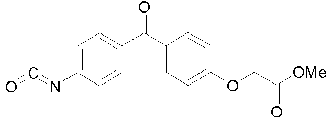 |
| 7a | 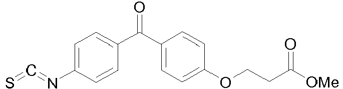 | 7b | 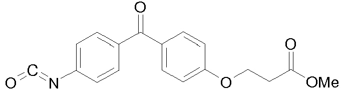 |
| 8a | 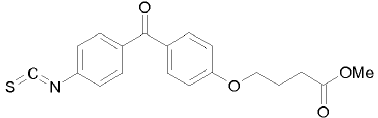 | 8b | 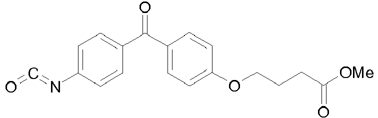 |
| 9a | 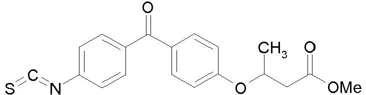 | 9b | 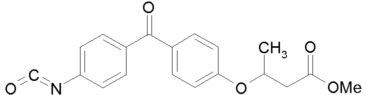 |
| 10 a | 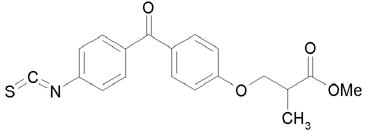 | 10 b | 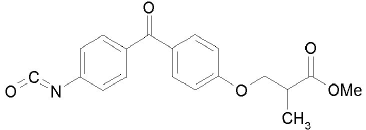 |
| 11 a | 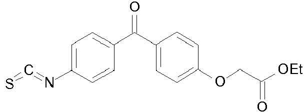 | 11 b | 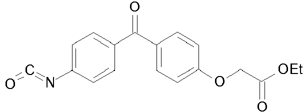 |
| 12 a | 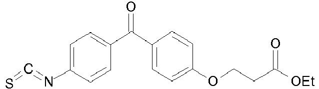 | 12 b | 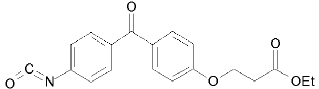 |
| 13 a | 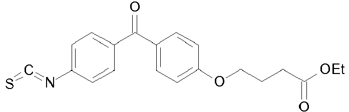 | 13 b | 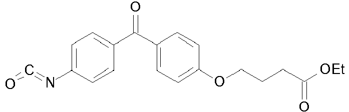 |
| 14 a | 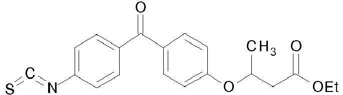 | 14 b | 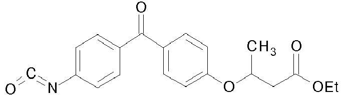 |
| 15 a | 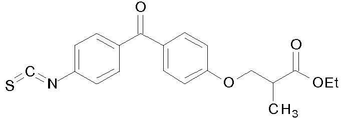 | 15 b | 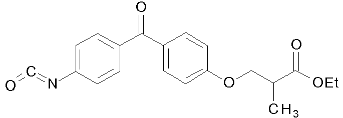 |
| 16 a | 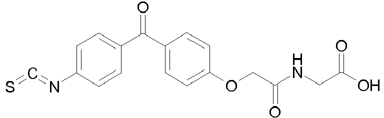 | 16 b | 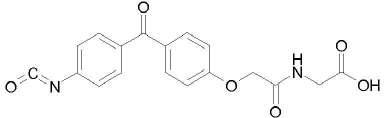 |
| 17 a | 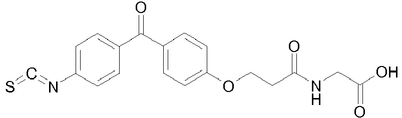 | 17 b | 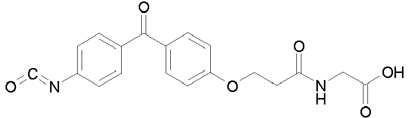 |
| 18 a | 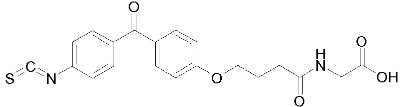 | 18 b | 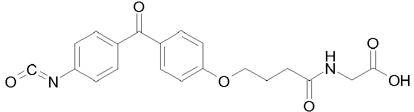 |
| 19 a | 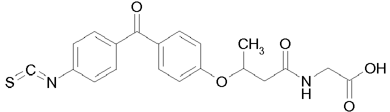 | 19 b | 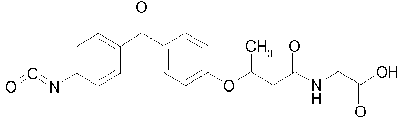 |
| 20 a | 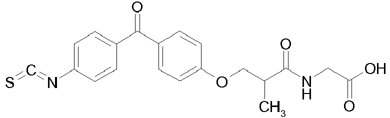 | 20 b | 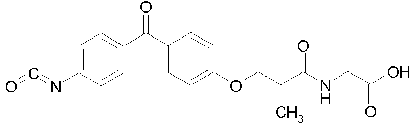 |
| 21 a | 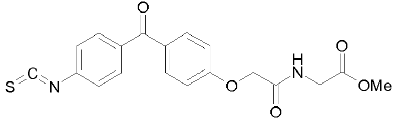 | 21 b | 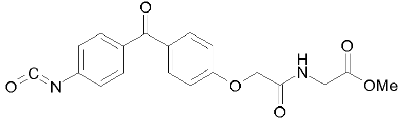 |
| 22 a | 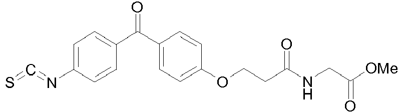 | 22 b | 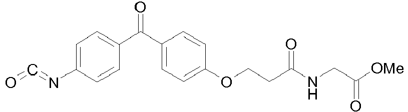 |
| 23 a | 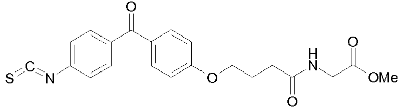 | 23 b | 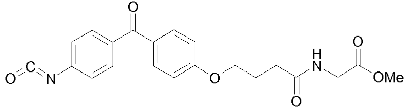 |
| 24 a | 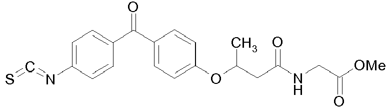 | 24 b | 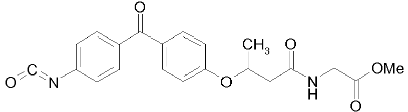 |
| 25 a | 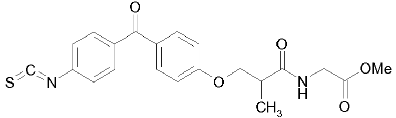 | 25 b | 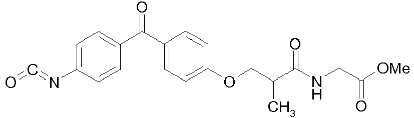 |
| 26 a | 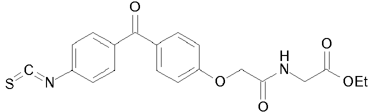 | 26 b | 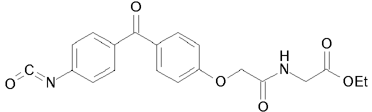 |
| 27 a | 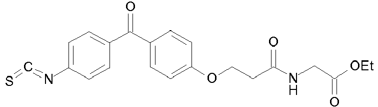 | 27 b | 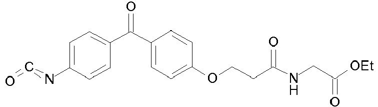 |
| 28 a | 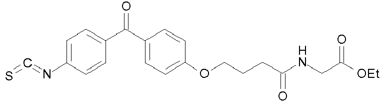 | 28 b | 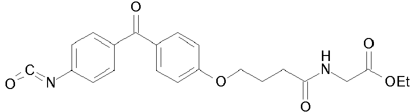 |
| 29 a | 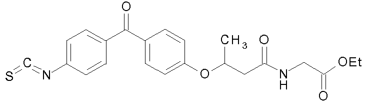 | 29 b | 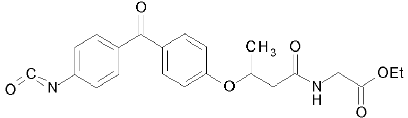 |
| 30 a | 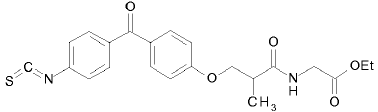 | 30 b | 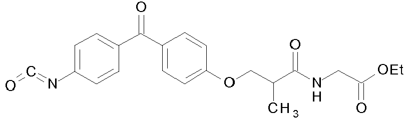 |
| 31 a | 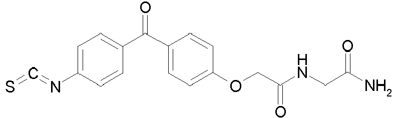 | 31 b | 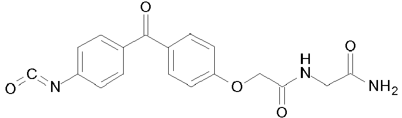 |
| 32 a | 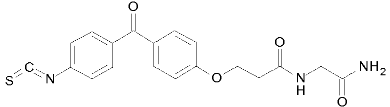 | 32 b | 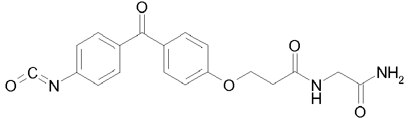 |
| 33 a | 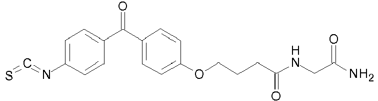 | 33 b | 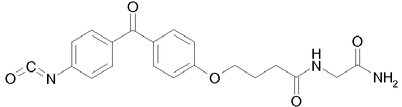 |
| 34 a | 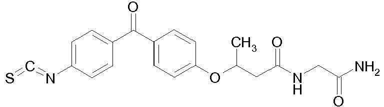 | 34 b | 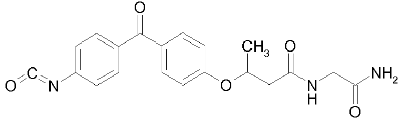 |
| 35 a | 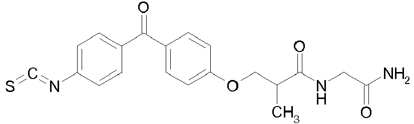 | 35 b | 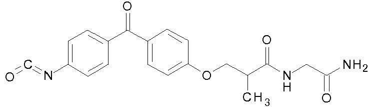 |
| 36 a | 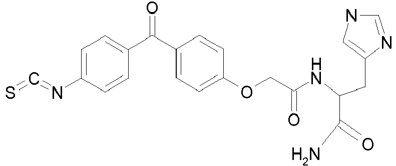 | 36 b | 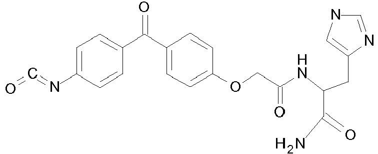 |
| 37 a | 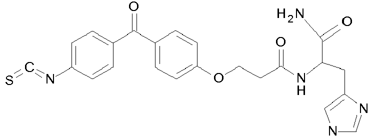 | 37 b | 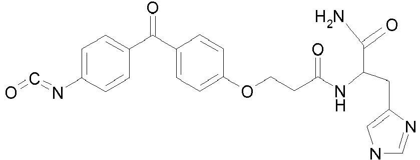 |
| 38 a | 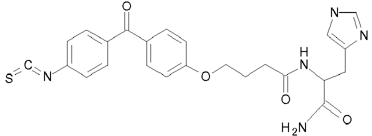 | 38 b | 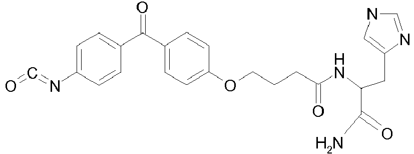 |
| 39 a | 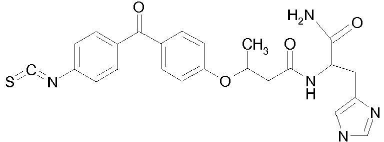 | 39 b | 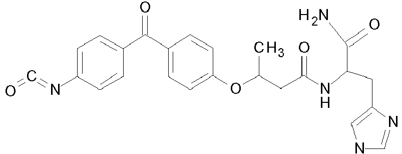 |
| 40 a | 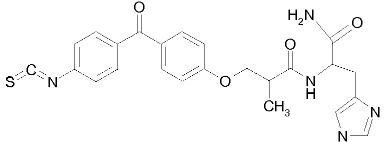 | 40 b | 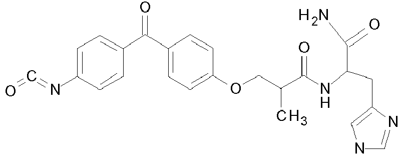 |
| 41 a | 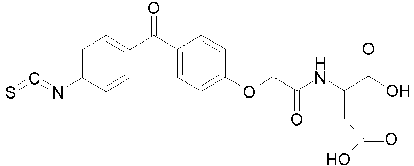 | 41 b | 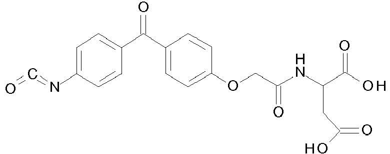 |
| 42 a | 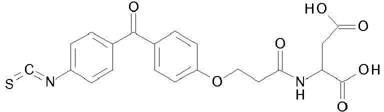 | 42 b | 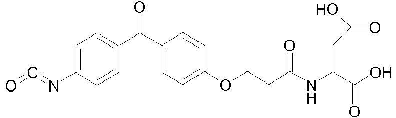 |
| 43 a | 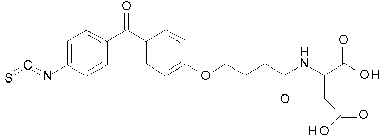 | 43 b | 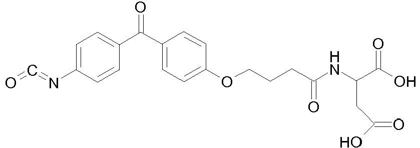 |
| 44 a | 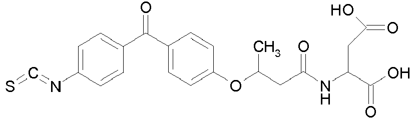 | 44 b | 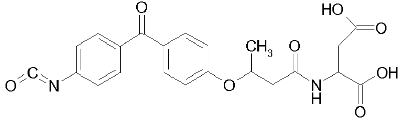 |
| 45 a | 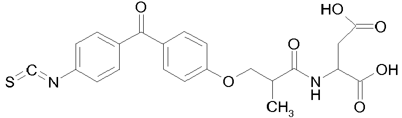 | 45 b | 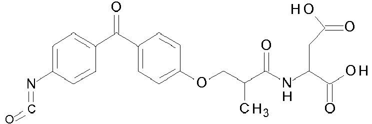 |
| 46 a | 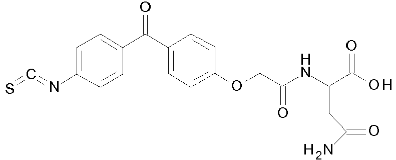 | 46 b | 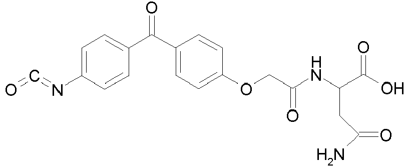 |
| 47 a | 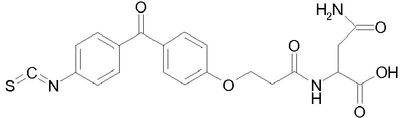 | 47 b | 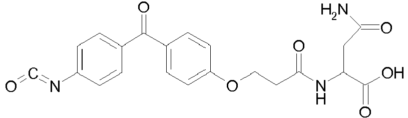 |
| 48 a | 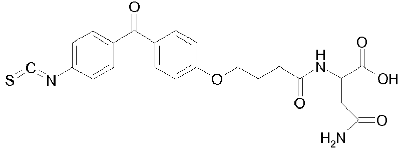 | 48 b | 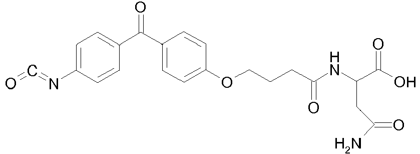 |
| 49 a | 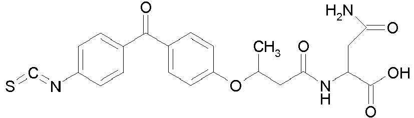 | 49 b | 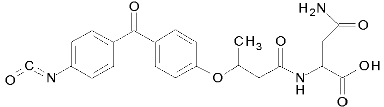 |
| 50 a | 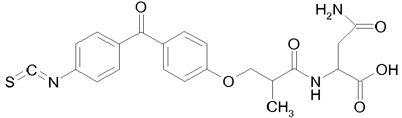 | 50 b | 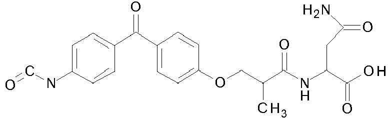 |
| 51 a | 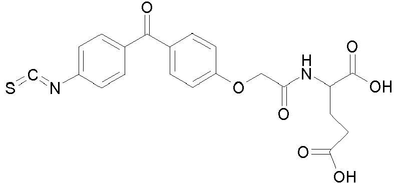 | 51 b | 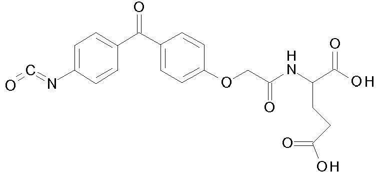 |
| 52 a | 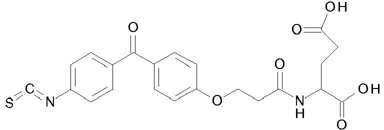 | 52 b | 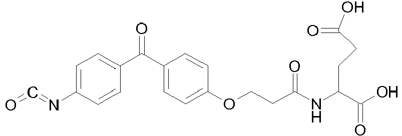 |
| 53 a | 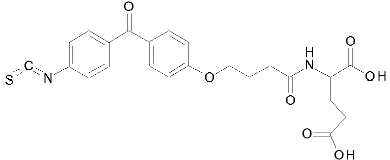 | 53 b | 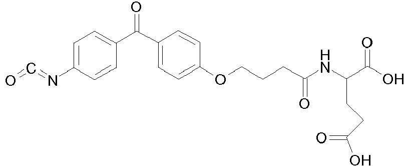 |
| 54 a | 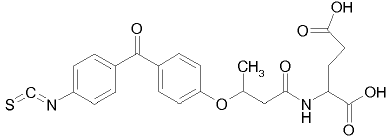 | 54 b | 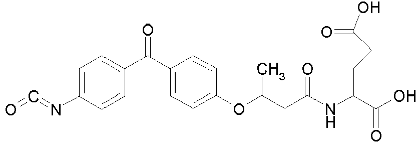 |
| 55 a | 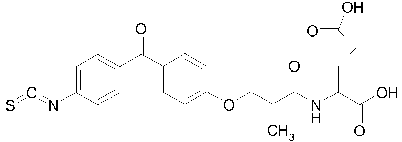 | 55 b | 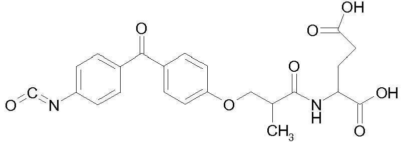 |
| 56 a | 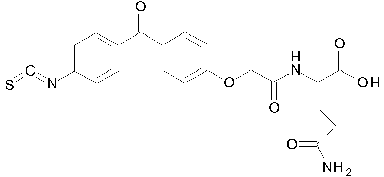 | 56 b | 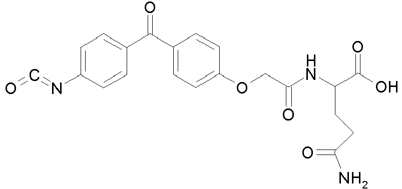 |
| 57 a | 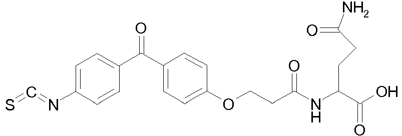 | 57 b | 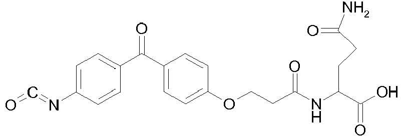 |
| 58 a | 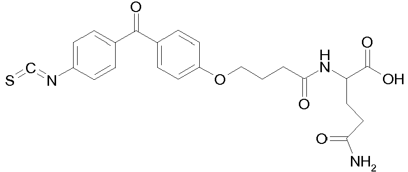 | 58 b | 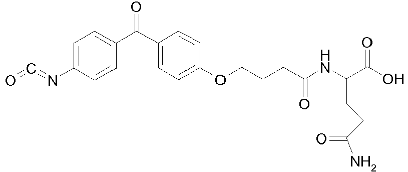 |
| 59 a | 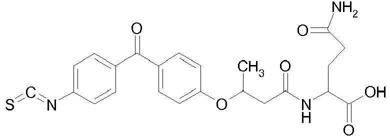 | 59 b | 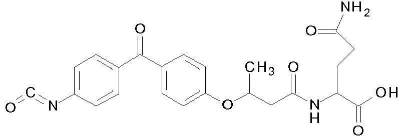 |
| 60 a | 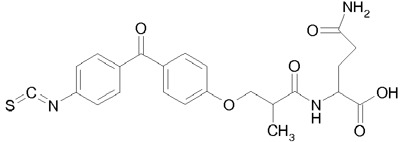 | 60 b | 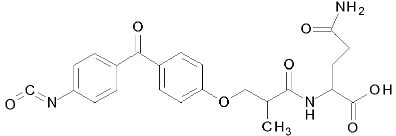 |
| 61 a | 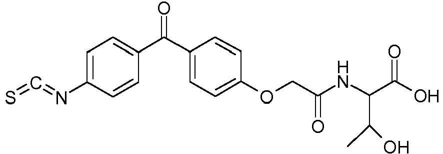 | 61 b | 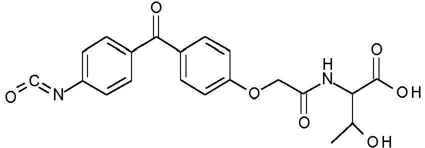 |
| 62 a | 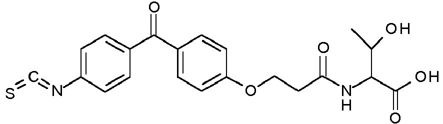 | 62 b | 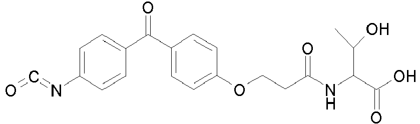 |
| 63 a | 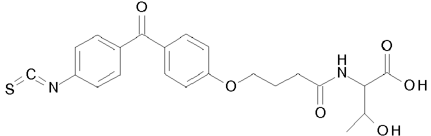 | 63 b | 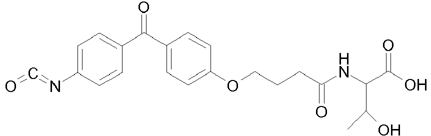 |
| 64 a | 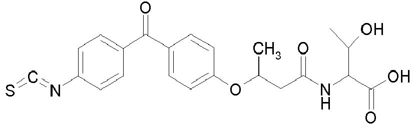 | 64 b | 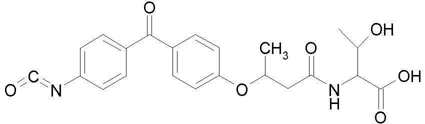 |
| 65 a | 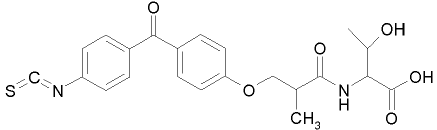 | 65 b | 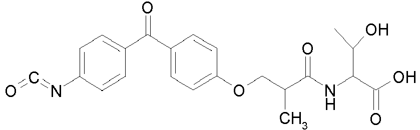 |
| 66 a | 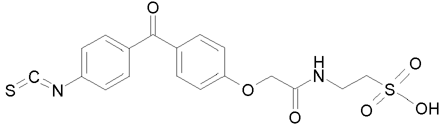 | 66 b | 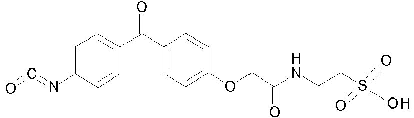 |
| 67 a | 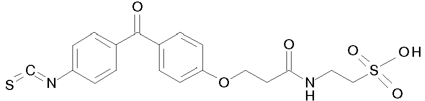 | 67 b | 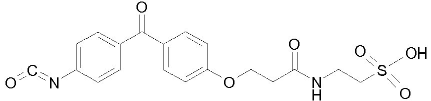 |
| 68 a | 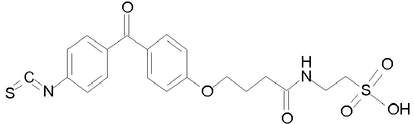 | 68 b | 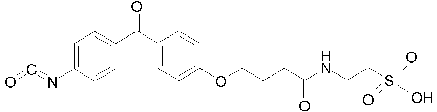 |
| 69 a | 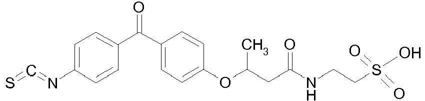 | 69 b | 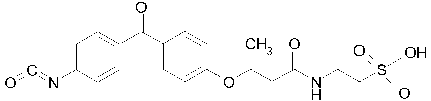 |
| 70 a | 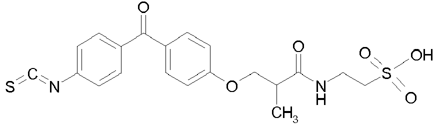 | 70 b | 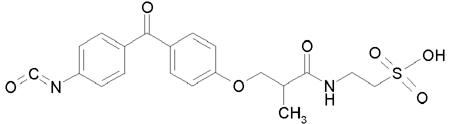 |
ou des sels pharmaceutiquement acceptables de ceux-ci.

10. Composés selon l'une ou plusieurs des revendications 1 à 9 destinés à être utilisés en tant qu'un médicament.

11. Composés selon l'une ou plusieurs des revendications 1 à 9 destinés à être utilisés en tant qu'un médicament pour le traitement des maladies cancéreuses.
